# EUROPEAN PATENT APPLICATION

(11) **EP 2 395 095 A1**
(43) Date of publication of application: **14.12.2011**
(21) Application number: 11178419.5
(22) Date of filing: 18.01.2008
(51) Int. Cl.: C12N 15/85

(54) **In vivo screening models for treatment of Alzheimer's disease and other QPCT-related disorders**

(30) Priority: 19.01.2007 US 885649 P
(62) Divisional of application: 08707977.8
(71) Applicant: Probiodrug AG, 06120 Halle/Saale (DE)
(72) Inventor: Schilling, Stephan, 06130 Halle/Sale (DE); Cynis, Holger, 06110 Halle/Saale (DE); Hoffmann, Torsten, 06114 Halle/Saale (DE); Demuth, Hans-Ulrich, 06120 Halle/Saale (DE); Wermann, Michael, 06118 Halle/Saale (DE); Schulz, Katrin, 06114 Halle/Saale (DE)
(74) Representative: Hoffmann, Matthias

(57) **Abstract**

The present invention provides a transgenic non-human animal, in particular a transgenic mouse encoding Qpct proteins, which have been implicated in Qpct-related diseases. The present invention additionally provides cells and cell lines comprising transgenes encoding for Qpct. The present invention further provides methods and compositions for evaluating agents that affect Qpct, for use in compositions for the treatment of Qpct-related diseases.

## Description

The present invention relates generally to transgenic animals as well as methods and compositions for screening and treating diseases, especially in relation to Qpct.

In particular the invention relates to Qpct (i.e. glutaminyl peptide cyclotransferase), also termed glutaminyl cyclase (QC, EC 2.3.2.5) that catalyzes the intramolecular cyclization of N-terminal glutamine residues into pyroglutamic acid (5-oxo-proline, pGlu*) under liberation of ammonia and the intramolecular cyclization of N-terminal glutamate residues into pyroglutamic acid under liberation of water.

A QC was first isolated by Messer from the Latex of the tropical plant Carica papaya in 1963 (Messer, M. 1963 Nature 4874, 1299). 24 years later, a corresponding enzymatic activity was discovered in animal pituitary (Busby, W. H. J. et al. 1987 J Biol Chem 262, 8532-8536; Fischer, W. H. and Spiess, J. 1987 Proc Natl Acad Sci U S A 84, 3628-3632). For the mammalian QC, the conversion of Gln into pGlu by QC could be shown for the precursors of TRH and GnRH (Busby, W. H. J. et al. 1987 J Biol Chem 262, 8532-8536; Fischer, W. H. and Spiess, J. 1987 Proc Natl Acad Sci U S A 84, 3628-3632). In addition, initial localization experiments of QC revealed a co-localization with its putative products of catalysis in bovine pituitary, further improving the suggested function in peptide hormone synthesis (Bockers, T. M. et al. 1995 J Neuroendocrinol 7, 445-453). In contrast, the physiological function of the plant QC is less clear. In case of the enzyme from C. papaya, a role in the plant defense against pathogenic microorganisms was suggested (El Moussaoui, A. et al. 2001 Cell Mol Life Sci 58, 556-570). Putative QCs from other plants were identified by sequence comparisons recently (Dahl, S. W. et al.2000 Protein Expr Purif 20, 27-36). The physiological function of these enzymes, however, is still ambiguous.

The QCs known from plants and animals show a strict specificity for L-Glutamine in the N-terminal position of the substrates and their kinetic behavior was found to obey the Michaelis-Menten equation (Pohl, T. et al. 1991 Proc Natl Acad Sci U S A 88, 10059-10063; Consalvo, A. P. et al. 1988 Anal Biochem 175, 131-138; Gololobov, M. Y. et al. 1996 Biol Chem Hoppe Seyler 377, 395-398). A comparison of the primary structures of the QCs from C. papaya and that of the highly conserved QC from mammals, however, did not reveal any sequence homology (Dahl, S. W. et al. 2000 Protein Expr Purif 20, 27-36). Whereas the plant QCs appear to belong to a new enzyme family (Dahl, S. W. et al. 2000 Protein Expr Purif 20, 27-36), the mammalian QCs were found to have a pronounced sequence homology to bacterial aminopeptidases (Bateman, R. C. et al. 2001 Biochemistry 40, 11246-11250), leading to the conclusion that the QCs from plants and animals have different evolutionary origins.

EP 02 011 349.4 discloses polynucleotides encoding insect glutaminyl cyclase, as well as polypeptides encoded thereby. This application further provides host cells comprising expression vectors comprising polynucleotides of the invention. Isolated polypeptides and host cells comprising insect QC are useful in methods of screening for agents that reduce glutaminyl cyclase activity. Such agents are described as useful as pesticides.

The subject matter of the present invention is particularly useful in the field of Qpct-related diseases, one example of those being Alzheimer's Disease. Alzheimer's disease (AD) is characterized by abnormal accumulation of extracellular amyloidotic plaques closely associated with dystrophic neurones, reactive astrocytes and microglia (Terry, R. D. and Katzman, R. 1983 Ann Neurol 14, 497-506; Glenner, G. G. and Wong, C. W. 1984 Biochem Biophys Res Comm 120, 885-890; Intagaki, S. et al. 1989 J Neuroimmunol 24, 173-182; Funato, H. et al. 1998 Am J Pathol 152, 983-992; Selkoe, D. J. 2001 Physiol Rev 81, 741-766). Amyloid-beta (abbreviated as Aβ) peptides are the primary components of senile plaques and are considered to be directly involved in the pathogenesis and progression of AD, a hypothesis supported by genetic studies (Glenner, G. G. and Wong, C. W. 1984 Biochem Biophys Res Comm 120, 885-890; Borchelt, D. R. et al. 1996 Neuron 17, 1005-1013; Lemere, C. A. et al. 1996 Nat Med 2, 1146-1150; Mann, D. M. and Iwatsubo, T. 1996 Neurodegeneration 5, 115-120; Citron, M. et al. 1997 Nat Med 3, 67-72; Selkoe, D. J. 2001 Physiol Rev 81, 741-766). Aβ is generated by proteolytic processing of the β-amyloid precursor protein (APP) (Kang, J. et al. 1987 Nature 325, 733-736; Selkoe, D. J. 1998 Trends Cell Biol 8, 447-453), which is sequentially cleaved by β-secretase at the N-terminus and by γ-secretase at the C-terminus of Aβ (Haass, C. and Selkoe, D. J. 1993 Cell 75, 1039-1042; Simons, M. et al. 1996 J Neurosci 16 899-908). In addition to the dominant Aβ peptides starting with L-Asp at the N-terminus (Aβ1-42/40), a great heterogeneity of N-terminally truncated forms occurs in senile plaques. Such shortened peptides are reported to be more neurotoxic in vitro and to aggregate more rapidly than the full-length isoforms (Pike, C. J. et al. 1995 J Biol Chem 270, 23895-23898). N-truncated peptides are known to be overproduced in early onset familial AD (FAD) subjects (Saido, T. C. et al. 1995 Neuron 14, 457-466; Russo, C, et al. 2000 Nature 405, 531-532), to appear early and to increase with age in Down's syndrome (DS) brains (Russo, C. et al. 1997 FEBS Lett 409, 411-416, Russo, C. et al. 2001 Neurobiol Dis 8, 173-180; Tekirian, T. L. et al. 1998 J Neuropathol Exp Neurol 57, 76-94). Finally, their amount reflects the progressive severity of the disease (Russo, C. et al. 1997 FEBS Lett 409, 411-416; Guntert, A. et al. 2006 Neuroscience 143, 461-475). Additional post-translational processes may further modify the N-terminus by isomerization or racemization of the aspartate at position 1 and 7 and by cyclization of glutamate at residues 3 and 11. Pyroglutamate-containing isoforms at position 3 [pGlu³Aβ3-40/42] represent the prominent forms -approximately 50 % of the total Aβ amount - of the N-truncated species in senile plaques (Mori, H. et al. 1992 J Biol Chem 267, 17082-17086, Saido, T. C. et al. 1995 Neuron 14, 457-466; Russo, C. et al. 1997 FEBS Lett 409, 411-416; Tekirian, T. L. et al. 1998 J Neuropathol Exp Neurol 57, 76-94; Geddes, J. W. et al. 1999 Neurobiol Aging 20, 75-79; Harigaya, Y. et al. 2000 Biochem Biophys Res Commun 276, 422-427) and they are also present in pre-amyloid lesions (Lalowski, M. et al. 1996 J Biol Chem 271, 33623-33631). The accumulation of ApN3 (pE) peptides is likely due to the structural modification that enhances aggregation and confers resistance to most aminopeptidases (Saido, T. C. et al. 1995 Neuron 14, 457-466 ; Tekirian, T. L. et al. 1999 J Neurochem 73, 1584-1589). This evidence provides clues for a pivotal roe of AβN3 (pE) peptides in AD pathogenesis. However, relatively little is known about their neurotoxicity and aggregation properties (He, W. and Barrow, C. J. 1999 Biochemistry 38, 10871-10877; Tekirian, T. L. et al. 1999 J Neurochem 73, 1584-1589). Moreover, the action of these isoforms on glial cells and the glial response to these peptides are completely unknown, although activated glia is strictly associated to senile plaques and might actively contribute to the accumulation of amyloid deposits. In recent studies the toxicity, aggregation properties and catabolism of Aβ1-42, Aβ1-40, [pGlu³]Aβ3-42, [pGlu³]Aβ3-40, [pGlu¹¹]Aβß11-42 and pGlu¹¹]Aβ11-40 peptides were investigated in neuronal and glial cell cultures, and it was shown that pyroglutamate modification exacerbates the toxic properties of Aβ-peptides and also inhibits their degradation by cultured astrocytes. Shirotani et al. investigated the generation of [pGlu³]Aβ peptides in primary cortical neurons infected by Sindbis virus *in vitro*. They constructed amyloid precursor protein complementary DNAs, which encoded a potential precursor for [pGlu³]Aβ by amino acid substitution and deletion. For one artificial precursor starting with a N-terminal glutamine residue instead of glutamate in the natural precursor, a spontaneous conversion or an enzymatic conversion by glutaminyl cyclase to pyroglutamate was suggested. The cyclization mechanism of N-terminal glutamate at position 3 in the natural precursor of [pGlu³]Aβ was neither determined *in vitro, in situ* nor *in vivo* (Shirotani, K. et al. 2002 NeuroSci Lett 327, 25-28).

### SUMMARY OF THE INVENTION

The present invention comprises methods and compositions for non-human transgenic, in particular mammal, models for Qpct-related diseases. Specifically, the present invention comprises non-human transgenic animal models that overexpress Qpct.

The present invention further comprises compositions and methods for screening for biologically active agents that modulate Qpct-related diseases including, but not limited to, Mild Cognitive Impairment (MCI), Alzheimer's Disease (AD), cerebral amyloid angiopathy, Lewy body dementia, neurodegeneration in Down Syndrome, hereditary cerebral hemorrhage with amyloidosis (Dutch type), Familial Danish Dementia, Familial British Dementia, ulcer disease and gastric cancer with or w/o *Helicobacter pylori* infections, pathogenic psychotic conditions, schizophrenia, infertility, neoplasia, inflammatory host responses, cancer, psoriasis, rheumatoid arthritis, atherosclerosis, restenosis, lung fibrosis, liver fibrosis, renal fibrosis, Acquired Immune Deficiency Syndrome, graft rejection, Chorea Huntington (HD), impaired humoral and cell-mediated immune responses, leukocyte adhesion and migration processes in the endothelium, impaired food intake, sleep-wakefulness, impaired homeostatic regulation of energy metabolism, impaired autonomic function, impaired hormonal balance and impaired regulation of body fluids and the Guam Parkinson-Dementia complex. Another aspect of the present invention comprises methods and compositions for screening for Qpct inhibitors.

Additionally, the present invention comprises methods and compositions for the treatment and/or prevention of Qpct-related diseases, particularly methods and compositions that inhibit or promote Qpct.

Accordingly, it is an object of the invention to provide a transgenic animal, which overexpresses Qpct.

It is another object of the invention to provide DNA constructs encoding Qpct.

It is an additional object of the invention to provide DNA constructs encoding Qpct linked to a promoter.

It is a further object of the invention to provide a non-human transgenic animal model system.

It is an additional object of the invention to provide a non-human transgenic animal model system to study the *in vivo* and *in vitro* regulation and effects of Qpct in specific tissue types.

It was shown by inhibition studies that human and murine QC are metal-dependent transferases. QC apoenzyme could be reactivated most efficiently by zinc ions, and the metal-binding motif of zinc-dependent aminopeptidases is also present in human QC. Compounds interacting with the active-site bound metal are potent inhibitors.

Unexpectedly, it was shown that recombinant human QC as well as QC-activity from brain extracts catalyze both, the N-terminal glutaminyl as well as glutamate cyclization. Most striking is the finding, that QC-catalyzed Glu¹-conversion is favored around pH 6.0 while Gln¹-conversion to pGlu-derivatives occurs with a pH-optimum of around 8.0. Since the formation of pGlu-Aβ-related peptides can be suppressed by inhibition of recombinant human QC and QC-activity from pig pituitary extracts, the enzyme QC is a target in drug development for treatment of e.g. Alzheimer's disease.

By administering effectors of QC activity to a mammal it can be possible to prevent or alleviate or treat conditions selected from Mild Cognitive Impairment (MCI), Alzheimer's Disease (AD), cerebral amyloid angiopathy, Lewy body dementia, neurodegeneration in Down Syndrome, hereditary cerebral hemorrhage with amyloidosis (Dutch type), Familial Danish Dementia, Familial British Dementia, ulcer disease and gastric cancer with or w/o *Helicobacter pylori* infections, pathogenic psychotic conditions, schizophrenia, infertility, neoplasia, inflammatory host responses, cancer, psoriasis, rheumatoid arthritis, atherosclerosis, restenosis, lung fibrosis, liver fibrosis, renal fibrosis, Acquired Immune Deficiency Syndrome, graft rejection, Chorea Huntington (HD), impaired humoral and cell-mediated immune responses, leukocyte adhesion and migration processes in the endothelium, impaired food intake, sleep-wakefulness, impaired homeostatic regulation of energy metabolism, impaired autonomic function, impaired hormonal balance and impaired regulation of body fluids.

Further, by administration of effectors of QC activity to a mammal it can be possible to stimulate gastrointestinal tract cell proliferation, preferably proliferation of gastric mucosal cells, epithelial cells, acute acid secretion and the differentiation of acid producing parietal cells and histamine-secreting enterochromaffin-like cells.

Furthermore, by administration of effectors of QC activity to a mammal it can be possible to suppress the proliferation of myeloid progenitor cells.

In addition, administration of QC inhibitors can lead to suppression of male fertility.

The present invention provides pharmaceutical compositions for parenteral, enteral or oral administration, comprising at least one effector of QC optionally in combination with customary carriers and/or excipients.

### BRIEF DESCRIPTION OF THE FIGURES

Further understanding of these and other aspects of the present invention will be gained by reference to the figures, which represent the following:

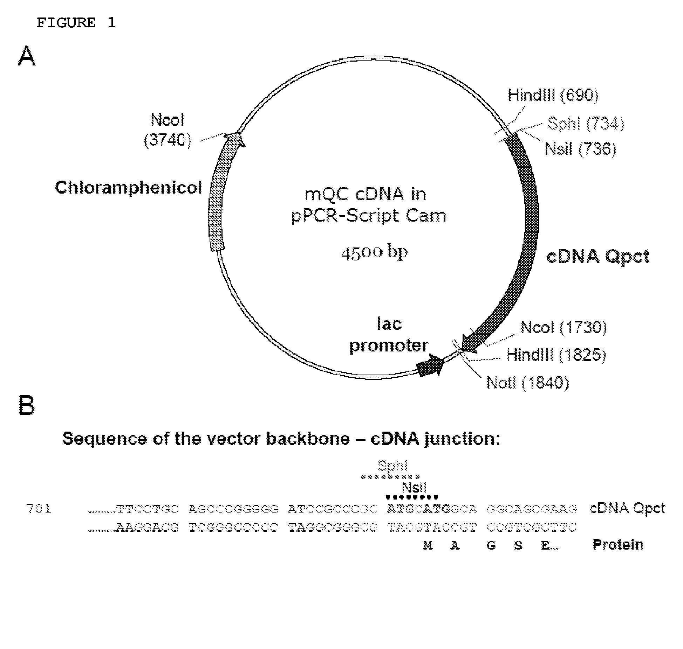

**Figure 1** **A and B:**
   **(A)** Map of the plasmid 'mQC cDNA in vector pPCR Script Cam SK(+)' showing the presence of the murine Qpct cDNA cassette. **(B)** Sequence of the vector backbone - cDNA junction. Due to the SphI restriction sequence in the primer utilized for cloning, an additional ATG was inserted 4 bp upstream of the start codon in vector pPCR Script Cam SK(+).

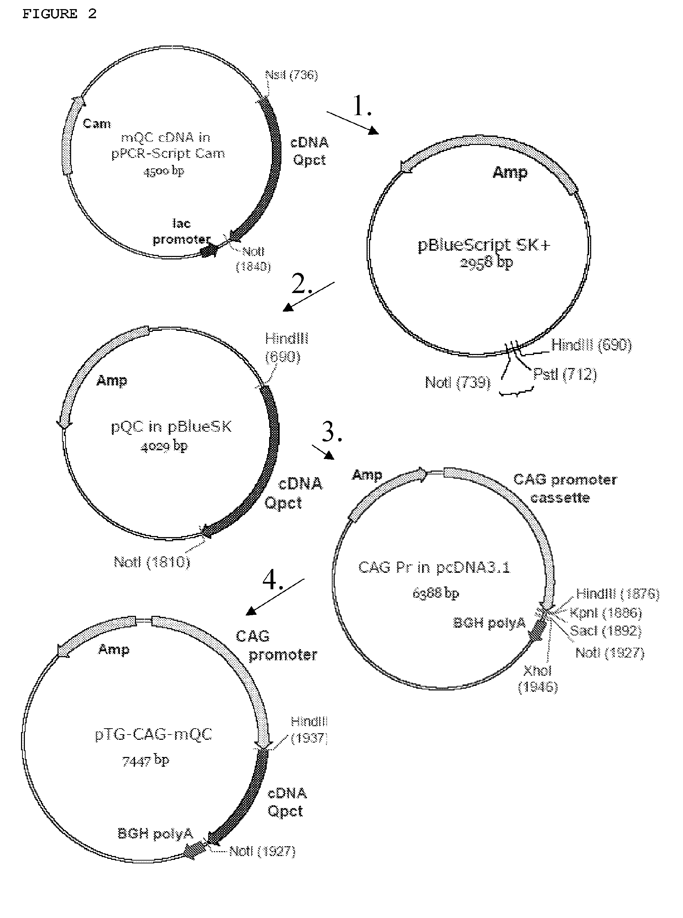

**Figure 2****: Cloning strategy.**
   The Qpct cDNA was isolated first via NsiI and NotI from the pPCR-Script Cam SK(+) vector backbone and subcloned via PstI and NotI into the pBlueScript SK+ (1 + 2). Subsequently the cDNA cassette was subcloned via the restriction sites HindIII and NotI into a plasmid CAG Pr in pcDNA3.1 (3 + 4). The resulting pTG-CAG-mQC consists of the Qpct cDNA cassette under the control of a ubiquitously expressing CAG promoter and the BGH polyA signal.

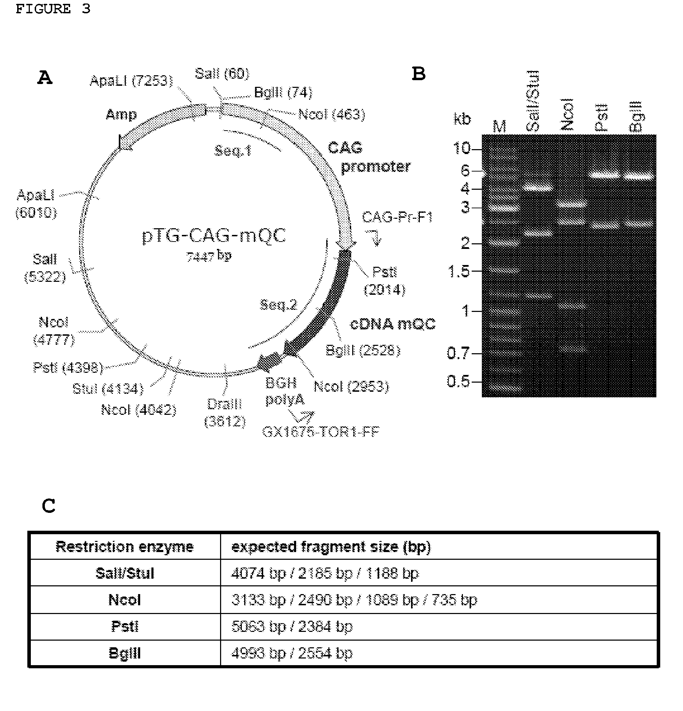

**Figure 3****: Restriction map of the transgenic construct plasmid pTG-CAG-mQC and its restriction analysis.**
   (A) The sequenced regions are labelled as narrow lines (SEQ ID No: 1 and 2). The binding sites of the genotyping primers and the restriction sites of the enzymes used in the restriction analysis are indicated in the plasmid map. The position of the genotyping primers (CAG-Pr-F1 and GX1675-TOR1-FF) are shown as grey arrows (B, C). All DNA digests resulted in the expected fragment sizes as predicted in the table M: MassRuler DNA-Ladder Mix (GeneRuler; MBI).

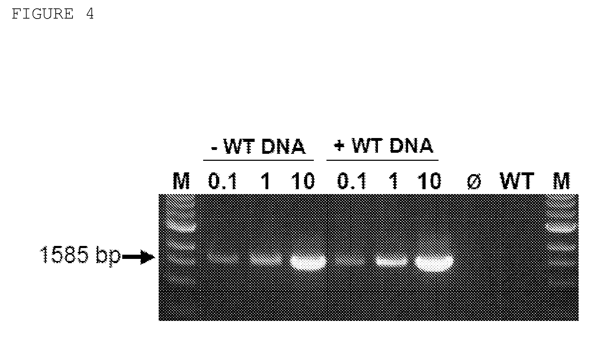

**Figure 4****: Testing the sensitivity of the screening PCR.**
   The optimised PCR screen was conducted using serial dilutions of the pTG-CAG-mQC plasmid in H₂O (- WT DNA) or in 150 µg genomic wild-type DNA (+ WT DNA). The equivalent of 0.1, 1 and 10 copies of the transgenic vector per mouse genome were tested. PCR amplification of the transgenic DNA with the primer pair CAG-Pr-F1/GX1675 yielded a 1585 bp product. Wild-type genomic DNA (WT) and H₂O (∅) were included as negative controls. M:1kb DNA ladder (New England Biolabs, NEB).
**Figure 5****: Preparation and validation of the pTG-CAG-mQC transgenic construct.**
   (A) Plasmid card of the vector pTG-CAG-mQC indicating the restriction sites of SalI and DraIII and the binding sites for the screening primers. (B) DraIII/SalI restriction of pTG-CAG-mQC (1) and the isolated transgenic construct (2). M: 1kb DNA ladder (New England Biolabs, NEB)
**Figure 6****: Pronuclei microinjections.**
   (A) Oocyte before injection. The male pronucleus is clearly visible. (B) Oocyte after injection. The male pronucleus is clearly enlarged, demonstrating the successful injection.
**Figure 7****: Screening for founders.**
   The PCR result for the last born 19 pups is shown. PCR with the primers CAG-Pr-F1/GX1675-TOR1-FF (transgene PCR; 1.6 kb) and with the primer pair BGH-F1/CAG-Pr-R2 (head-to-tail PCR; 0.8 kb) was performed with genomic DNA from the F0 generation. Positive control (+) for the transgene PCR was the plasmid pTG-CAG-mQC, diluted with wild-type genomic DNA. The quality of the DNA was confirmed by a control PCR amplifying a 431 bp wild-type signal. Positive control (TG) for the head-to-tail PCR was the genomic DNA of a transgenic line with same promoter-polyA signal sequences giving rise to a 1.3 kb PCR product. Negative control was wild-type DNA (WT). As an additional negative control H₂O (∅) was used. M: 100 bp DNA-Ladder (New England Biolabs, NEB)

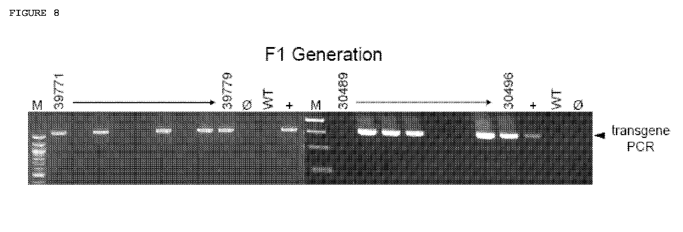

**Figure 8****: Genotyping of F1 generation.**
   The PCR result of 17 pups is shown as an example. PCR with primers CAG-Pr-F1/GX1675-TOR1-FF (transgene PCR; 1.6 kb) was performed with genomic DNA from the F1 generation. Positive control (+) for the transgene PCR was the plasmid pTG-CAG-mQC, diluted with wild-type genomic DNA. Negative control was wild-type DNA (WT). As an additional negative control H₂O (∅) was used. M1: 100 bp DNA-Ladder (NEB)
**Figure 9**
   **9A:** Standard curve of pyroglutamyl-beta-naphthylamine, prepared using a Merck-Hitachi L-6210 HPLC system equipped with a Merck RP18-LiChrocart 125-4 column. The compound was detected by absorption at 280 nm.
   **9B:** Determination of QC-activity in brain tissue of a QC-transgenic mouse (in triplicates, left row) and wildtype littermates (in triplicates, right row) by HPLC-detection of pyroglutamyl-beta-naphthylamine as product from the QC-mediated cyclization of glutaminyl-beta-naphthylamine. The slopes of the regression curves correspond to the initial velocity of QC-catalyzed product formation. Prior to plotting, the area units (read-out HPLC) were converted into a concentration using a standard curve of pyroglutamyl-beta-naphthylamine.

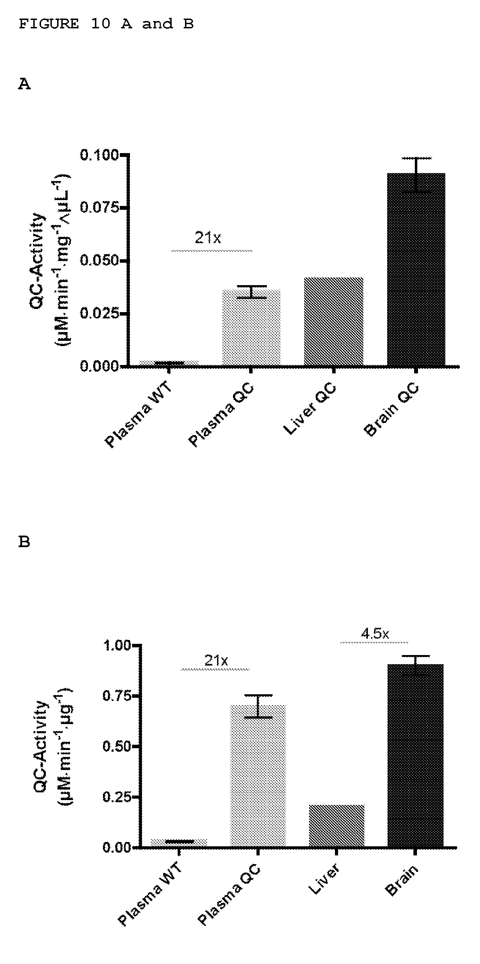

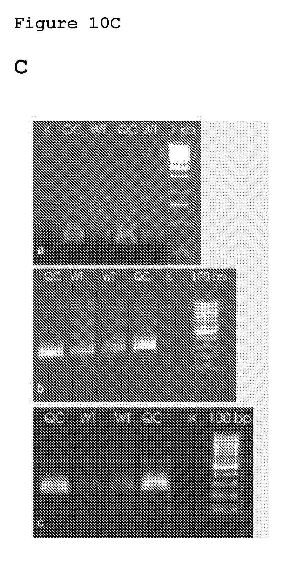

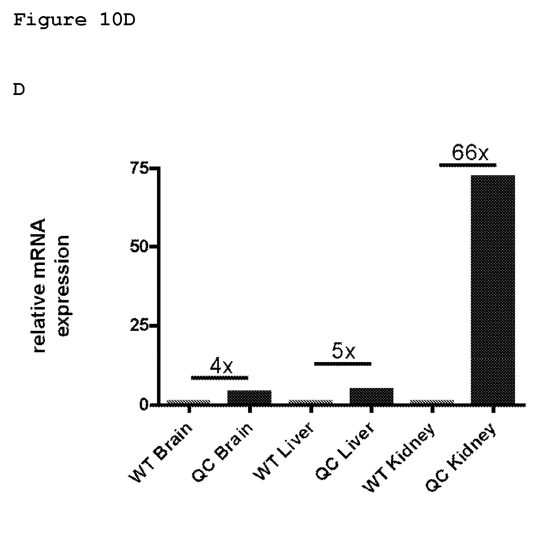

**Figure 10****: Evaluation of QC-transcript using PCR and real-time PCR**
   **10A:** QC activity in EDTA plasma, liver and brain referred to volume (plasma) or wet weight (tissue), determined by quantification of pyroglutamyl-beta-naphthylamine using HPLC.
   **10B:** QC activity in EDTA plasma, liver and brain referred protein content.
   **10C:** Qualitative PCR of mouse QC from generated cDNA of brain (a), liver (b) and kidney (c) from two transgenic QC-mice (QC) and controls (WT).
   **10D:** Quantitative real time RT-PCR using cDNA from brain, liver and kidney of QC transgenic (QC, N=2) mice and control (WT, N=2) mice as a template.

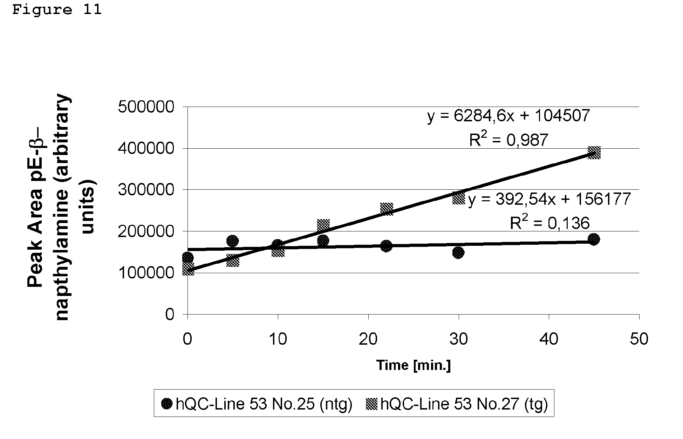

**Figure 11****: QC activity in brain homogenate of a transgenic mouse (hQC-line 53 No.27, squares) and a non-transgenic littermate (hQC-line 53 No.25, circles).** Activity was determined using an assay, which is based on detection and quantification of pyroglutamyl-beta-naphthylamine using HPLC. The higher slope in case of the transgenic animal proves expression of the transgene (human QC).

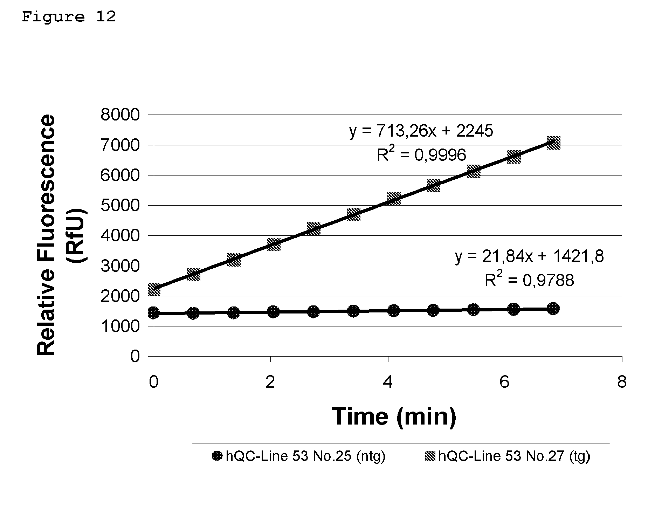

**Figure 12****: QC activity in brain homogenate of a transgenic mouse (hQC-line 53 No.27, squares) and a non-transgenic littermate (hQC-line 53 No.25, circles).** Activity was determined using an assay, which is based on fluorometric detection of QC activity. The higher slope in case of the transgenic animal proves expression of the transgene QC.

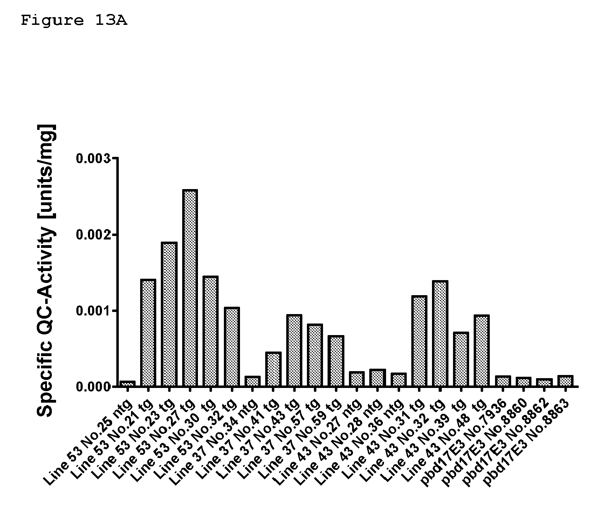

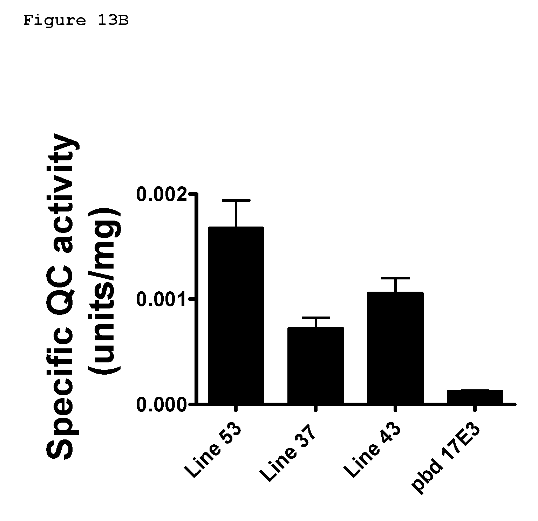

**Figure 13****: Comparison of QC-activity in the brain homogenates of transgenic mice expressing human QC neuron-specifically driven by the Thy-1 promotor (Line 53, Line 37 and Line 43) or murine QC ubiquitously (pbd17E3).** A) Comparison of QC-activity in brain samples of animals of different founder lines. Obviously, only hQC transgenic animals displayed significant QC-activity, not the non-transgenic littermates, proving that the transgene mediates the raise in QC activity. B) on the basis of these results, human QC-transgenic founder Line 53 was selected, displaying the highest QC activity in the brain sample.

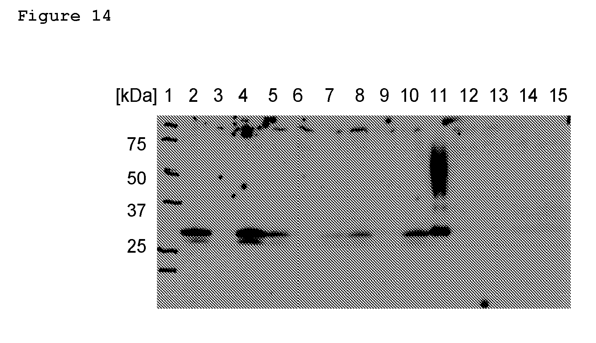

**Figure 14****: Western-Blot analysis of brain homogenates of different transgenic animals.** A primary polyclonal antibody was used, detecting murine and human QC. The results reflect the analysis of QC activity exactly. Highest QC immunoreactivity was obtained in transgenic animals of line 53. Only a faint staining was observed in pbd17E3 mice. The migration of QC is shown by the positive control, recombinant human QC. Lines: 1: Standard Proteins, 2: hQC-Line 53 No.23 (tg, female), 3: hQC-Line 53 No.25 (ntg, female), 4: hQC-Line 53 No.27 (tg, female), 5: hQC-Line 37 No.43 (tg, female), 6: hQC-Line 37 No.43 Nr.34 (ntg, male), 7: hQC-Line 37 No.43 Nr.57 (tg, female), 8: hQC-Line 43 No.31 (tg, female), 9: hQC-Line 43 No.36 (ntg, male), 10: hQC-Line 43 Nr.32 (tg, male), 11: hQC, Positive control [40ng], lines 12-15: PBD17E3 12: No.7936 tg, 13: Nr.8860 tg, 14: Nr.8862 tg, 15: Nr.8863 tg

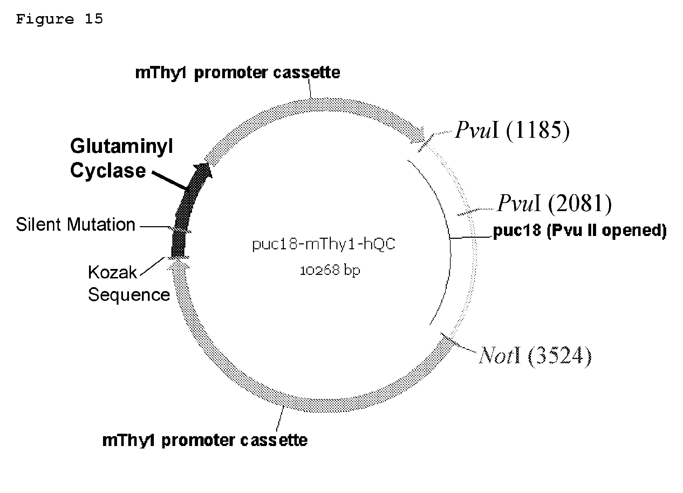

**Figure 15****: Schematic representation of the Plasmid used for generation of transgenic mice overexpressing human QC neuron-specifically.**

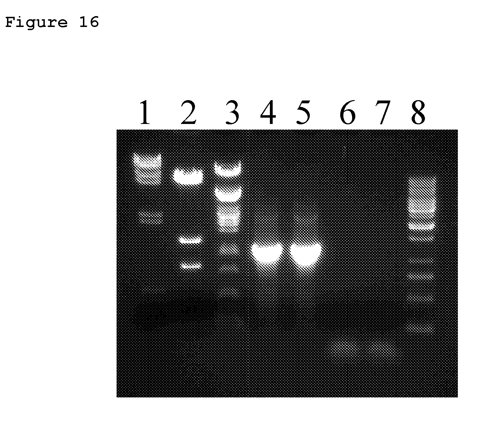

**Figure 16****: Analytical restriction digestion of the generated plasmid vector.** The fragment with a size of 7929 was used for microinjection. Lanes : 1, Hind III - Ladder; 2, pUC18-mThy1-hQC / Not I / Pvu I (7929bp + 1443bp + 896 bp); 3, Pst I - Ladder; 4, PCR: pUC18-mThy1-hQC 27 ng (1316 bp); 5, PCR: pUC18-mThy1-hQC 27 ng + 100 ng gDNA (1316 bp); 6, PCR: 100 ng gDNA; 7, PCR: Non template control; 8, 1 kb Ladder

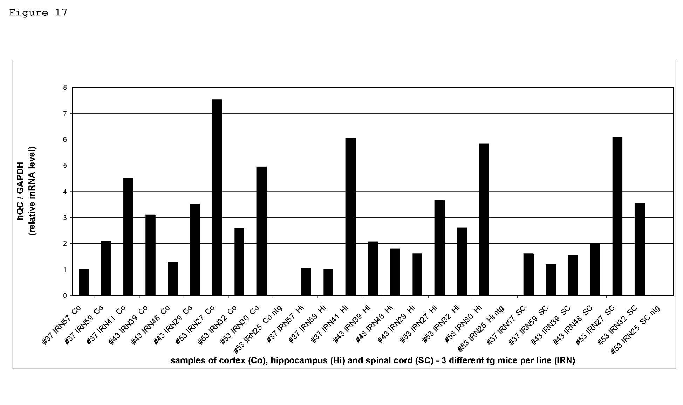

**Figure 17****: Quantitative determination of transcript levels of human QC (real-time PCR) of three different founder lines.** The levels are depicted as relative values compared with a standard, GAPDH (Glyceraldehyd-3-Phosphate Dehydrogenase). Highest transcript levels were determined with line 53, which also displayed the highest QC activity.

Other objects, advantages and features of the invention will become apparent upon consideration of the following detailed description.

### DETAILED DESCRIPTION OF THE INVENTION

The present invention comprises methods and compositions for the generation of a transgenic animal model for the study of Qpct-related diseases and the transgenic non-human animal *per se*. The present invention specifically comprises methods and compositions for generating a transgenic animal model that overexpresses glutaminyl-peptide cyclotransferase enzyme (Qpct) and the transgenic non-human animal *per se.* The present invention further comprises methods and compositions for testing Qpct inhibitors and promoters and methods of prevention/treatment and pharmaceutical compositions with Qpct inhibitors/promoters.

The present invention also provides a new method for the treatment of Mild Cognitive Impairment (MCI), Alzheimer's disease, Familial Danish Dementia, Familial British Dementia and neurodegeneration in Down Syndrome. The N-termini of the amyloid β-peptides deposited in Alzheimer's disease and Down syndrome brain, and the amyloid peptides ADan and ABri deposited in Familial Danish Dementia and Familial British Dementia as well, bear pyroglutamic acid. The pGlu formation is an important event in the development and progression in the disease, since the modified amyloid β-peptides, ADan and ABri show an enhanced tendency to amyloid aggregation and toxicity, likely worsening the onset and progression of the disease. (Russo, C. et al. 2002 J Neurochem 82, 1480-1489; Ghiso, J. et al. 2001 Amyloid 8, 277-284).

In contrast, in the natural β-peptides (3-40/42), glutamic acid is present as an N-terminal amino acid.

### DEFINITIONS

The term "transgene" means a segment of DNA that has been incorporated into a host genome or is capable of autonomous replication in a host cell and is capable of causing the expression of one or more cellular products. Exemplary transgenes will provide the host cell, or animals developed therefrom, with a novel phenotype relative to the corresponding non-transformed cell or animal.

The term "transgenic animal" means a non-human animal, usually a mammal, having a non-endogenous nucleic acid sequence present as an extrachromosomal element in a portion of its cells or stably integrated into its germ line DNA.

The term "construct" means a recombinant nucleic acid, generally recombinant DNA, that has been generated for the purpose of the expression of a specific nucleotide sequence(s), or is to be used in the construction of other recombinant nucleotide sequences. The recombinant nucleic acid can encode e.g. a chimeric or humanized polypeptide.

Polypeptide here pertains to all possible amino acid sequences comprising more than 10 amino acids.

The term "operably linked" means that a DNA sequence and a regulatory sequence (s) are connected in such a way as to permit gene expression when the appropriate molecules (e.g., transcriptional activator proteins) are bound to the regulatory sequence(s).

The term "operatively inserted" means that a nucleotide sequence of interest is positioned adjacent a nucleotide sequence that directs transcription and translation of the introduced nucleotide sequence of interest.

### Transgenes

The Qpct polynucleotides comprising the transgene of the present invention include Qpct cDNA and shall also include modified Qpct cDNA. As used herein, a "modification" of a nucleic acid can include one or several nucleotide additions, deletions, or substitutions with respect to a reference sequence. A modification of a nucleic acid can include substitutions that do not change the encoded amino acid sequence due to the degeneracy of the genetic code, or which result in a conservative substitution. Such modifications can correspond to variations that are made deliberately, such as the addition of a Poly A tail, or variations which occur as mutations during nucleic acid replication.

As employed herein, the term "substantially the same nucleotide sequence" refers to DNA having sufficient identity to the reference polynucleotide, such that it will hybridize to the reference nucleotide under moderately stringent, or higher stringency, hybridization conditions. DNA having "substantially the same nucleotide sequence" as the reference nucleotide sequence, can have an identity ranging from at least 60% to at least 95% with respect to the reference nucleotide sequence.

The phrase "moderately stringent hybridization" refers to conditions that permit a target-nucleic acid to bind a complementary nucleic acid. The hybridized nucleic acids will generally have an identity within a range of at least about 60% to at least about 95%. Moderately stringent conditions are conditions equivalent to hybridization in 50% formamide, 5x Denhart's solution, 5x saline sodium phosphate EDTA buffer (SSPE), 0.2% SDS (Aldrich) at about 42°C, followed by washing in 0.2x SSPE, 0.2% SDS (Aldrich), at about 42° C.

High stringency hybridization refers to conditions that permit hybridization of only those nucleic acid sequences that form stable hybrids in 0.018M NaCl at about 65° C, for example, if a hybrid is not stable in 0.018M NaCl at about 65° C, it will not be stable under high stringency conditions, as contemplated herein. High stringency conditions can be provided, for example, by hybridization in 50% formamide, 5x Denhart's solution, 5x SSPE, 0.2% SDS at about 42° C, followed by washing in 0.1x SSPE, and 0.1% SDS at about 65° C.

Other suitable moderate stringency and high stringency hybridization buffers and conditions are well known to those of skill in the art and are described, for example, in Sambrook et al., Molecular Cloning: A Laboratory Manual, 2nd ed., Cold Spring Harbor Press, Plainview, N.Y. (1989); and Ausubel et al. (Current Protocols in Molecular Biology (Supplement 47), John Wiley & Sons, New York (1999)).

The amino acid sequence encoded by the transgene of the present invention can be a Qpct sequence from a human or the Qpct homologue from any species, preferably from a murine species. The amino acid sequence encoded by the transgene of the present invention can also be a fragment of the Qpct amino acid sequence so long as the fragment retains some or all of the function of the full-length Qpct sequence. The sequence may also be a modified Qpct sequence. Individual substitutions, deletions or additions, which alter, add or delete a single amino acid or a small percentage of amino acids (typically less than 10%, more typically less than 5%, and still more typically less than 1%.) A "modification" of the amino acid sequence encompasses conservative substitutions of the amino acid sequence. Conservative substitution tables providing functionally similar amino acids are well known in the art. The following six groups each contain amino acids that are conservative substitutions for one another:
1) Alanine (A), Serine (S), Threonine (T);
2) Aspartic acid (D), Glutamic acid (E);
3) Asparagine (N), Glutamine (Q);
4) Arginine (R), Lysine (K);
5) Isoleucine (1), Leucine (L), Methionine (M), Valine (V); and
6) Phenylalanine (F), Tyrosine (Y), Tryptophan (W).

Other minor modifications are included within the sequence so long as the polypeptide retains some or all of the structural and/or functional characteristics of a Qpct polypeptide. Exemplary structural or functional characteristics include sequence identity or substantial similarity, antibody reactivity, the presence of conserved structural domains such as RNA binding domains or acidic domains.

### DNA Constructs and Vectors

The invention further provides a DNA construct comprising the Qpct transgene as described above. As used herein, the term "DNA construct" refers to a specific arrangement of genetic elements in a DNA molecule. In addition to human Qpct, or mutant forms thereof, the invention also provides a DNA construct using polypeptides from other species as well as Qpct mutant non-human mammals expressing BACE1 from non-human species.

If desired, the DNA constructs can be engineered to be operatively linked to appropriate expression elements such as promoters or enhancers to allow expression of a genetic element in the DNA construct in an appropriate cell or tissue. The use of the expression control mechanisms allows for the targeted delivery and expression of the gene of interest. For example, the constructs of the present invention may be constructed using an expression cassette which includes in the 5'-3' direction of transcription, a transcriptional and translational initiation region associated with gene expression in brain tissue, DNA encoding a mutant or wild-type Qpct protein, and a transcriptional and translational termination region functional in the host animal. One or more introns also can be present. The transcriptional initiation region can be endogenous to the host animal or foreign or exogenous to the host animal.

The DNA constructs described herein, may be incorporated into vectors for propagation or transfection into appropriate cells to generate Qpct overexpressing mutant non-human mammals and are also comprised by the present invention. One skilled in the art can select a vector based on desired properties, for example, for production of a vector in a particular cell such as a mammalian cell or a bacterial cell.

Vectors can contain a regulatory element that provides tissue specific or inducible expression of an operatively linked nucleic acid. One skilled in the art can readily determine an appropriate tissue-specific promoter or enhancer that allows expression of Qpct polypeptides in a desired tissue. It should be noted that tissue-specific expression as described herein does not require a complete absence of expression in tissues other than the preferred tissue. Instead, "cell-specific" or "tissue-specific" expression refers to a majority of the expression of a particular gene of interest in the preferred cell type or tissue.

Any of a variety of inducible promoters or enhancers can also be included in the vector for expression of a Qpct polypeptide or nucleic acid that can be regulated. Such inducible systems, include, for example, tetracycline inducible System (Gossen & Bizard, Proc. Natl. Acad. Sci. USA, 89:5547-5551 (1992); Gossen et al., Science, 268:17664769 (1995); Clontech, Palo Alto, Calif.); metallothionein promoter induced by heavy metals; insect steroid hormone responsive to ecdysone or related steroids such as muristerone (No et al., Proc. Natl. Acad. Sci. USA, 93:3346-3351 (1996); Yao et al., Nature, 366:476-479 (1993); Invitrogen, Carlsbad, Calif.); mouse mammary tumor virus (MMTV) induced by steroids such as glucocorticoid and estrogen (Lee et al., Nature, 294:228-232 (1981); and heat shock promoters inducible by temperature changes; the rat neuron specific enolase gene promoter (Forss-Petter, et al., Neuron 5; 197-197 (1990)); the human β-actin gene promoter (Ray, et al., Genes and Development (1991) 5:2265-2273); the human platelet derived growth factor B (PDGF-B) chain gene promoter (Sasahara, et al., Cell (1991) 64:217-227); the rat sodium channel gene promoter (Maue, et al., Neuron (1990) 4:223-231); the human copper-zinc superoxide dismutase gene promoter (Ceballos-Picot, et al., Brain Res. (1991) 552:198-214); and promoters for members of the mammalian POU-domain regulatory gene family (Xi et al., (1989) Nature 340:35-42).

Regulatory elements, including promoters or enhancers, can be constitutive or regulated, depending upon the nature of the regulation, and can be regulated in a variety of tissues, or one or a few specific tissues. The regulatory sequences or regulatory elements are operatively linked to one of the polynucleotide sequences of the invention such that the physical and functional relationship between the polynucleotide sequence and the regulatory sequence allows transcription of the polynucleotide sequence. Vectors useful for expression in eukaryotic cells can include, for example, regulatory elements including the CAG promoter, the SV40 early promoter, the cytomegalovirus (CMV) promoter, the mouse mammary tumor virus (MMTV) steroid-inducible promoter, Pgtf, Moloney marine leukemia virus (MMLV) promoter, thy-1 promoter and the like.

If desired, the vector can contain a selectable marker. As used herein, a "selectable marker" refers to a genetic element that provides a selectable phenotype to a cell in which the selectable marker has been introduced. A selectable marker is generally a gene whose gene product provides resistance to an agent that inhibits cell growth or kills a cell. A variety of selectable markers can be used in the DNA constructs of the invention, including, for example, Neo, Hyg, hisD, Gpt and Ble genes, as described, for example in Ausubel et al. (Current Protocols in Molecular Biology (Supplement 47), John Wiley & Sons, New York (1999)) and U.S. Patent No. 5,981,830. Drugs useful for selecting for the presence of a selectable marker include, for example, G418 for Neo, hygromycin for Hyg, histidinol for hisD, xanthine for Gpt, and bleomycin for Ble (see Ausubel et al, supra, (1999); U.S. Patent No. 5,981,830). DNA constructs of the invention can incorporate a positive selectable marker, a negative selectable marker, or both (see, for example, U.S. Patent No. 5,981,830).

### Non-Human Transgenic Animals

The invention primarily provides a non-human transgenic animal whose genome comprises a transgene encoding a Qpct polypeptide. The DNA fragment can be integrated into the genome of a transgenic animal by any method known to those skilled in the art. The DNA molecule containing the desired gene sequence can be introduced into pluripotent cells, such as ES cells, by any method that will permit the introduced molecule to undergo recombination at its regions of homology. Techniques that can be used include, but are not limited to, calcium phosphate/DNA co-precipitates, microinjection of DNA into the nucleus, electroporation, bacterial protoplast fusion with intact cells, transfection, and polycations, (e.g., polybrene, polyornithine, etc.) The DNA can be single or double stranded DNA, linear or circular. (See for example, Hogan et al., Manipulating the Mouse Embryo: A Laboratory Manual Cold Spring Harbor Laboratory (1986); Hogan et al., Manipulating the Mouse Embryo: A Laboratory Manual, second ed., Cold Spring Harbor Laboratory (1994), U.S. Patent Nos. 5,602,299; 5,175,384; 6,066,778; 4,873,191 and 6,037,521; retrovirus mediated gene transfer into germ lines (Van der Putten et al., Proc. Natl. Acad. Sci. USA 82:6148-6152 (1985)); gene targeting in embryonic stem cells (Thompson et al., Cell 56:313-321 (1989)); electroporation of embryos (Lo, Mol Cell. Biol. 3:1803-1814 (1983)); and spermmediated gene transfer (Lavitrano et al., Cell 57:717-723 (1989)).

For example, the zygote is a good target for microinjection, and methods of microinjecting zygotes are well known (see US 4, 873, 191).

Embryonal cells at various developmental stages can also be used to introduce transgenes for the production of transgenic animals. Different methods are used depending on the stage of development of the embryonal cell. Such transfected embryonic stem (ES) cells can thereafter colonize an embryo following their introduction into the blastocoele of a blastocyst-stage embryo and contribute to the germ line of the resulting chimeric animal (reviewed in Jaenisch, Science 240:1468-1474 (1988)). Prior to the introduction of transfected ES cells into the blastocoele, the transfected ES cells can be subjected to various selection protocols to enrich the proportion of ES cells that have integrated the transgene if the transgene provides a means for such selection. Alternatively, PCR can be used to screen for ES cells that have integrated the transgene. In addition, retroviral infection can also be used to introduce transgenes into a non-human animal. The developing non-human embryo can be cultured *in vitro* to the blastocyst stage. During this time, the blastomeres can be targets for retroviral infection (Janenich, Proc. Nati. Acad. Sci. USA 73:1260-1264 (1976)). Efficient infection of the blastomeres is obtained by enzymatic treatment to remove the zona pellucida (Hogan et al., supra, 1986). The viral vector system used to introduce the transgene is typically a replication-defective retrovirus carrying the transgene (Jahner et al., Proc. Natl. Acad Sci. USA 82:6927-6931 (1985); Van der Putten et al., Proc. Natl. Acad Sci. USA 82:6148-6152 (1985)). Transfection is easily and efficiently obtained by culturing the blastomeres on a monolayer of virus-producing cells (Van der Putten, supra, 1985; Stewart et al., EMBO J. 6:383-388 (1987)). Alternatively, infection can be performed at a later stage. Virus or virus-producing cells can be injected into the blastocoele (Jahner D. et al., Nature 298:623-628 (1982)). Most of the founders will be mosaic for the transgene since incorporation occurs only in a subset of cells, which form the transgenic animal. Further, the founder can contain various retroviral insertions of the transgene at different positions in the genome, which generally will segregate in the offspring. In addition, transgenes may be introduced into the germline by intrauterine retroviral infection of the mid-gestation embryo (Jahner et al., supra, 1982). Additional means of using retroviruses or retroviral vectors to create transgenic animals known to those of skill in the art involves the micro-injection of retroviral particles or mitomycin C-treated cells producing retrovirus into the perivitelline space of fertilized eggs or early embryos (WO 90/08832 (1990); Haskell and Bowen, Mal. Reprod. Dev. 40:386 (1995)).

Any other technology to introduce transgenes into a non-human animal, e.g. the knock-in or the rescue technologies can also be used to solve the problem of the present invention. The knock-in technology is well known in the art as described e.g. in Casas et al. (2004) Am J Pathol 165, 1289-1300.

Once the founder animals are produced, they can be bred, inbred, outbred, or crossbred to produce colonies of the particular animal. Examples of such breeding strategies include, but are not limited to: outbreeding of founder animals with more than one integration site in order to establish separate lines; inbreeding of separate lines in order to produce compound transgenics that express the transgene at higher levels because of the effects of additive expression of each transgene; crossing of heterozygous transgenic mice to produce mice homozygous for a given integration site in order to both augment expression and eliminate the need for screening of animals by DNA analysis; crossing of separate homozygous lines to produce compound heterozygous or homozygous lines; breeding animals to different inbred genetic backgrounds so as to examine effects of modifying alleles on expression of the transgene and the effects of expression.

The transgenic animals are screened and evaluated to select those animals having the phenotype of interest. Initial screening can be performed using, for example, Southern blot analysis or PCR techniques to analyze animal tissues to verify that integration of the transgene has taken place. The level of mRNA expression of the transgene in the tissues of the transgenic animals can also be assessed using techniques which include, but are not limited to, Northern blot analysis of tissue samples obtained from the animal, *in situ* hybridization analysis, and reverse transcriptase-PCR (rt-PCR). Samples of the suitable tissues can be evaluated immunocytochemically using antibodies specific for Qpct or with a tag such as EGFP. The transgenic non-human mammals can be further characterized to identify those animals having a phenotype useful in methods of the invention. In particular, transgenic non-human mammals overexpressing Qpct can be screened using the methods disclosed herein. For example, tissue sections can be viewed under a fluorescent microscope for die present of fluorescence, indicating the presence of the reporter gene.

Another method to affect tissue specific expression of the Qpct protein is through the use of tissue-specific promoters. Nonlimiting examples of suitable tissue-specific promoters include the albumin promoter (liver-specific; Pinkert et al., (1987) Genes Dev. 1:268-277); lymphoid-specific promoters (Calame and Eaton (1988) Adv. Immunol. 43:235-275), in particular promoters of T cell receptors (Winoto and Baltimore (1989) EMBO J. 8:729-733) and immunoglobulins (Banerji et al., (1983) Cell 33:729-740; Queen and Baltimore (1983) Cell 33:741-748), neuron-specific promoters (e.g., the neurofilament promoter, the Thy-1 promoter or the Bri-protein promoter; Sturchler-Pierrat et al., (1997) Proc. Natl. Acad Sci. USA 94:13287-13292, Byrne and Ruddle (1989) PNAS 86:5473-5477), pancreas-specific promoters (Edlund et al., (1985) Science 230:912-916), cardiac specific expression (alpha myosin heavy chain promoter, Subramaniam, A, Jones WK, Gulick J, Wert S, Neumann J, and Robbins J. Tissue-specific regulation of the alpha-myosin heavy chain gene promoter in transgenic mice. J Biol Chem 266: 24613-24620, 1991.), and mammary gland-specific promoters (e.g., milk whey promoter; U.S. Patent No. 4,873,316 and European Application Publication No. 264,166).

The invention further provides an isolated cell containing a DNA construct of the invention. The DNA construct can be introduced into a cell by any of the well-known transfection methods (Sambrook et al., Molecular Cloning: A Laboratory Manual, 2nd ed., Cold Spring Harbor Press, Plainview, N.Y. (1989); Ausubel et al., supra, (1999)). Alternatively, the cell can be obtained by isolating a cell from a mutant non-human mammal created as described herein. Thus, the invention provides a cell isolated from a Qpct mutant non-human mammal of the invention, in particular, a Qpct mutant mouse. The cells can be obtained from a homozygous Qpct mutant non-human mammal such as a mouse or a heterozygous Qpct mutant non-human mammal such as a mouse.

### Effectors

Effectors, as that term is used herein, are defined as molecules that bind to enzymes and increase (≙ promote) or decrease (≙ inhibit) their activity *in vitro* and/or *in vivo*. Some enzymes have binding sites for molecules that affect their catalytic activity; a stimulator molecule is called an activator. Enzymes may even have multiple sites for recognizing more than one activator or inhibitor. Enzymes can detect concentrations of a variety of molecules and use that information to vary their own activities.

Effectors can modulate enzymatic activity because enzymes can assume both active and inactive conformations: activators are positive effectors, inhibitors are negative effectors. Effectors act not only at the active sites of enzymes, but also at regulatory sites, or allosteric sites, terms used to emphasize that the regulatory site is an element of the enzyme distinct from the catalytic site and to differentiate this form of regulation from competition between substrates and inhibitors at the catalytic site (Darnell, J., Lodish, H. and Baltimore, D. 1990, Molecular Cell Biology 2"d Edition, Scientific American Books, New York, page 63).

### Peptides

If peptides or amino acids are mentioned in the present invention, each amino acid residue is represented by a one-letter or a three-letter designation, corresponding to the trivial name of the amino acid, in accordance with the following conventional list:

| Amino Acid | One-Letter Symbol | Three-Letter Symbol |
|---|---|---|
| Alanine | A | Ala |
| Arginine | R | Arg |
| Asparagine | N | Asn |
| Aspartic acid | D | Asp |
| Cysteine | C | Cys |
| Glutamine | Q | Gln |
| Glutamic acid | E | Glu |
| Glycine | G | Gly |
| Histidine | H | His |
| Isoleucine | I | Ile |
| Leucine | L | Leu |
| Lysine | K | Lys |
| Methionine | M | Met |
| Phenylalanine | F | Phe |
| Proline | P | Pro |
| Serine | S | Ser |
| Threonine | T | Thr |
| Tryptophan | W | Trp |
| Tyrosine | Y | Tyr |
| Valine | V | Val |

### QC

The terms "QC" or "Qpct" as used herein are both intended to refer to the same and comprise glutaminyl cyclase (QC), i.e.glutaminyl-peptidecyclotransferase (EC 2.3.2.5.) and QC-like enzymes. QC and QC-like enzymes have identical or similar enzymatic activity, further defined as QC activity. In this regard, QC-like enzymes can fundamentally differ in their molecular structure from QC.

The term "QC activity" as used herein is defined as intramolecular cyclization of N-terminal glutamine residues into pyroglutamic acid (pGlu*) or of N-terminal L-homoglutamine or L-ß-homoglutamine to a cyclic pyro-homoglutamine derivative under liberation of ammonia. See schemes 1 and 2.

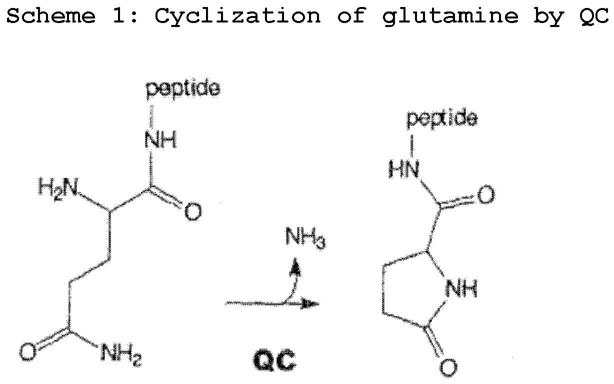

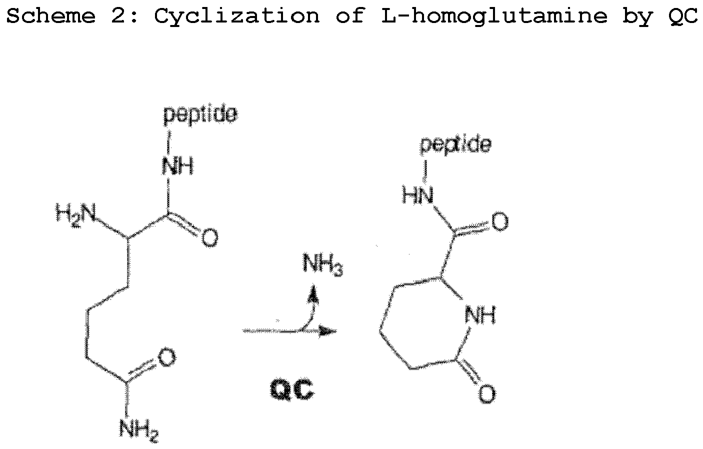

The term "EC" as used herein comprises the side activity of QC and QC-like enzymes as glutamate cyclase (EC), further defined as EC activity.

The term "EC activity" as used herein is defined as intramolecular cyclization of N-terminal glutamate residues into pyroglutamic acid (pGlu*) by QC. See scheme 3.

The term "metal-dependent enzyme" as used herein is defined as enzyme(s) that require a bound metal ion in order to fulfil their catalytic function and/or require a bound metal ion in order to form the catalytically active structure.

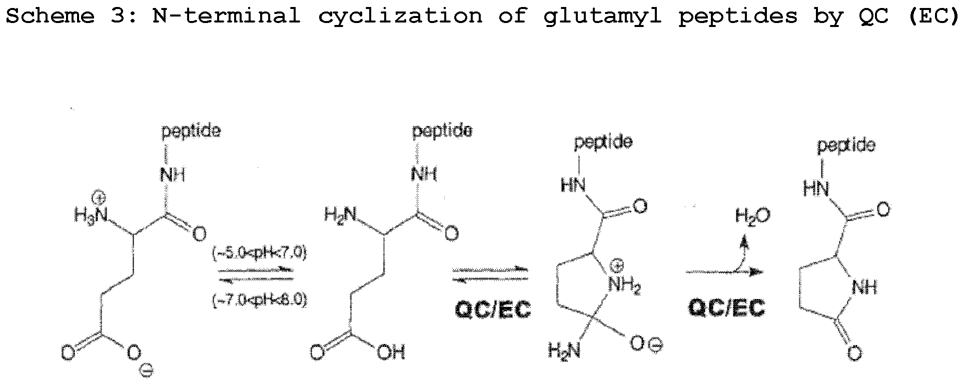

The term "Qpct-related disease" as used herein refers to all those diseases, disorders or conditions that are modulated by Qpct.

### Assays and Identification of Therapeutic Agents

The methods and compositions of the present invention are particularly useful in the evaluation of effectors of Qpct and for the development of drugs and therapeutic agents for the treatment and prevention of amyloid-associated diseases such as Mild Cognitive Impairment, Alzheimer's disease, neurodegeneration in Down Syndrome, Familial Danish Dementia and Familial British Dementia.

The transgenic animal or the cells of the transgenic animal of the invention can be used in a variety of screening assays. For example, any of a variety of potential agents suspected of affecting Qpct and amyloid accumulation, as well as the appropriate antagonists and blocking therapeutic agents, can be screened by administration to the transgenic animal and assessing the effect of these agents upon the function and phenotype of the cells and on the (neurological) phenotype of the transgenic animals.

Behavioral studies may also be used to test potential therapeutic agents, such as those studies designed to assess motor skills, learning and memory deficits. An example of such a test is the Morris Water maze (Morris (1981) Learn Motivat 12:239-260). Additionally, behavioral studies may include evaluations of locomotor activity such as with the rotor-rod and the open field.

The methods of the invention can advantageously use cells isolated from a homozygous or heterozygous Qpct mutant non-human mammal, to study amyloid accumulation as well as to test potential therapeutic compounds. The methods of the invention can also be used with cells expressing Qpct such as a transfected cell line.

A cell overexpressing Qpct can be used in an *in vitro* method to screen compounds as potential therapeutic agents for treating Aβ associated disease. In such a method, a compound is contacted with a cell overexpressing Qpct, a transfected cell or a cell derived from a Qpct mutant non-human animal, and screened for alterations in a phenotype associated with expression of Qpct. The changes in Aβ production in the cellular assay and the transgenic animal can be assessed by methods well known to those skilled in the art.

A Qpct fusion polypeptide such as Qpct can be particularly useful for such screening methods since the expression of Qpct can be monitored by fluorescence intensity. Other exemplary fusion polypeptides include other fluorescent proteins, or modifications thereof, glutathione S transferase (GST), maltose binding protein, poly His, and the like, or any type of epitope tag. Such fusion polypeptides can be detected, for example, using antibodies specific to the fusion polypeptides. The fusion polypeptides can be an entire polypeptide or a functional portion thereof so long as the functional portion retains desired properties, for example, antibody binding activity or fluorescence activity.

The invention further provides a method of identifying a potential therapeutic agent for use in treating the diseases as mentioned above. The method includes the steps of contacting a cell containing a DNA construct comprising polynucleotides encoding a Qpct polypeptide with a compound and screening the cell for decreased Qpct production, thereby identifying a potential therapeutic agent for use in treating Qpct-related diseases. The cell can be isolated from a transgenic non-human mammal having nucleated cells containing the Qpct DNA construct. Alternatively, the cell can contain a DNA construct comprising a nucleic acid encoding a green fluorescent protein fusion, or other fusion polypeptide, with a Qpct polypeptide.

Additionally, cells expressing a Qpct polypeptide can be used in a preliminary screen to identify compounds as potential therapeutic agents having activity that alters a phenotype associated with Qpct expression. As with *in vivo* screens using Qpct mutant non-human mammals, an appropriate control cell can be used to compare the results of the screen. The effectiveness of compounds identified by an initial *in vitro* screen using cells expressing Qpct can be further tested *in vivo* using the invention Qpct mutant non-human mammals, if desired. Thus, the invention provides methods of screening a large number of compounds using a cell-based assay, for example, using high throughput screening, as well as methods of further testing compounds as therapeutic agents in an animal model of Aβ-related disorders.

QC is involved in the formation of pyroglutamic acid that favors the aggregation of amyloid β-peptides. Thus, an inhibition of QC leads to a prevention of the precipitation of the plaque-forming [pGlu³]Aβ3-40/42/43 or [pGlu¹¹]Aβ11-40/42/43, causing the onset and progression of Alzheimer's disease and Down Syndrome, independently of the mechanism by which cyclization occurs.

Glutamate is found in positions 3, 11 and 22 of the amyloid β-peptide. Among them the mutation from glutamic acid (E) to glutamine (Q) in position 22 (corresponds to amino acid 693 of the amyloid precursor protein APP770, Swissprot entry: P05067) has been described as the so-called Dutch type cerebroarterial amyloidosis mutation.

The β-amyloid peptides with a pyroglutamic acid residue in position 3, 11 and/or 22 have been described to be more cytotoxic and hydrophobic than Aβ1-40/4243 (Saido T.C. 2000 Medical Hypotheses 54(3): 427-429).

The multiple N-terminal variations can be generated by the β-secretase enzyme β-site amyloid precursor protein-cleaving enzyme (BACE) at different sites (Huse J.T. et al. 2002 Biol. Chem. 277 (18): 16278-16284), and/or by aminopeptidase processing.

There had been no experimental evidence supporting the enzymatic conversion of Glu¹-peptides into pGlu-peptides by an unknown glutamyl cyclase (EC) (Garden, R. W., Moroz, T. P., Gleeson, J. M., Floyd, P. D., Li, L. J., Rubakhin, S. S., and Sweedler, J. V. (1999) J Neurochem 72, 676-681; Hosoda R. et al. (1998) J Neuropathol Exp Neurol. 57, 1089-1095). No such enzyme activity had been identified, capable of cyclizing Glu¹-peptides, which are protonated N-terminally and possess a negatively charged Glu¹ γ-carboxylate moiety under mildly alkaline or neutral pH-conditions.

QC-activity against Gln¹-substrates is dramatically reduced below pH 7.0. In contrast, it appears that Glu¹-conversion can occur at acidic reaction conditions (e.g. Iwatsubo, T., Saido, T. C., Mann, D. M., Lee, V. M., and Trojanowski, J. Q. (1996) Am J Pathol 149, 1823-1830).

Earlier, it was investigated whether QC is able to recognize and to turnover amyloid-β derived peptides under mildly acidic conditions (WO 2004/098625). Therefore, the peptides [Gln³]Aβ1-11a, Aβ3-11a, [Gln³]Aβ3-11a, Aβ3-21a, [Gln³]Aβ3-21a and [Gln³]Aβ3-40 as potential substrates of the enzyme were synthesized and investigated. These sequences were chosen for mimicking natural N-terminally and C-terminally truncated [Glu³]Aβ peptides and [Gln³]Aβ peptides which could occur due to posttranslational Glu-amidation.

It was shown that papaya and human QC catalyze both glutaminyl and glutamyl cyclization. Apparently, the primary physiological function of QC is to finish hormone maturation in endocrine cells by glutamine cyclization prior or during the hormone secretion process. Such secretory vesicles are known to be acidic in pH. Thus, a side activity of the enzyme in the narrow pH-range from 5.0 to 7.0 could be its newly discovered glutamyl cyclase activity cyclizing also Glu-Aβ peptides. However, due to the much slower occurring Glu-cyclization compared to Gln-conversion, it is questionable whether the glutamyl cyclization plays a significant physiological role. In the pathology of neurodegenerative disorders, however, the glutamyl cyclization is of relevance.

Investigating the pH-dependency of this enzymatic reaction, it has been shown that the unprotonated N-terminus was essential for the cyclization of Gln¹-peptides and accordingly that the pKa-value of the substrate was identical to the pKa-value for QC-catalysis. Thus, QC stabilizes the intramolecular nucleophilic attack of the unprotonated α-amino moiety on the γ-carbonyl carbon.

In contrast to the monovalent charge present on N-terminal glutamine containing peptides, the N-terminal Glu-residue in Glu-containing peptides is predominantly bivalently charged at neutral pH. Glutamate exhibits pkₐ-values of about 4.2 and 7.5 for the γ-carboxylic and for the α-amino moiety, respectively, i.e. at neutral pH and above, although the α-amino nitrogen is in part or fully unprotonated and nucleophilic, the γ-carboxylic group is unprotonated, and so exercising no electrophilic carbonyl activity. Hence, intramolecular cyclization is impossible.

However, in the pH-range of about 5.2-6.5, between their respective pkₐ-values, the two functional groups are present both in non-ionized forms, in concentrations of about 1-10% (-NH₂) or 10-1% (-C00H) of total N-terminal Glu-containing peptide. As a result, over a mildly acidic pH-range species of N-terminal Glu-peptides are present which carry both groups uncharged, and, therefore, it is possible that QC could stabilize the intermediate of intramolecular cyclization into the pGlu-peptide, i.e. if the γ-carboxylic group is protonated, the carbonyl carbon is electrophilic enough to allow nucleophilic attack by the unprotonated α-amino group. At this pH the hydroxyl ion functions as a leaving group. These assumptions are corroborated by the pH-dependence data obtained for the QC catalyzed conversion of Glu-βNA. In contrast to glutamine conversion of Gln-βNA by QC, the pH-optimum of catalysis shifts to the acidic range around pH 6.0, i.e. the pH-range, in which substrate molecule species are simultaneously abundant carrying a protonated γ-carboxyl and unprotonated α-amino group. Furthermore, the kinetically determined pKa-value of 7.55 +/-0.02 is in excellent agreement with that of the α-amino group of Glu-β3NA, determined by titration (7.57 ± 0.05).

Physiologically, at pH 6.0 the second-order rate constant (or specificity constant, k_{Cat}/K_{M}) of the QC-catalyzed glutamate cyclization might be in the range of 1*10⁵ - 1*10⁶ fold slower than the one for glutamine cyclization. However, the nonenzymatic turnover of both model substrates Glu-βNA and Gln-βNA is negligible, being conform with the observed negligible pGlu-peptide formation. Hence, for the pGlu-formation by QC an acceleration of at least 10⁸ can be estimated from the ratio of the enzymatic versus non-enzymatic rate constants (comparing the second-order rate constants for the enzyme catalysis with the respective nonenzymatic cyclization first-order rate constants the catalytic proficiency factor is 10⁹-10¹⁰ M⁻¹ for the Gln- and the Glu-conversion, respectively). The conclusion from these data is, that *in vivo* only an enzymatic path resulting pGlu-formations seems conceivable.

Since QC is highly abundant in the brain and taking into account the high turnover rate of 0.9 min⁻¹ recently found for the maturation of 30 µM of (Gln-)TRH-like peptide (Prokal, L., Prokai-Tatrai, K., Ouyang, X., Kim, H. S., Wu, W. M., Zharikova, A., and Bodor, N. (1999) J Med Chem 42, 4563-4571), one can predict a cyclization half-life of about 100 hours for an appropriate glutamate-substrate, if similar reaction conditions are provided. Moreover, given compartmentalization and localization of brain QC/EC in the secretory pathway, the actual *in vivo* enzyme and substrate concentrations and reaction conditions might be even more favorable for the enzymatic cyclization in the intact cell. And, if N-terminal Glu is transformed to Gln a much more rapid pGlu-formation mediated by QC could be expected. *In vitro*, both reactions were suppressed by applying inhibitors of QC/EC-activity.

In summary, it was shown that human QC, which is highly abundant in the brain, is likely a catalyst of the formation of the amyloidogenic pGlu-Aβ peptides from Glu-Aβ and Gln-Aβ precursors, which make up more than 50% of the plaque deposits found in Alzheimer's disease. These findings identify QC/EC as a player in senile plaque formation and thus as a novel drug target in the treatment of Alzheimer's disease, neurodegeneration in Down Sydrome, Famlilial Danish Dementia and Familial British Dementia.

In a preferred embodiment, the present invention provides the use of activity-decreasing effectors of QC and EC, as selected with use of the present inventive animal model, for the suppression of pGlu-Amyloid peptide formation in Mild Cognitive Impairment, Alzheimer's disease, Down Sydrome, Famlilial Danish Dementia and Familial British Dementia.

In a further embodiment, the present invention provides the use of activity-increasing effectors of QC, as selected with use of the present inventive animal model, for the stimulation of gastrointestinal tract cell proliferation, especially gastric mucosal cell proliferation, epithelial cell proliferation, the differentiation of acid-producing parietal cells and histamine-secreting enterochromaffin-like (ECL) cells, and the expression of genes associated with histamine synthesis and storage in ECL cells, as well as for the stimulation of acute acid secretion in mammals by maintaining or increasing the concentration of active[pGlu¹-]-Gastrin.

In a further embodiment, the present invention provides the use of activity decreasing effectors of QC, as selected with use of the present inventive animal model, for the treatment of duodenal ulcer disease and gastric cancer with or without Helicobacter pylori in mammals by decreasing the conversion rate of inactive [Gln¹]Gastrin to active [pGlu¹]Gastrin.

Neurotensin (NT) is a neuropeptide implicated in the pathophysiology of schizophrenia that specifically modulates neurotransmitter systems previously demonstrated to be misregulated in this disorder. Clinical studies in which cerebrospinal fluid (CSF) NT concentrations have been measured revealed a subset of schizophrenic patients with decreased CSF NT concentrations that are restored by effective antipsychotic drug treatment. Considerable evidence also exists concordant with the involvement of NT systems in the mechanism of action of antipsychotic drugs. The behavioural and biochemical effects of centrally administered NT remarkably resemble those of systemically administered antipsychotic drugs, and antipsychotic drugs increase NT neurotransmission. This concatenation of findings led to the hypothesis that NT functions as an endogenous antipsychotic. Moreover, typical and atypical antipsychotic drugs differentially alter NT neurotransmission in nigrostriatal and mesolimbic dopamine terminal regions, and these effects are predictive of side effect liability and efficacy, respectively (Binder, E. B. et al. 2001 Biol Psychiatry 50 856-872).

In another embodiment, the present invention provides the use of activity increasing effectors of QC, as selected with use of the present inventive animal model, for the preparation of antipsychotic drugs and/or for the treatment of schizophrenia in mammals. The effectors of QC either maintain or increase the concentration of active [pGlu¹]neurotensin.

Fertilization promoting peptide (FPP), a tripeptide related to thyrotrophin releasing hormone (TRH), is found in seminal plasma. Recent evidence obtained *in vitro* and *in vivo* showed that FPP plays an important role in regulating sperm fertility. Specifically, FPP initially stimulates nonfertilizing (incapacitated) spermatozoa to "switch on" and become fertile more quickly, but then arrests capacitation so that spermatozoa do not undergo spontaneous acrosome loss and therefore do not lose fertilizing potential. These responses are mimicked, and indeed augmented, by adenosine, known to regulate the adenylyl cyclase (AC)/cAMP signal transduction pathway. Both FPP and adenosine have been shown to stimulate cAMP production in incapacitated cells but inhibit it in capacitated cells, with FPP receptors somehow interacting with adenosine receptors and G proteins to achieve regulation of AC. These events affect the tyrosine phosphorylation state of various proteins, some being important in the initial "switching on", and others possibly being involved in the acrosome reaction itself. Calcitonin and angiotensin II, also found in seminal plasma, have similar effects *in vitro* on incapacitated spermatozoa and can augment responses to FPP. These molecules have similar effects *in vivo*, affecting fertility by stimulating and then maintaining fertilizing potential. Either reductions in the availability of FPP, adenosine, calcitonin, and angiotensin II or defects in their receptors contribute to male infertility (Fraser, L.R. and Adeoya-Osiguwa, S. A. 2001 Vitam Horm 63, 1-28).

In a further embodiment, the present invention provides the use of activity-lowering effectors of QC, as selected with the present inventive animal model, for the preparation of fertilization prohibitive drugs and/or to reduce the fertility in mammals. The activity lowering effectors of QC decrease the concentration of active [pGlu¹] FPP, leading to a prevention of sperm capacitation and deactivation of sperm cells. In contrast it could be shown that activity-increasing effectors of QC are able to stimulate fertility in males and to treat infertility.

In another embodiment, the present invention provides the use of effectors of QC, as selected with use of the present inventive animal model, for the preparation of a medicament for the treatment of pathophysiological conditions, such as suppression of proliferation of myeloid progenitor cells, neoplasia, inflammatory host responses, cancer, malign metastasis, melanoma, psoriasis, rheumatoid arthritis, atherosclerosis, lung fibrosis, liver fibrosis, renal fibrosis, graft rejection, acquired immune deficiency syndrom, impaired humoral and cell-mediated immunity responses, leukocyte adhesion and migration processes at the endothelium.

In a further embodiment, the present invention provides the use of effectors of QC, as selected with use of the present inventive animal model, for the preparation of a medicament for the treatment of impaired food intake and sleep-wakefulness, impaired homeostatic regulation of energy metabolism, impaired autonomic function, impaired hormonal balance and impaired regulation of body fluids.

Polyglutamine expansions in several proteins lead to neurodegenerative disorders, such as Chorea Huntington, Parkinson disease and Kennedy's disease. The mechanism therefore remains largely unknown. The biochemical properties of polyglutamine repeats suggest one possible explanation: endolytic cleavage at a glutaminyl-glutaminyl bond followed by pyroglutamate formation may contribute to the pathogenesis through augmenting the catabolic stability, hydrophobicity, amyloidogenicity, and neurotoxicity of the polyglutaminyl proteins (Saido, T.C.; Med Hypotheses (2000) Mar; 54(3):427-9).

In a further embodiment, the present invention therefore provides the use of effectors of QC, as selected with the present inventive animal model, for the preparation of a medicament for the treatment of Parkinson disease and Huntington's disease.

In another embodiment, the present invention provides a general way to reduce or inhibit the enzymatic activity of QC by using the test agent selected above.

Inhibition of a mammalian QC was only detected initially for 1,10-phenanthroline and reduced 6-methylpterin (Busby, W. H. J. et al. 1987 J Biol Chem 262, 8532-8536). EDTA did not inhibit QC, thus it was concluded that QC is not a metal-dependent enzyme (Busby, W. H. J. et al, 1987 J Biol Chem 262, 8532-8536, Bateman, R.C.J. et al. 2001 Biochemistry 40, 11246-11250, Booth, R.E. et al. 2004 BMC Biology 2). However, it was shown, that human QC and other animal QCs are metal-dependent enzymes, as revealed by the inhibition characteristics of QC by 1,10-phenanthroline, dipicolinic acid, 8-hydroxy-quinoline and other chelators and by the reactivation of QC by transition metal ions. Finally, the metal dependence is outlined by a sequence comparison to other metal-dependent enzymes, showing a conservation of the chelating amino acid residues also in human QC. The interaction of compounds with the active-site bound metal ion represents a general way to reduce or inhibit QC activity.

Preferred for the use in the above-described screening methods are mammalian QC, in particular human or murine QC, or Papaya QC. Especially preferred is mammalian QC, since the effectors identified by these screening methods shall be used for the treatment of diseases in mammals, especially in humans.

Also provided are non-human transgenic animals wherein the transgene encodes an isoenzyme of Qpct.

These isoenzymes having significant sequence similarity to glutaminyl cyclase are proteins (Qpctl's) from human (further named as human isoQC) (GenBank accession no. NM_017659), mouse (GenBank accession no. NM_027455), Macaca fascicularis (GenBank accession no. AB168255), Macaca mulatta (GenBank accession no. XM_001110995), cat (GenBank accession no. XM_541552), rat (GenBank accession no. XM_001066591), cow (GenBank accession no. BT026254) or an analogue thereof having at least 50% / 75% sequence identity / similarity, preferably 70% / 85% sequence identity / similarity, most preferably 90% / 95% sequence identity / similarity.

The sequences are given in SEQ. ID NOS: 15 to 25. Further disclosed are nucleic acid sequences coding for these proteins (SEQ. ID NOS: 26 to 36).

Preferred according to the present invention are Qpctl's selected from the group consisting of human Qpctl including isoforms and spliceforms thereof, given in SEQ. ID NOS: 15 to 17, 24 and 25; rat (SEQ. ID NO: 21) and mouse (SEQ. ID NO: 22).

More preferred according to the present invention are Qpctl's selected from the group consisting of human Qpctl including isoforms, given in SEQ. ID NOS: 15 to 17; and mouse (SEQ. ID NO: 22).

Most preferred according to the present invention are Qpctl's selected from the group consisting of human (SEQ. ID NO: 15), and mouse (SEQ. ID NO: 22).

In this regard, specific reference is made to US 60/846,244 for specific further disclosure of the Qpct-isoenzymes. This application is incorporated herein by reference.

The agents selected by the above-described screening methods can work by decreasing the conversion of at least one substrate of QC (negative effectors, inhibitors), or by increasing the conversion of at least one substrate of QC (positive effectors, activators).

The compounds of the present invention can be converted into acid addition salts, especially pharmaceutically acceptable acid addition salts.

The salts of the compounds of the invention may be in the form of inorganic or organic salts.

The compounds of the present invention can be converted into and used as acid addition salts, especially pharmaceutically acceptable acid addition salts. The pharmaceutically acceptable salt generally takes a form in which a basic side chain is protonated with an inorganic or organic acid. Representative organic or inorganic acids include hydrochloric, hydrobromic, perchloric, sulfuric, nitric, phosphoric, acetic, propionic, glycolic, lactic, succinic, maleic, fumaric, malic, tartaric, citric, benzoic, mandelic, methanesulfonic, hydroxyethanesulfonic, benzenesulfonic, oxalic, pamoic, 2-naphthalenesulfonic, p-toluenesulfonic, cyclohexanesulfamic, salicylic, saccharinic or trifluoroacetic acid. All pharmaceutically acceptable acid addition salt forms of the compounds of the present invention are intended to be embraced by the scope of this invention.

In view of the close relationship between the free compounds and the compounds in the form of their salts, whenever a compound is referred to in this context, a corresponding salt is also intended, provided such is possible or appropriate under the circumstances.

Where the compounds according to this invention have at least one chiral center, they may accordingly exist as enantiomers. Where the compounds possess two or more chiral centers, they may additionally exist as diastereomers. It is to be understood that all such isomers and mixtures thereof are encompassed within the scope of the present invention. Furthermore, some of the crystalline forms of the compounds may exist as polymorphs and as such are intended to be included in the present invention. In addition, some of the compounds may form solvates with water (i.e. hydrates) or common organic solvents, and such solvates are also intended to be encompassed within the scope of this invention.

The compounds, including their salts, can also be obtained in the form of their hydrates, or include other solvents used for their crystallization.

In a further embodiment, the present invention provides a method of preventing or treating a condition mediated by modulation of the QC enzyme activity in a subject in need thereof which comprises administering any of the compounds of the present invention or pharmaceutical compositions thereof in a quantity and dosing regimen therapeutically effective to treat the condition. Additionally, the present invention includes the use of the compounds of this invention, and their corresponding pharmaceutically acceptable acid addition salt forms, for the preparation of a medicament for the prevention or treatment of a condition mediated by modulation of the QC activity in a subject. The compound may be administered to a patient by any conventional route of administration, including, but not limited to, intravenous, oral, subcutaneous, intramuscular, intradermal, parenteral and combinations thereof.

In a further preferred form of implementation, the invention relates to pharmaceutical compositions, that is to say, medicaments, that contain at least one compound of the invention or salts thereof, optionally in combination with one or more pharmaceutically acceptable carriers and/or solvents.

The pharmaceutical compositions may, for example, be in the form of parenteral or enteral formulations and contain appropriate carriers, or they may be in the form of oral formulations that may contain appropriate carriers suitable for oral administration. Preferably, they are in the form of oral formulations.

The effectors of QC activity administered according to the invention may be employed in pharmaceutically administrable formulations or formulation complexes as inhibitors or in combination with inhibitors, substrates, pseudosubstrates, inhibitors of QC expression, binding proteins or antibodies of those enzyme proteins that reduce the QC protein concentration in mammals. The compounds of the invention make it possible to adjust treatment individually to patients and diseases, it being possible, in particular, to avoid individual intolerances, allergies and side-effects.

The compounds also exhibit differing degrees of activity as a function of time. The physician providing treatment is thereby given the opportunity to respond differently to the individual situation of patients: he is able to adjust precisely, on the one hand, the speed of the onset of action and, on the other hand, the duration of action and especially the intensity of action.

A preferred treatment method according to the invention represents a new approach for the prevention or treatment of a condition mediated by modulation of the QC enzyme activity in mammals. It is advantageously simple, susceptible of commercial application and suitable for use, especially in the treatment of diseases that are based on unbalanced concentration of physiological active QC substrates in mammals and especially in human medicine.

The compounds may be advantageously administered, for example, in the form of pharmaceutical preparations that contain the active ingredient in combination with customary additives like diluents, excipients and/or carriers known from the prior art. For example, they can be administered parenterally (for example i*.v.* in physiological saline solution) or enterally (for example orally, formulated with customary carriers).

Depending on their endogenous stability and their bioavailability, one or more doses of the compounds can be given per day in order to achieve the desired normalisation of the blood glucose values. For example, such a dosage range in humans may be in the range of from about 0.01 mg to 250.0 mg per day, preferably in the range of about 0.01 to 100 mg of compound per kilogram of body weight.

By administering effectors of QC activity to a mammal it could be possible to prevent or alleviate or treat conditions selected from Mild Cognitive Impairment, Alzheimer's disease, Down Syndrome, Familial Danish Dementia, Familial British Dementia, Huntington's Disease, ulcer disease and gastric cancer with or w/o *Helicobacter* pylori infections, pathogenic psychotic conditions, schizophrenia, infertility, neoplasia, inflammatory host responses, cancer, psoriasis, rheumatoid arthritis, atherosclerosis, restenosis, lung fibrosis, liver fibrosis, renal fibrosis, graft rejection, acquired immune deficiency syndrome, impaired humoral and cell-mediated immune responses, leukocyte adhesion and migration processes in the endothelium, impaired food intake, sleep-wakefulness, impaired homeostatic regulation of energy metabolism, impaired autonomic function, impaired hormonal balance and impaired regulation of body fluids.

Further, by administering effectors of QC activity to a mammal it could be possible to stimulate gastrointestinal tract cell proliferation, preferably proliferation of gastric mucosal cells, epithelial cells, acute acid secretion and the differentiation of acid producing parietal cells and histamine-secreting enterochromaffin-like cells.

In addition, administration of QC inhibitors to mammals may lead to a loss of sperm cell function thus suppressing male fertility. Thus, the prevent invention provides a method for the regulation and control of male fertility and the use of activity lowering effectors of QC for the preparation of contraceptive medicaments for males.

Furthermore, by administering effectors of QC activity to a mammal it may be possible to suppress the proliferation of myeloid progenitor cells.

The compounds used according to the invention can accordingly be converted in a manner known per se into conventional formulations, such as, for example, tablets, capsules, drag6es, pills, suppositories, granules, aerosols, syrups, liquid, solid and cream-like emulsions and suspensions and solutions, using inert, non-toxic, pharmaceutically suitable carriers and additives or solvents. In each of those formulations, the therapeutically effective compounds are preferably present in a concentration of approximately from 0.1 to 80 % by weight, more preferably from 1 to 50 % by weight, of the total mixture, that is to say, in amounts sufficient for the mentioned dosage latitude to be obtained.

The substances can be used as medicaments in the form of dragées, capsules, bitable capsules, tablets, drops, syrups or also as suppositories or as nasal sprays.

The formulations may be advantageously prepared, for example, by extending the active ingredient with solvents and/or carriers, optionally with the use of emulsifiers and/or dispersants, it being possible, for example, in the case where water is used as diluent, for organic solvents to be optionally used as auxiliary solvents.

Examples of excipients useful in connection with the present invention include: water, non-toxic organic solvents, such as paraffins (for example natural oil fractions), vegetable oils (for example rapeseed oil, groundnut oil, sesame oil), alcohols (for example ethyl alcohol, glycerol), glycols (for example propylene glycol, polyethylene glycol); solid carriers, such as, for example, natural powdered minerals (for example highly dispersed silica, silicates), sugars (for example raw sugar, lactose and dextrose); emulsifiers, such as non-ionic and anionic emulsifiers (for example polyoxyethylene fatty acid esters, polyoxyethylene fatty alcohol ethers, alkylsulphonates and arylsulphonates), dispersants (for example lignin, sulphite liquors, methylcellulose, starch and polyvinylpyrrolidone) and lubricants (for example magnesium stearate, talcum, stearic acid and sodium lauryl sulphate) and optionally flavourings. Administration may be carried out in the usual manner, preferably enterally or parenterally, especially orally. In the case of enteral administration, tablets may contain in addition to the mentioned carriers further additives such as sodium citrate, calcium carbonate and calcium phosphate, together with various additives, such as starch, preferably potato starch, gelatin and the like. Furthermore, lubricants, such as magnesium stearate, sodium lauryl sulphate and talcum, can be used concomitantly for tabletting. In the case of aqueous suspensions and/or elixirs intended for oral administration, various taste correctives or colourings can be added to the active ingredients in addition to the above-mentioned excipients.

In the case of parenteral administration, solutions of the active ingredients using suitable liquid carriers can be employed. In general, it has been found advantageous to administer, in the case of intravenous administration, amounts of approximately from 0.01 to 2.0 mg/kg, preferably approximately from 0.01 to 1.0 mg/kg, of body weight per day to obtain effective results and, in the case of enteral administration, the dosage is approximately from 0.01 to 2 mg/kg, preferably approximately from 0.01 to 1 mg/kg, of body weight per day.

It may nevertheless be necessary in some cases to deviate from the stated amounts, depending upon the body weight of the experimental animal or the patient or upon the type of administration route, but also on the basis of the species of animal and its individual response to the medicament or the interval at which administration is carried out. Accordingly, it may be sufficient in some cases to use less than the above-mentioned minimum amount, while, in other cases, the mentioned upper limit will have to be exceeded. In cases where relatively large amounts are being administered, it may be advisable to divide those amounts into several single doses over the day. For administration in human medicine, the same dosage latitude is provided. The above remarks apply analogously in that case.

For examples of pharmaceutical formulations, specific reference is made to the examples of WO 2004/098625, pages 50-52, which are incorporated herein by reference in their entirety.

The above disclosure describes the present invention in general. A more complete understanding can be obtained by reference to the following examples. These examples are described solely for purposes of illustration and are not intended to limit the scope of the invention. Although specific terms have been employed herein, such terms are intended in a descriptive sense and not for purposes of limitation.

### Reference Example 1: Preparation of Human and Papaya QC

### Host strains and media

*Pichia pastoris* strain X33 (AOX1, AOX2), used for the expression of human QC was grown, transformed and analyzed according to the manufacturer's instructions (Invitrogen). The media required for *P. pastoris,* i.e. buffered glycerol (BMGY) complex or methanol (BMMY) complex medium, and the fermentation basal salts medium were prepared according to the manufacturer's recommendations.

### Molecular cloning of plasmid vectors encoding the human QC

All cloning procedures were done applying standard molecular biology techniques. For expression in yeast, the vector pPIC2ocB (Invitrogen) was used. The pQE-31 vector (Qiagen) was used to express the human QC in *E*. *coli.* The cDNA of the mature QC starting with codon 38 was fused in frame with the plasmid encoded 6xhistidine tag. After amplification utilizing the primers pQCyc-1 and pQCyc-2 (WO 2004/098625) and subcloning, the fragment was inserted into the expression vector employing the restriction sites of SphI and HindIII.

### Transformation of P. pastoris and mini-scale expression

Plasmid DNA was amplified in *E*. *coli* JM109 and purified according to the recommendations of the manufacturer (Qiagen). In the expression plasmid used, pPICZαB, three restriction sites are provided for linearization. Since SacI and BstXI cut within the QC cDNA, PmeI was chosen for linearization. 20-30 µg plasmid DNA was linearized with PmeI, precipitated by ethanol, and dissolved in sterile, deionized water. 10 µg of the DNA was then applied for transformation of competent *P*. *pastoris* cells by electroporation according to the manufacturer's instructions (BioRad). Selection was done an plates containing 150 µg/ml Zeocin. One transformation using the linearized plasmid yielded several hundred transformants.

In order to test the recombinant yeast clones for QC expression, recombinants were grown for 24 h in 10 ml conical tubes containing 2 ml BMGY. Afterwards, the yeast was centrifuged and resuspended in 2 ml BMMY containing 0.5 % methanol. This concentration was maintained by addition of methanol every 24 h up to 72 h. Subsequently, QC activity in the supernatant was determined. The presence of the fusion protein was confirmed by western blot analysis using an antibody directed against the 6xhistidine tag (Qiagen). Clones that displayed the highest QC activity were chosen for further experiments and fermentation.

### Large-scale expression in a fermenter

Expression of the QC was performed in a 5 1 reactor (Biostat B, B. Braun biotech), essentially as described in the *"Pichia* fermentation process guidelines" (Invitrogen). Briefly, the cells were grown in the fermentation basal salts medium supplemented with trace salts, and with glycerol as the sole carbon source (pH 5.5). During an initial batch phase for about 24 h and a subsequent fed-batch phase for about 5 h, cell mass was accumulated. Once a cell wet weight of 200 g/l was achieved, induction of QC expression was performed using methanol applying a three-step feeding profile for an entire fermentation time of approximately 60 h. Subsequently, cells were removed from the QC-containing supernatant by centrifugation at 6000xg, 4°C for 15 min. The pH was adjusted to 6.8 by addition of NaOH, and the resultant turbid solution was centrifuged again at 37000xg, 4°C for 40 min. In cases of continued turbidity, an additional filtration step was applied using a cellulose membrane (pore width 0.45 µm).

Purification of 6 x histidine tagged QC expressed in *P*. *pastoris*

The His-tagged QC was first purified by immobilized metal affinity chromatography (IMAC). In a typical purification, 1000 ml of culture supernatant were applied to a Ni²⁺-loaded Chelating Sepharose FF column (1.6 x 20 cm, Pharmacia), that was equilibrated with 50 mM phosphate buffer, pH 6.8, containing 750 mM NaCl, at a flow rate of 5 ml/min. After washing with 10 column volumes of equilibration buffer and 5 column volumes of equilibration buffer containing 5 mM histidine, the bound protein was eluted by a shift to 50 mM phosphate buffer, pH 6.8, containing 150 mM NaCl and 100 mM histidine. The resulting eluate was dialyzed against 20 mM Bis-Tris/HCl, pH 6.8, at 4°C overnight. Subsequently, the QC was further purified by anion exchange chromatography an a Mono Q6 column (BioRad), equilibrated with dialysis buffer. The QC-containing fraction was loaded onto the column using a flow rate of 4 ml/min. The column was then washed with equilibration buffer containing 100 mM NaCl. The elution was performed by two gradients resulting in equilibration buffer containing 240 mM and 360 mM NaCl in 30 or 5 column volumes, respectively. Fractions of 6 ml were collected and the purity was analyzed by SDS-PAGE. Fractions containing homogenous QC were pooled and concentrated by ultrafiltration. For long-term storage (-20°C), glycerol was added to a final concentration of 50 %. Protein was quantified according to the methods of Bradford or Gill and von Hippel (Bradford, M. M. 1976 Anal Biochem 72, 248-254; Gill, S.C. and von Hippel, P.H. 1989 Anal Biochem 182, 319-326.).

### Expression and purification of QC in E. coli

The construct encoding the QC was transformed into M15 cells (Qiagen) and grown an selective LB agar plates at 37°C. Protein

expression was carried out in LB medium containing 1 % glucose and 1 % ethanol at room temperature. When the culture reached an OD₆₀₀ of approximately 0.8, expression was induced with 0,1 mM IPTG overnight. After one cycle of freezing and thawing, cells were lysed at 4°C by addition of 2.5 mg/ml lysozyme in 50 mM phosphate buffer, pH 8.0, containing 300 mM NaCl and 2 mM histidine for approximately 30 min. The solution was clarified by centrifugation at 37000 xg, 4 °C for 30 min, followed by a filtration applying a glass frit (DNA separation) and two additional filtration steps applying cellulose filters for crude and fine precipitates. The supernatant (approx. 500 ml) was applied onto a Ni²⁺-affinity column (1.6 x 20 cm) at a flow rate of 1 ml/min. Elution of QC was carried out with 50 mM phosphate buffer containing 150 mM NaCl and 100 mM histidine. The QC-containing fraction was concentrated by ultrafiltration.

### Purification of QC from papaya latex

QC from papaya latex was prepared using the BioCAD 700E (Perseptive Biosystems, Wiesbaden, Germany) with a modified version of a previously reported method (Zerhouni, S. et al. 1989 Biochim Biophys Acta 138, 275-290). 50 g latex was dissolved in water and centrifugated as described therein. Inactivation of proteases was performed with S-methyl methane thiosulfonate, and the resultant crude extract was dialyzed. After dialysis, the entire supernatant was loaded onto a (21X2.5 cm i.d.) SP Sepharose Fast Flow column, equilibrated with 100 mM sodium acetate buffer, pH 5.0 (flow rate 3 ml/min). Elution was performed in three steps by increasing sodium acetate buffer concentration at a flow rate of 2 ml/min. The first step was a linear gradient from 0.1 to 0.5 M acetate buffer in 0.5 column volumes. The second step was a linear increase in buffer concentration from 0.5 to 0.68 M in four column volumes. During the last elution step, one column volume of 0.85 M buffer was applied. Fractions (6 ml) containing the highest enzymatic activity were pooled. Concentration and buffer changes to 0.02 M Tris/HCI, pH 8.0 were performed via ultrafiltration (Amicon; molecular mass cut-off of the membrane 10 kDa).

Ammonium sulfate was added to the concentrated papaya enzyme, obtained from the ion exchange chromatography step to a final concentration of 2 M. This solution was applied onto a (21X2.5 cm i.d.) Butyl Sepharose 4 Fast Flow column (flow rate 1.3 ml/min), equilibrated with 2 M ammonium sulfate, 0,02 M Tris/HCl, pH 8.0. Elution was performed in three steps with decreasing concentrations of ammonium sulfate. During the first step a linear gradient from 2 to 0.6 M ammonium sulfate, 0.02 M Tris/HCl, pH 8.0 was applied for 0.5 column volumes at a flow rate of 1.3 ml/min. The second step was a linear gradient from 0.6 to 0 M ammonium sulfate, 0.02 M Tris/HCl, pH 8.0, in 5 column volumes at a flow rate of 1.5 ml/min. The last elution step was carried out by applying 0.02 M Tris/HCl at pH 8.0 for 2 column volumes at a flow rate of 1.5 ml/min. All fractions containing QC activity were pooled and concentrated by ultrafiltration. The resultant homogenous QC was stored at - 70°C. Final protein concentrations were determined using the method of Bradford, compared to a standard curve obtained with bovine serum albumin.

The cDNA sequence of human Qpct is shown in SEQ ID No:12, while the protein sequence is shown in SEQ ID No: 13.

### Reference Example 2: Assays for glutaminyl cyclase activity

### Fluorometric assays

Measurements were performed with a BioAssay Reader HTS-7000Plus (Perkin Eimer) or a NovoStar (BMG Labtechnologies) reader for microplates at 30°C. QC activity was evaluated fluorometrically using H-Gln-βNA. The samples consisted of 0.2 mM fluorogenic substrate, 0.25 U pyroglutamyl aminopeptidase (Unizyme, Horsholm, Denmark) in 0.2 M Tris/HCl, pH 8.0 containing 20 mM EDTA and an appropriately diluted aliquot of QC in a final volume of 250 µl. Excitation/emission wavelengths were 320/410 nm. The assay reactions were initiated by addition of glutaminyl cyclase. QC activity was determined from a standard curve of β-naphthylamine under assay conditions. One unit is defined as the amount of QC catalyzing the formation of 1 µmol pGlu-βNA from H-Gln-βNA per minute under the described conditions.

In a second fluorometric assay, QC was activity was determined using H-Gln-AMC as substrate. Reactions were carried out at 30°C utilizing the NOVOStar reader for microplates (BMG labtechnologies). The samples consisted of varying concentrations of the fluorogenic substrate, 0.1 U pyroglutamyl aminopeptidase (Qiagen) in 0.05 M Tris/HCI, pH 8.0 containing 5 mM EDTA and an appropriately diluted aliquot of QC in a final volume of 250 µl. Excitation/emission wavelengths were 380/460 nm. The assay reactions were initiated by addition of glutaminyl cyclase. QC activity was determined from a standard curve of 7-amino-4-methylcoumarin under assay conditions. The kinetic data were evaluated using GraFit sofware.

### Spectrophotometric assay of QC

This novel assay was used to determine the kinetic parameters for most of the QC substrates. QC activity was analyzed spectrophotometrically using a continuous method, that was derived by adapting a previous discontinuous assay (Bateman, R. C. J. 1989 J Neurosci Methods 30, 23-28) utilizing glutamate dehydrogenase as auxiliary enzyme. Samples consisted of the respective QC substrate, 0.3 mM NADH, 14 mM α-ketoglutaric acid and 30 U/ml glutamate dehydrogenase in a final volume of 250 µl. Reactions were started by addition of QC and pursued by monitoring of the decrease in absorbance at 340 nm for 8-15 min.

The initial velocities were evaluated and the enzymatic activity was determined from a standard curve of ammonia under assay conditions. All samples were measured at 30°C, using either the SPECTRAFIuor Plus or the Sunrise (both from TECAN) reader for microplates. Kinetic data was evaluated using GraFit software.

### Inhibitor assay

For inhibitor testing, the sample composition was the same as described above, except for the putative inhibitory compound added. For a rapid test of QC-inhibition, samples contained 4 mM of the respective inhibitor and a substrate concentration at 1 K_{M}. For detailed investigations of the inhibition and determination of Kᵢ-values, influence of the inhibitor on the auxiliary enzymes was investigated first. In every case, there was no influence on either enzyme detected, thus enabling the reliable determination of the QC inhibition. The inhibitory constant was evaluated by fitting the set of progress curves to the general equation for competitive inhibition using GraFit software.

### Reference Example 3: MALDI-TOF mass spectrometry

Matrix-assisted laser desorption/ionization mass spectrometry was carried out using the Hewlett-Packard G2025 LD-TOF System with a linear time of flight analyzer. The instrument was equipped with a 337 nm nitrogen laser, a potential acceleration source (5 kV) and a 1.0 m flight tube. Detector operation was in the positive-ion mode and signals were recorded and filtered using LeCroy 9350M digital storage oscilloscope linked to a personal computer. Samples (5 µl) were mixed with equal volumes of the matrix solution. For matrix solution we used DHAP/DAHC, prepared by solving 30 mg 2',6'-dihydroxyacetophenone (Aldrich) and 44 mg diammonium hydrogen citrate (Fluka) in 1 ml acetonitrile/0.1% TFA in water (1/1, v/v). A small volume (≈ 1 µl) of the matrix-analyte-mixture was transferred to a probe tip and immediately evaporated in a vacuum chamber (Hewlett-Packard G2024A sample prep accessory) to ensure rapid and homogeneous sample crystallization.

For long-term testing of Glu¹-cyclization, Aβ-derived peptides were incubated in 100 µl 0.1 M sodium acetate buffer, pH 5.2 or 0.1 M Bis-Tris buffer, pH 6.5 at 30°C. Peptides were applied in 0.5 mM [Aβ3-11 a] or 0.15 mM [Aβ3-21a] concentrations, and 0.2 U QC was added all 24 hours. In case of Aβ3-21a, the assays contained 1 % DMSO. At different times, samples were removed from the assay tube, peptides extracted using ZipTips (Millipore) according to the manufacturer's recommendations, mixed with matrix solution (1:1 1 v/v) and subsequently the mass spectra recorded. Negative controls contained either no QC or heat deactivated enzyme. For the inhibitor studies the sample composition was the same as described above, with exception of the inhibitory compound added (5 mM benzimidazole or 2 mM 1,10-phenanthroline).

### Reference Example 4: murine Qpct

Murine Qpct was isolated from murine insulinoma cell line β-TC 3 by RT-PCR using primers derived from a putative murine QC cDNA, which was deposited in the nucleotide database as entry AK017598 and sub-cloned into vector pPCR Script CAM SK(+) (Schilling S. et al.; Biochemistry 44(40) 13415-13424). Murine Qpct as used in the following examples is comprised in a construct, i.e. a gene cassette (SEQ ID No: 9) ; the amino acid sequence is shown in SEQ ID No:10.

### Cloning of murine QC

The primers for isolation of the open reading frame of mQC were designed using PubMed nucleotide entry AK017598, encoding the putative mQC. The primer sequences were as follows: sense 5' ATATGCATGCATGGCAGGCAGCGAAGACAAGC (SEQ ID NO:11); antisense 5' ATATAAGCTTTTACAAGTGAAGATATTCCAACACAAAGAC (SEQ ID NO:12). Total RNA was isolated from murine insulinoma cell line β-TC 3 cells using the RNeasy Mini Kit (Qiagen) and reversely transcribed by SuperScriptII (Invitrogen). Subsequently, mQC cDNA was amplified on a 1:12.5 dilution of generated product in a 50 µl reaction with Herculase Enhanced DNA-Polymerase (Stratagene), inserted into the PCR Script CAM Cloning vector (Stratagene) and verified by sequencing. The cDNA fragment encoding the mature mQC was amplified using the primers 5' ATACTCGAGAAAAGAGCCTGGACGCAGGAGAAG (SEQ ID NO:13) (XhoI, sense) and 5' ATATCTAGATTACAAGTGAAGATATTCCAAC (SEQ ID NO:14)(XbaI, antisense). The digested fragment was ligated into the vector pPICZaB, propagated in E. coli and verified by sequencing of the sense and antisense strand. Finally, the expression plasmid was linearized using PmeI, precipitated, and stored at -20°C.

### Transformation of P. pastoris and Mini-Scale Expression of Murine QC

1-2 µg of plasmid DNA were applied for transformation of competent P. pastoris cells by electroporation according to the manufacturer's instructions (BioRad). Selection was done on plates containing 100 µg/ml Zeocin. In order to test the recombinant yeast clones upon mQC expression, recombinants were grown for 24 h in 10 ml conical tubes containing 2 ml BMGY. Afterwards, the yeast was centrifuged and resuspended in 2 ml BMMY containing 0.5 % methanol. This concentration was maintained by addition of methanol every 24 h for about 72 h. Subsequently, QC activity in the supernatant was determined. Clones that displayed the highest activity were chosen for further experiments and fermentation.

### Large Scale Expression and Purification of Murine QC

The expression of mQC was performed in a 5 L reactor (Biostad B, B. Braun biotech, Melsungen, Germany). Fermentation was carried out in basal salts medium supplemented with trace salts at pH 5.5. Initially, biomass was accumulated in a batch and a fed batch phase with glycerol as the sole carbon source for about 28 h. Expression of mQC was initiated by methanol feeding according to a three-step profile recommended by Invitrogen for an entire fermentation time of approximately 65 h. Subsequently, cells and turbidity were removed from the mQC-containing supernatant by two sequential centrifugation steps at 6000 x g and 38000 x g for 15 min and 4 h, respectively. For purification, the fermentation broth was diluted with water to a conductivity of about 5 mS/cm and applied in reversed flow direction (15 mL/min) onto a Streamline SP XL column (2.5 x 100 cm), equilibrated with 0.05 M phosphate buffer, pH 6.4. After a washing step in reversed flow direction with equilibration buffer for 2 column volumes, proteins were eluted at a flow rate of 8 mL/min using 0.15 M Tris buffer, pH 7.6, containing 1.5 M NaCl in forward direction. QC-containing fractions were pooled and ammonium sulfate added to a final concentration of 1 M. The resulting solution was applied onto a Butyl Sepharose FF column (1.6 x 13 cm) at a flow rate of 4 mL/min. Bound mQC was washed with 0.05 M phosphate buffer, pH 6.8 containing 0.75 M ammonium sulfate for 5 column volumes and eluted in reversed flow direction with 0.05 M phosphate buffer, pH 6.8. The fractions containing mQC were pooled and desalted overnight by dialysis against 0.025 M Tris, pH 7.5. Afterwards, the pH was adjusted to 8.0 by addition of NaOH and applied (4.0 mL/min) onto an Uno Q column (Bio Rad), equilibrated with 0.02 M Tris, pH 8.1. After a washing step using equilibration buffer, mQC was eluted using the same buffer containing 0.18 M NaCl. Fractions exhibiting QC activity were pooled and the pH adjusted to 7.4 by addition of 1 M Bis-Tris buffer, pH 6.0. mQC was stable at 4 °C for up to 1 month. For long-term storage at - 20 °C, 50 % glycerol was added.

### BEST EMBODIMENT FOR CARRYING OUT THE INVENTION

### Example 1: Cloning of the transgenic vector pTG-CAG-mQC

The vector 'mQC cDNA in pPCR Script Cam (mQC K10' is an aliquot of circular plasmid DNA. It contains the open reading frame (ORF) of murine Qpct as shown above using primers containing additional restriction sites. This amplified cDNA was inserted into the vector pPCR Script CAM SK(+) (Stratagene) via the Srfl restriction site (see Figure 1A).

The delivered plasmid was verified by restriction analysis using NcoI, NotI and NsiI. The expected digestion profile was obtained and no sign of degradation was observed.

Due to the additional SphI restriction site within the 5'-primer that was used for ORF isolation, the nucleic acid sequence ATG had been inserted 4 base pairs downstream of the Qpct start codon (see figure 1B). If this cDNA cassette is cloned downstream of a promoter this additional ATG will lead to a frame shift in the translation. Therefore the ATG of the SphI recognition site had to be removed before the assembly of the promoter and cDNA in the transgenic vector could occur.

For this purpose, the Qpct cDNA cassette was isolated via the restriction sites NsiI (5') and NotI (3') and was inserted within the cloning vector pBlue Script SK+ (Stratagene), which was linearised using the restriction enzyme PstI and NotI (see figure 2). The religation of the NsiI-/PstI-overhangs led to the deletion of the intervening ATG leaving the remaining of the start-ATG of the Qpct-ORF intact. Afterwards the modified cDNA cassette was isolated via HindIII and NotI from the pBlue Script SK+ backbone and inserted into an expression vector already containing the CAG-promoter and the BGH polyA signal sequences within the pcDNA3.1 vector backbone (Invitrogen) (see figure 2).

The resulting plasmid contains the ORF of the murine glutaminyl-peptide cyclotransferase gene downstream of the ubiquitously overexpressing CAG promoter cassette and upstream of the BGH polyA signal. The transgenic vector was named pTG-CAG-mQC.

The plasmid pTG-CAG-mQC was verified by restriction analysis (see figure 3) and sequencing. The transgenic construct can be removed from the plasmid backbone using an ApaLI/ DraIII restriction double-digest.

### Example 2: Sequence verification

Relevant junction regions of the transgenic construct pTG-CAG-mQC were verified by DNA sequencing. Table 1 shows the sequences of the primers used. The obtained sequences of the cloning junction fragments are shown below (SEQ ID No: 1, SEQ ID No: 2). These sequences confirmed the correct insertion of the Qpct cDNA cassette.

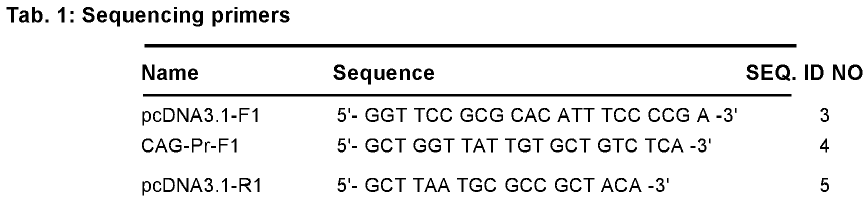

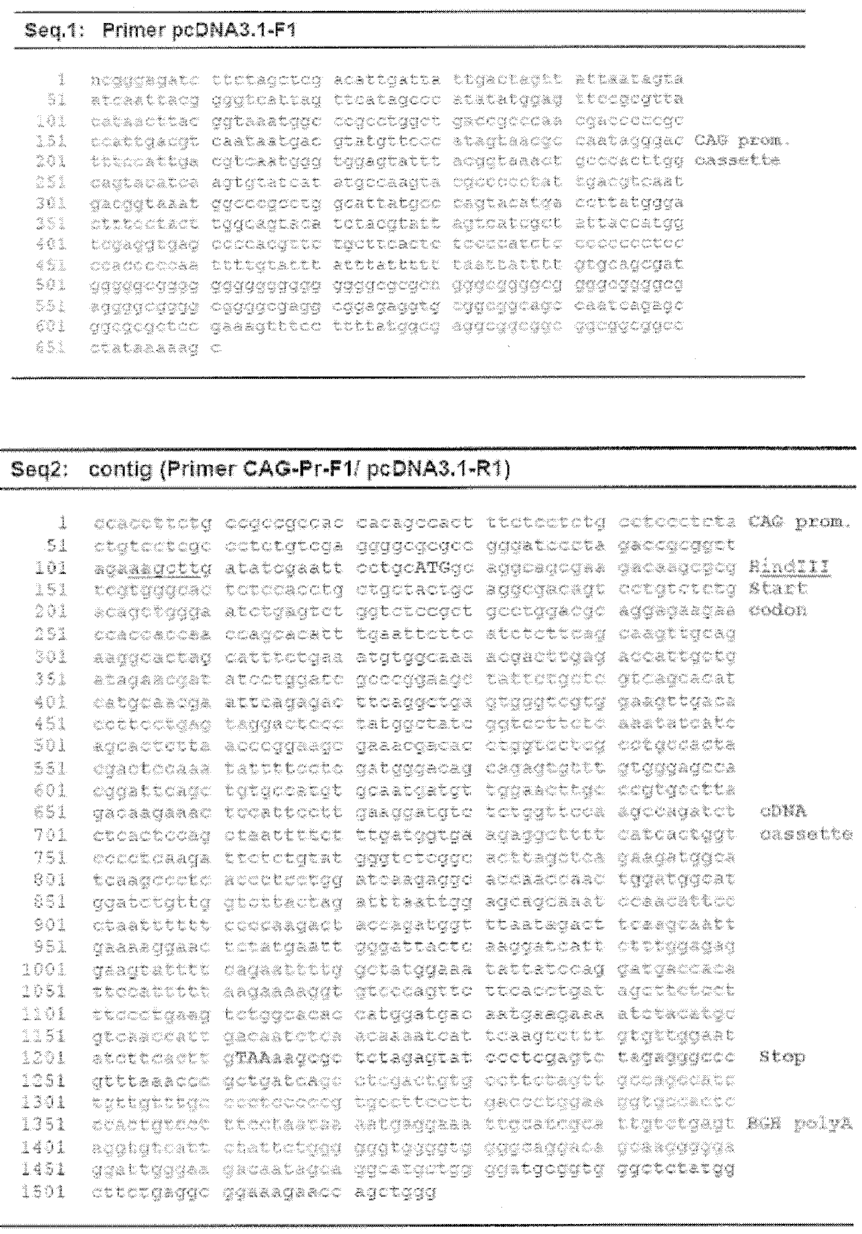

### Example 3: Establishing the screening PCR

To establish the PCR screening for the genotyping of the generated transgenic mice, two primers were designed amplifying a specific PCR product of approx. 1585 bp from the pTG-CAG-mQC DNA (see figure 3). The forward primer CAG-Pr-F1 (SEQ ID No: 2) binds specifically in the CAG promoter segment and the reverse primer GX1675-TOR1-FF (SEQ ID No:6) binds within the BGH polyA sequence of the Qpct cDNA cassette. To confirm that the screening PCR is sufficiently sensitive at the level of genomic DNA, the transgenic vector DNA was serially diluted with wild-type genomic DNA. The primer sequences and the optimised PCR conditions are listed in table 2. Figure 4 shows the result of the sensitivity screen.

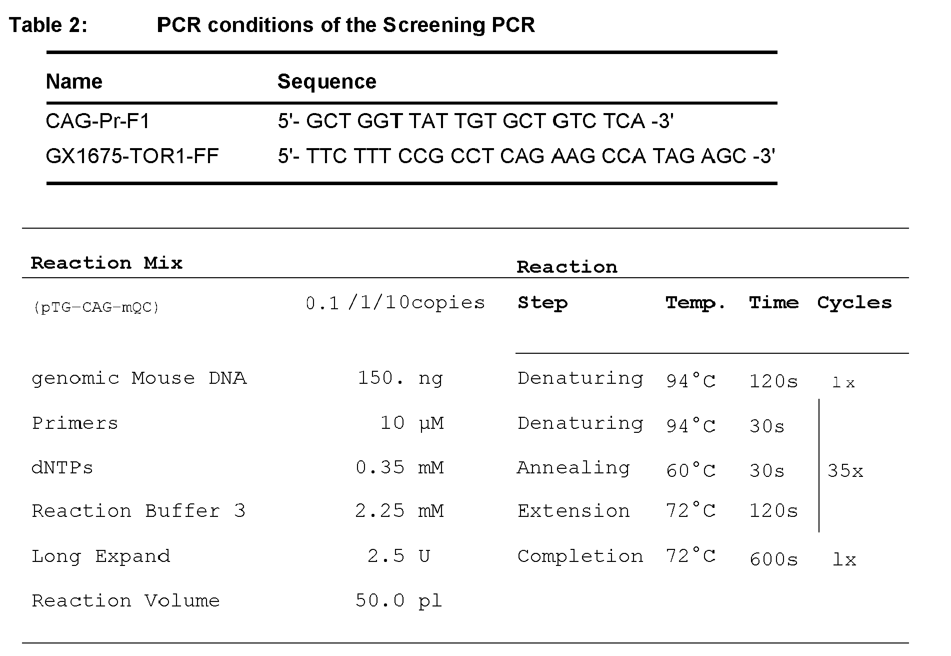

The pTG-CAG-mQC DNA was still detectable at a dilution of 0.1 copy per reaction mix, confirming the PCR screen is sufficiently sensitive to detect an integration event for the screening of potential founder mice.

As shown in Figure 3, the ApaLI/DraIII restriction sites allow the transgenic construct DNA to be isolated from the plasmid backbone. The plasmid pTG-CAG-mQC will be digested with this enzyme and the resulting 3806 bp sized fragment will be isolated and purified for microinjection into mouse oocytes.

The transgenic construct pTG-CAG-mQC, generated in Examples 1 to 3 as shown above, was injected into the male pronucleus of fertilized oocytes. Following overnight cultivation, the resulting two-cell embryos were transplanted into pseudo-pregnant foster mothers. The mice born were characterised for transgene integration by PCR analysis.

The following describes the work performed for the pronucleus injection (PNI) phase of the project and details the identification of founders.

### Example 4: Preparation of the construct

The pTG-CAG-mQC vector was cloned as shown above. The Qpct gene cassette was directly cloned downstream of the hybrid CMV enhancer/ chicken beta-actin (CAG) promoter and upstream of the bovine growth hormone (BGH) polyA signal, generating the transgenic vector pTG-CAG-mQC.

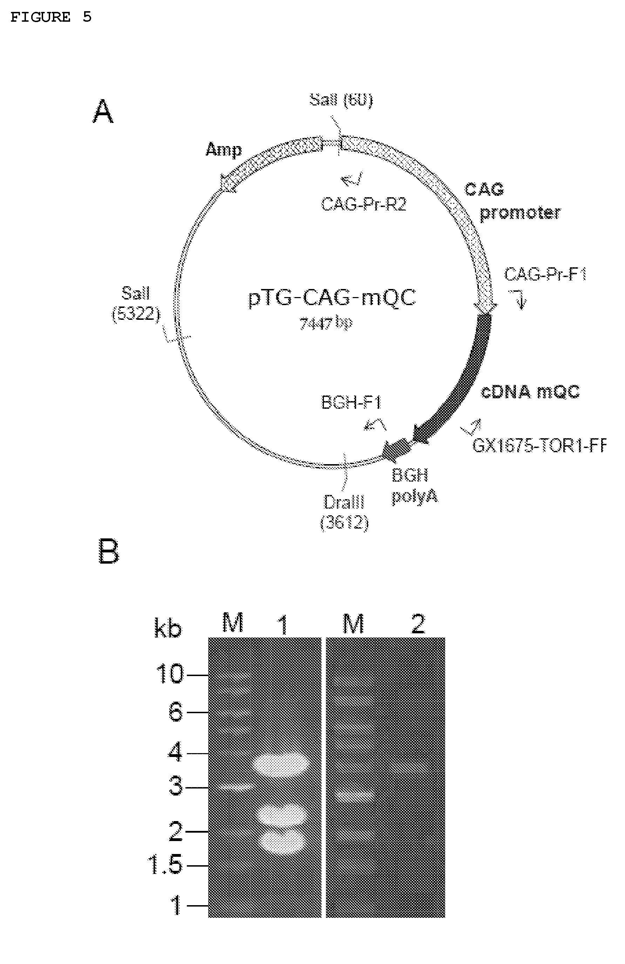

The pTG-CAG-mQC plasmid was digested by DraIII and SalI restriction enzymes and the 3552 bp fragment containing the transgenic construct of interest was separated from the vector backbone electrophoretically (Figure 5). The ~3.6 kb transgenic construct fragment was isolated, purified and diluted in microinjection buffer to a concentration of 5 ng/ µl. The construct purity and the concentration were verified by agarose gel electrophoresis (Figure 5).

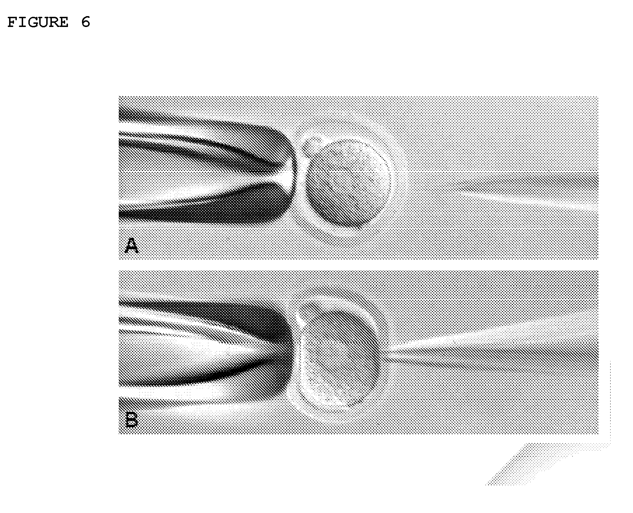

3-4 week old female C57BL/6 mice were mated with male C57BL/6 mice. The resulting fertilized oocytes were collected from the oviduct of plugged female mice and cultured until two clear pronuclei were visible. The purified transgenic construct was microinjected into the male pronucleus (figure 6) and injected embryos were cultivated overnight to the two-cell stage. Two-cell embryos were then implanted into the oviduct of pseudo-pregnant foster mothers at 0.5 days post-coitum.

After 18-19 days, pups were born from the foster mothers. Table 3 summarizes the DNA microinjection sessions performed.

**Table 3: Summary of the PNI sessions**

| **PNI No.** | **Injection date** | **Transferred embryos** | **No. of fosters** | **Pregnant fosters** | **Abortion** | **No. of born pups** | **Viable pups** |
|---|---|---|---|---|---|---|---|
| 1 | 27.01.05 | 40 | 1 | 1 | 1 x, ° | 0 | 0 |
| 2 | 29.01.05 | 38 | 1 | 1 | - | 2 | 1 |
| 3 | 31.01.05 | 40 | 1 | 1 | 1 x | 0 | 0 |
| 4 | 09.02.05 | 100 | 3 | 2 | - | 7 | 4 |
| 5 | 16.02.05 | 49 | 2 | 1 | - | 8 | 8 |
| 6 | 02/03.03.05 | 83 | 2 | 1 | - | 5 | 5 |
| 7 | 16./17.03. | 111 | 3 | 2 | - | 20 | 20 |
| 8 | 06.04.05 | 82 | 4 | 0 | - | 0 | 0 |
| 9 | 03./04.05. | 146 | 6 | 4 | 1 x | 23 | 23 |
| | **In tota**l | 689 | 2 | 13 | 3 x | 65 | 61 |

As presented in table 3, a total of 689 embryos were transferred into foster mothers, leading to the birth of 61 viable pups.

### Example 5: Screening for transgenic founders

Tail tip biopsies from the 61 weaned pups were prepared and DNA was extracted. The individual DNAs were genotyped by the transgenic screening PCR which was established in the previous examples. The primer pair CAG-Pr-F1/ GX1675-TOR1-FF (depicted in figure 5A as dark arrows, SEQ ID Nos: 4 and 7) amplifies the promoter-cDNA junction, yielding a specific 1585 bp PCR product when the transgenic construct has integrated into the host genome.

A second PCR using a forward primer BGH-F1 (SEQ ID No:7), binding to the BGH polyA sequence of the transgenic construct, and a reverse primer CAG-Pr-R2 (SEQ ID No:8), binding to the 5' end of the promoter cassette, takes advantage of the frequently observed head-to-tail integration of the transgenic construct and amplifies an approx. 834 bp fragment. This amplification product only occurs when more than one copy of the transgenic construct has tandemly integrated.

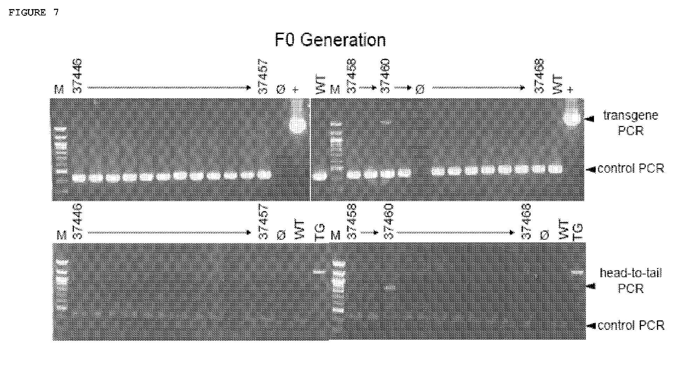

The primer sequences are listed in table 4 and the optimised PCR conditions of both PCRs are listed in table 5 and 6. The quality of the DNA was confirmed using primers specific for a control gene. Figure 7 shows an example of the PCR screening results.

**Table 4: Genotyping Primer**

| **PCR type** | **Primer name** | **Primer Sequence (5' -3')** | **Expected size** |
|---|---|---|---|
| transgene | CAG-Pr-F1 | GCTGGTTATTGTGCTGTCTCA | 1585 bp |
| | GX1675-TOR1 -FF | TTCTTTCCGCCTCAGAAGCCATAGAGC | |
| head-to-tail | BGH-F1 | TGTAGGTGGCAGAGAGACTA | 834 bp |
| | CAG-Pr-R2 | CTGCCAAGTAGGAAAGTCCCAT | |

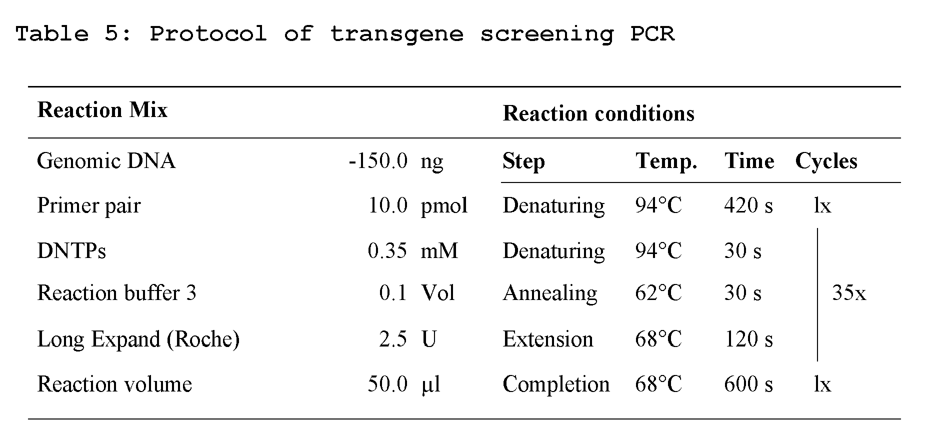

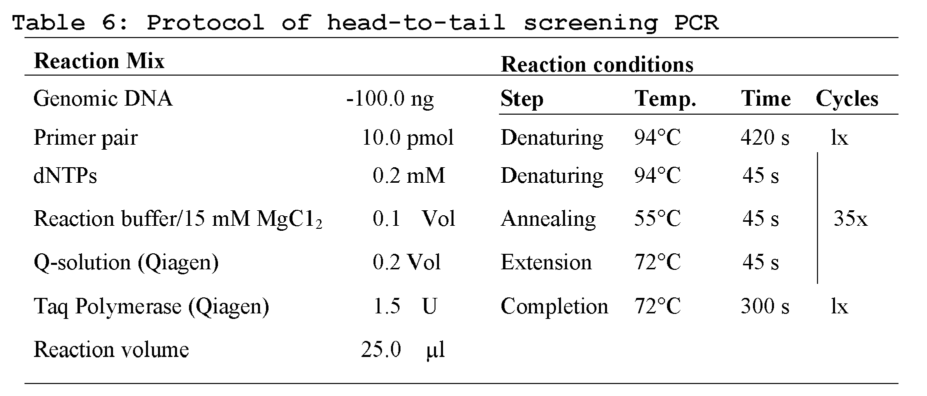

The PCR screening of the 61 pups with both genotyping PCR types resulted in the identification of one positive transgenic animal. As can be seen in figure 7, the founder No. 37460 (listed in table 7) could be identified using the transgene PCR. It is also proved positive for the head-to-tail PCR, suggesting tandem integration of the transgenic construct had occurred.

The result of the DNA microinjection reveals that only 1.6 % genotyped animals had integrated the transgene DNA.

**Table 7: Positive identified founder for the transgenic line CAG-mQC**

| **Mouse No.** | **Generation** | **Date of birth** | **Sex** | **Genotype** |
|---|---|---|---|---|
| 37460 | F0 | 25.05.05 | female | TG/+ |

Examples 4 and 5 summarize the work performed for the generation of the CAG-mQC transgenic mouse model using the pronuclear microinjection (PNI) approach.

The transgenic construct pTG-CAG-mQC was prepared, purified and injected into the male pronucleus of fertilized oocytes and a total of 689 embryos were transferred into foster mothers. These microinjection sessions led to the birth of 61 viable pups. The PCR screening led to the identification of one female founder carrying the transgenic construct integrated into the genome.

A low rate of transgenic construct integration is frequently associated with either oocyte toxicity or with a phenotypic effect which is incompatible with embryonic development.

In the previous examples the transgenic construct pTG-CAG-mQC was injected into the male pronucleus of 689 fertilized oocytes, resulting in the identification of one born transgenic founder, namely animal No. 37460. This female founder was crossed with C57BL/6 male generating F1 mice carrying the transgene CAG-mQC in their genome.

The following describes the work performed for the breeding to the F1 generation phases.

### Example 6: Summary of pronucleus injection phase

### 6-1 Injection sessions

The transgenic construct was isolated from the pTG-CAG-mQC vector, cloned as shown above.

Recipient oocytes were isolated from pregnant C57BL/6J females with a SPF (Specific and Pathogen Free) health status. The transgene DNA were injected into the male pronucleus of 689 fertilized oocytes and these manipulated embryos were re-implanted into 23 OF1 pseudo-pregnant females with an SOPF (Specific and Opportunistic Pathogen Free) health status.

### 6-2 Transgenic founders

The injection sessions gave rise to born 69 pups, among which 61 pups survived the weaning time. All 61 viable F1 pups were characterised by the established screening PCRs and only one female (No. 37460, *25.05.05) was identified carrying the transgenic DNA randomly integrated in the genome.

### Example 7: Generation of F1 animals

The transgenic female No. 37460 was bred with a wild-type C57BL/6 male (health status SOPF - Specific an Opportunist Pathogen Free) from 12th July 2005 to establish the transgenic CAG-mQC mouse line by generating F1 transgenic mice.

Table 8 below summarizes the results of transgenic F1 breeding of 3 litters. Unfortunately the first litter, born *05.08.05, was cannibalized by their mother.

As documented in table 8 more than 50 % of the F1 born animals carry the transgene in their genome indicating that the transgenic founder is able to transmit the transgene through the germ layer.

**Table 8: Summary of the F1 breeding**

| **litter No.** | **birth date** | **No. of born pups** | **No. of viable pups** | **Male transgenic F1** | **female transgenic F1** |
|---|---|---|---|---|---|
| 1 | 05.08.05 | 9 | 0 (cannibalized) | - | - |
| 2 | 16.09.05 | 9 | 9 | 1 | 4 |
| 3 | 26.10.05 | 9 | 8 | 2 | 3 |
| | **In total** | 27 | 17 | 3 | 7 |

### Example 8: Genotyping of the F1 generation

Tail tip biopsies from the 17 weaned pups were prepared and DNA was extracted. The individual DNAs were genotyped by the transgenic screening PCR which was established as shown above. The primer pair CAG-Pr-F1/ GX1675-TOR1-FF (SEQ ID No: 4 and 6) amplifies the promoter-cDNA junction, yielding a specific 1585 bp PCR product when the transgenic construct has integrated into the host genome.

The primer sequences and the optimised PCR condition are listed in table 9. The quality of the DNA was confirmed using primers specific for a control gene. Figure 8 shows an example of the PCR screening results.

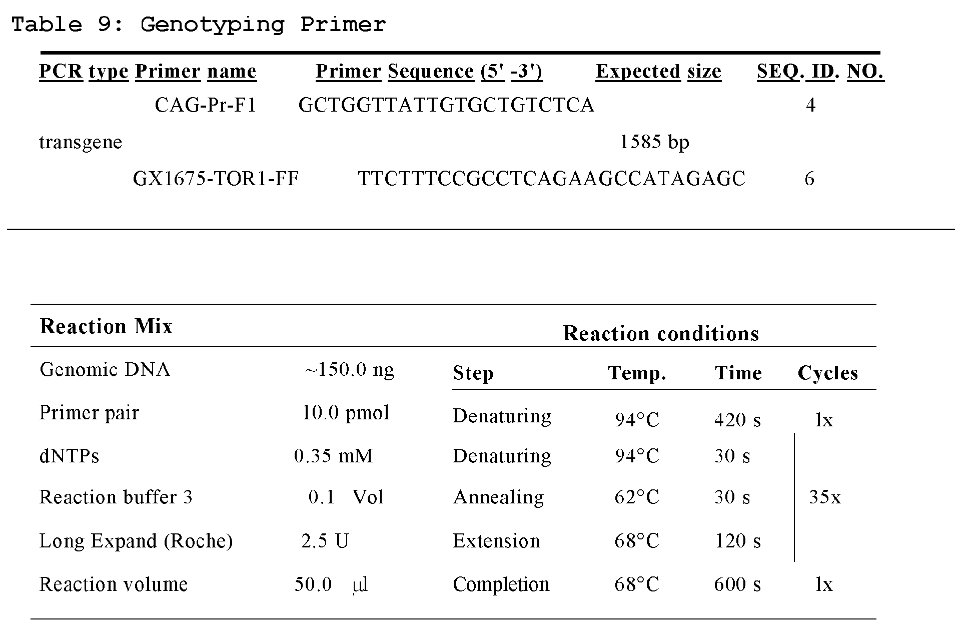

The PCR screening of the 17 F1 pups resulted in the identification of 10 positive transgenic animals.

**Table 10: Genotype of the F1 generation of the transgenic line CAG-mQC**

| **Genotype** | **Generation** | **Date of birth** | **Sex** | **Mouse No.** |
|---|---|---|---|---|
| TG/+ | FO | 25.05.05 | female | 37460 |
| TG/+ | F1 | 16.09.05 | male | 39771 |
| TG/+ | F1 | 16.09.05 | female | 39773, 39776, 39778, 39779 |
| TG/+ | F1 | 26.10.05 | male | 30490,30491 |
| TG/+ | F1 | 26.10.05 | female | 30492, 30495, 30496 |
| WT | F1 | 16.09.05 | male | 39772 |
| WT | F1 | 16.09.05 | female | 39774, 39775, 39777 |
| WT | F1 | 26.10.05 | male | 30489 |
| WT | F1 | 26.10.05 | female | 30493,30494 |

The examples 6-8 summarize the work performed for the establishment of the CAG-mQC transgenic mouse line generated by breeding the female founder No. 37460 with C57BL/6 males.

The breeding of the transgenic female No. 37460 resulted in the birth of 3 litters. The first litter, born at *05.08.2005 was unfortunately cannibalized by their mother. The genotyping of the further 2 litters resulted in the identification of 3 transgenic males and 7 transgenic females among the 17 viable F1 pups.

Thus, the F1 breeding for the generation of a ubiquitous overexpressing Qpct mouse line could be successfully completed.

The results of the following examples prove an overexpression of the target gene. Expression was assessed by determination of QC activity in EDTA-Plasma and tissue homogenates of liver, kidney and brain. In EDTA-Plasma, pbd17E3 displayed a 21fold higher specific QC activity compared with wildtype littermates. QC expression in the tissue homogenates was only reliably quantified for the tg animals, suggesting again a conspicuous overexpression.

In brain, liver and kidney, target gene expression was also analyzed by RT-PCR and real time PCR. Consequently, the results corroborate the results concerning enzymatic activity. In brain and liver, a 4- and 5-fold mRNA concentration compared to the wt was determined, respectively. The mRNA level in kidney was drastically raised by 66-fold.

The data prove the suitability of pbd17E3 for further studies regarding the role of QC in pathophysiological conditions, e.g. Alzheimer's disease, preferably by breeding with established animal models or for generation of further AD mouse models.

The purpose of the following examples was the determination of the activity of the enzyme glutaminyl cyclase (QC) in heterozygous transgenic mice supposed to overexpress this enzyme. For this phenotyping the activity of glutaminyl cyclase should be determined in brain, liver, kidney and EDTA-plasma of two transgenic animals and two wildtype controls with an equal genetic background. Furthermore, the expression of QC-mRNA in the described organs should be examined. All animals were of female gender.

### Example 9: Material and Methods

### 9.1. HPLC-Assay for QC-activity

The QC activity in tissue of wild type and transgenic animals was assessed by quantification of the QC-mediated cyclization of Glutaminyl-beta-naphthylamine to pyroglutamyl-beta-naphthylamine by use of a HPLC based assay. The measurement was carried out using an HPLC system "La chrome", manufactured by Merck-Hitachi and a RP18 LiChroCART 125-4 column supplied by Merck KGaA. For the separation, a gradient of water and acetonitrile containing 1% TFA each was used p. r. t. as follows:

**Table 9-1 HPLC protocol for measurement of QC activity in tissue lysates and EDTA plasma**

| **Time (min)** | **Eluent A Acetonitrile/ TFA( 1%)** | **Eluent B Water/TFA (1%)** |
|---|---|---|
| 0 | 23 | 77 |
| 8 | 45 | 55 |
| 10 | 95 | 5 |
| 15 | 95 | 5 |
| 20 | 23 | 77 |

Detection of glutaminyl-beta-naphthylamine and pyroglutamyl-beta-naphthylamine was performed by use of a Diode Array Detector L7455 manufactured by Hitachi Corporation at a wavelength of 280 nm. All measurements were carried out at room temperature. The concentration of pyroglutamyl-beta-naphthylamine was calculated using a standard curve.

### Example 9-1: Sample Preparation

Determinations of QC-activity were performed using tissue homogenates from brain, liver, kidney and plasma. The plasma was centrifuged at 4 °C and 13000 xg for 10 min and applied for the QC-assay. Besides, the plasma of the control group could be used directly unwatered for measurement (100 µl), while that of the QC-transgenic mice was diluted 1:25 using MOPS-buffer (25 mM, pH 7.0). Tissue of brain, liver and kidney was mixed in 40fold volume lysis buffer and homogenized by use of a Downs homogenizer. The lysis-buffer (pH 7.5) consisted of Tris-base (10 mM), EDTA (5mM), Triton (0.5%) and glycerine (10%).

Subsequently, the samples were treated with an ultrasonic stick (16 cycles, intensity 70%) and afterwards centrifuged (25 min, 13000 xg, 4 °C). The supernatants were taken and immediately used for the measurement (100 µl).

### 9.3. Determination of QC activity

After the sample preparation, every determination of pyroglutamyl-beta-naphthylamine was carried out according to the following protocol, which has been identical for measurement of the standard curve with purified recombinant QC enzyme and of the time-turnover-graphs with QC from the homogenized tissue samples.

In a 1.5 mL tube, 500 µL substrate solution (100 µM Glutaminyl-beta-naphthylamine in 25 mM MOPS-buffer, pH 7. 0) were mixed with 400 µL N-ethyl-maleimide solution (250 µM, in 25 mM MOPS-buffer, pH 7. 0) as a cysteine-protease-inhibitor. The mixture was allowed to equilibrate at 30 °C for 10 min in a heating block at 350 rpm. After equilibration, the reaction was started by addition of 100 µL of cell lysate or plasma (diluted in case of tissue of transgenic animals) to a total volume of 1000 µL. The reaction mixture was then incubated for 45 min at 30 °C at 350 rpm. From the total reaction volume, samples (100 µL) were removed after 0, 4, 8, 12, 16, 20, 24, 35 and 45 min. To stop the ongoing conversion from glutaminyl-beta-naphthylamine into pyroglutamyl-beta-naphtylamine, these samples were immediately heated for 5 min in boiling water to inactivate the enzyme glutaminyl-cyclase. Afterwards, the samples were immediately frozen at -20°C. The experiment was run in three replicates at the same time. Prior to analysis using HPLC, all samples have been frozen once. For the measurement the samples were thawed and thereafter centrifuged for 10 min at 13000 rpm at room temperature before starting the measuring process on the HPLC system. Then, 25 µL of the sample were diluted 1:1 1 with water bidest and mixed. This solution was injected completely with a 100 µL Hamilton syringe into the 20 µL sample loop of the HPLC system. Between the measurements, the Hamilton syringe was cleaned two times with water, two times with acetone and thereafter two times with water again. Before drawing the syringe with a new sample the needle was rinsed two times (2 x 3 µL) with the new sample.

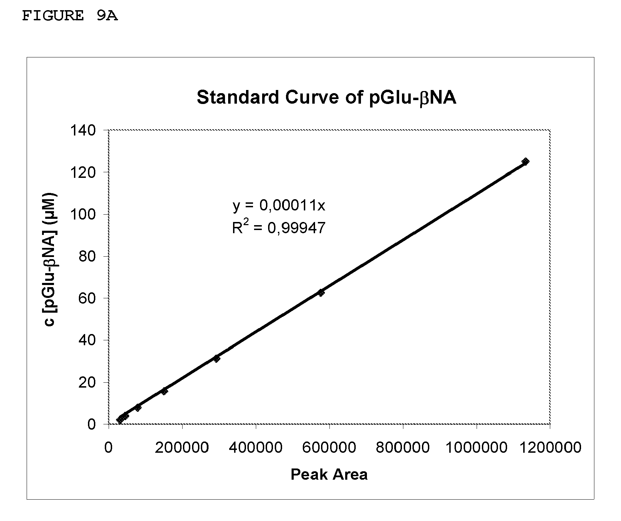

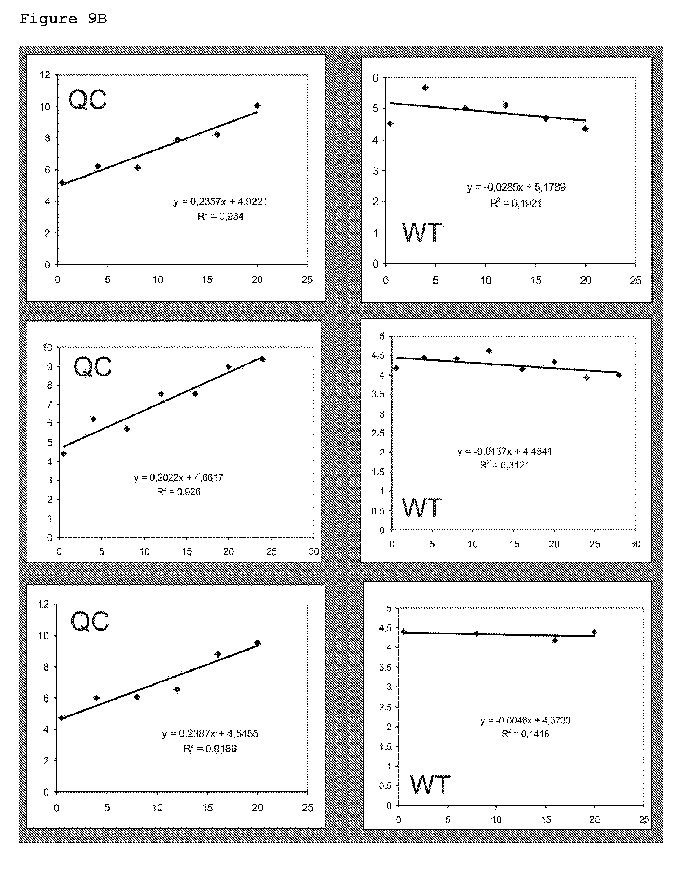

The resulting peak areas for pyroglutamyl-beta-naphthylamine (Rt: ~6. 8 min, the retention time of glutaminyl-beta-naphthylamine was at Rₜ: ~4. 85 min) were converted into concentrations of pyroglutaminyl-beta-naphthylamine by use of the standard curve. The resulting figures were plotted in a time (x-axis) - turnover (y-axis) - diagram. The initial velocity of the reaction converting glutaminyl-beta-naphtylamine into pyroglutaminyl-beta-naphtylamine was calculated by linear regression in the region of linear product formation, which was usually observed during the first 20 minutes of the reaction. The resulting diagrams and initial velocities are exemplary shown for QC-activity in brain (see Fig. 9).

### Example 10: QC activity in EDTA plasma and tissue homogenates

In the plasma of female QC-transgenic mice, a 21fold increase of QC-activity was detected referred to volume. In tissue homogenates of liver, QC-activity (referred to weight) was comparable to that of plasma of QC-animals. A nearly 3fold increase of QC-activity was observed in brain tissue of QC tg mice compared with plasma of the transgenic animals. It was not possible to compare QC-activities measured in tissues from transgenic animals to those in non-transgenic controls. In the control group, QC-activity was not determined if sample preparation was done as with QC transgenic animals (40fold dilution of lysates). Thus, in theory an infinite multiple in QC-activity would occur. If tissue lysates from a control group were measured in higher concentrations than 1:40, most of the glutaminyl-beta-naphthylamine given to the reaction as a substrate for QC got lost without conversion to pyroglutamyl-beta-naphthylamine. Therefore, a determination of QC-activity in tissues of control mice was not possible and a direct comparison between QC transgenic and control mice was only available for EDTA plasma. Here, QC mice showed a 21-fold relative increase of QC activity. (Figures 10A and B).

### 10.2 Evaluation of QC transcript levels using PCR and real time PCR

Total mRNA was isolated from tissue of liver, kidney and brain by use of the NucleoSpin-Kit (Macherey Nagel) according to the manufacturers' protocol and thereafter 1 µg of isolated mRNA was transcribed into cDNA applying random hexamer oligonucleotides for priming.

Qualitative PCR of all tissues was performed using DNA polymerase from Thermus aquaticus (Taq-Polymerase, Promega) according to the manufacturer's instructions. The reaction was carried out applying the following conditions: Annealing: 57°C, 45 s; Extension: 72°C, 60 s; melting: 95°C, 30 s. The samples were analyzed on a 1.4% Agarose gel in TBE buffer.

As could be expected, a remarkably higher concentration of mouse QC mRNA was detected in QC transgenic mice compared to the control group (Fig. 10-C).

To quantify the expression of QC on mRNA-level, a real-time RT-PCR was performed using a Corbett Research "Rotor Gene 3000".

In brain, liver and kidney, QC mRNA concentrations were strongly increased compared to the control group (Fig. 10D). The highest expression was observed in the kidney with a 66-fold raise of QC mRNA concentration.

### Example 11: Characterization of transgenic mice overexpressing human QC neuron-specifically.

Transgenic mice overexpressing human QC, driven by the Thy-1 promoter were generated, essentially as described in examples 1-9 for transgenic mice overexpressing murine QC ubiquitously and is further outlined in Example 12.

Three founders were efficiently crossbred, and the QC-expression characterized using QC-activity assays and Western-Blotting applying specific antibodies. For determination of QC-activity, 50 mg of brain tissue were homogenized in 1 ml of buffer consisting of 10mM Tris pH 7,5, 100mM NaCl, 5mM EDTA and 0,5% Triton X-100 and 10% Glycerol. The tissue was homogenized by several strokes in Downs-homogenizer and poured into a 12ml conical tube. The homogenate was then subjected to 3x 10s of ultrasonic shock. The resulting homogenate was centrifuged at 4°C for 25min and the QC-activity of the supernatant determined.

The hQC activity was determined applying a HPLC-assay, essentially as described in example 9. Briefly, samples consisted of 500µl substrate (Q-βNA, 50µM final concentration), 400µl N-Ethylmaleimide (100µM final concentration) and 100µl sample containing QC. The reaction was incubated at 30°C and samples were removed after 0, 5, 10, 15, 22, 30 und 45min. Subsequently, the reaction was terminated by incubation in a boiling water bath for 5 min.

As illustrated in Figure 11, the sample obtained from a transgenic animal carrying the hQC transgene contained a significantly reduced QC-activity compared to a non-transgenic littermate, clearly proving the hQC expression in the transgenic animals.

To further validate the QC-expression in transgenics and to select the founder line displaying the highest QC-activity, a second assay was applied, which is based on fluorometric determination of enzymatic activity (Schilling, et al., Anal. Biochem. (2002) 303:49-56). As indicated in Figure 12, a 35-fold increase of pGlu-pNA formation was observed in the brain homogenate of the hQC-transgenic mouse compared to the non-transgenic littermate, proving the overexpression of the enzyme.

The expression of human QC was characterized for three different founder mouse lines (line 53, 37 and 43) and the murine QC overexpressing line pbd17E3, as shown in Figure 13A. Significant overexpression of QC was detected in all hQC tg animals. In comparison, murine QC of pbd17E3 was less expressed in brain, caused by the different promoters used (ubiquitous CAG promoter in pbd17E3 and murine Thy-1 neuron-specific promoter in hQC transgenic mice).

Based on these results, the hQC-transgenic mouse founder line 53 displayed the highest expression of QC (Figure 13B).

The results of the analysis of QC-activity were finally substantiated by Western-Blot analysis using a QC-specific antibody (Figure 14). The most prominent band was observed in samples, which were derived from the human QC transgenic mouse line 53, which already displayed the highest enzymatic activity. Line 43 showed a medium signal and 37 the lowest QC signal, corresponding to a molecular mass of approximately 32 kDa. Only a faint signal was obtained with pbd17E3 mice, reflecting the results of the determination of QC activity in brain samples. Summarizing, on the basis of the determination of the QC by two different assays for the enzymatic activity and by Western-Blot analysis, an efficacious expression of human QC in transgenic mice was achieved. The neuron specific promotor thus triggers the expression very efficiently. Therefore, these mice are best suited for the development of transgenic mice, which aim at the modelling of disease conditions which are related to QC and/or which are facilitated or accelerated by QC, e.g. Alzheimer's disease, Familial British Dementia, Familial Danish Dementia, Huntington's disease.

### Example 12: Generation of transgenic mice overexpressing human QC neuron-specifically.

### 12.1 Establishment of transgenic plasmid and microinjection

The plasmid pcDNA3.1-hQC containing the open reading frame of human QC (swiss-prot entry Q16769), was used as template for PCR amplification of the hQC cDNA with the following primers:
- mThyl-hQC-XhoI-F (5'-AAT AAT CTC GAG GCC ACC ATG GCA GGC GGA AGA CAC CG-3', SEQ ID No. 42)
- mThyl-hQC-BsrGI-R (5'-ACA TAT GTA CAT TAC AAA TGA AGA TA-3', SEQ ID No. 43).

The PCR product was digested with XhoI and BsrGI and ligated with the pUC18-mThy1 vector plasmid (Figure 15). The correct plasmid clone was identified by restriction and sequencing.

The transgenic plasmid pUC18-mThy1-hQC was linearized with Pvu I and Not I to eliminate plasmid sequences. The 7929 bp fragment corresponding to the transgenic construct was separated from the vector backbone by agarose gel electrophoresis and further purified. The plasmid backbone was removed by digestion with Not I and Pvu I (Figure 16). A resulting 7929 bp DNA fragment was applied for pronuclear microinjection into (C57BL/6 x CBA) F2 oocytes followed by Re-implantation of minimal 150 viable microinjected oocytes into pseudo-pregnant mice.

### 12.2 Identification of transgenic founders

A PCR screening strategy was established using the following primers:
Forward primer hQCFI:·5'- TCCTACAAGTCTTTGTGTTGGAA -3' (SEQ ID No. 37)
Reverse primer mThy1R1: 5'- GAAGGACTTGGGGAGGGAG -3' (SEQ ID No. 38)
Probe 13p: 5'-FAM- CAAGTAAGTCGAGGTCCTTCCTCTGCA -TAMRA-3' (SEQ ID No. 39)

In addition, head -to tail PCR was used to identify tandem integration:
Forward primer HTT-mthy1-F: 5'- AGCAAGCCTGGAAGACCTGGGA -3' (SEQ ID No. 40)
Reverse primer HTT-mThy1-R: 5'- AGACTCAGCCCATCCACTCCTT -3 (SEQ ID No. 41).

The identified Founders are listed in Table 12-1.

**Table 12-1**

| **Founder** | **color** | **sex** |
|---|---|---|
| Fo#37 | brown | male |
| Fo#38 | brown | male |
| Fo#43 | brown | male |
| Fo#48 | brown | male |
| Fo#53 | brown | male |

We conducted a head-to-tail PCR to investigate transgenic construct integrity and multiple transgenic copies (Primers HTT-mthyl-F and HTT-mthy1-R). Here we've got the following results:

| | |
|---|---|
| Fo#37 | weak correct PCR fragment band |
| Fo#38 | strong correct PCR fragment band |
| Fo#43 | weak correct PCR fragment band |
| Fo#48 | weak correct PCR fragment band |
| Fo#53 | strong correct PCR fragment band |

This result leads to the conclusion that all founders have multiple transgenic fragments integrated in tandem direction (as illustrated in Figure 3).

The more constructs are combined in tandem orientation, the stronger is the head-to-tail PCR band signal.

All founders were bred with B6CBA breeding partners. F1 mice were screened with the above described qPCR primer/probe set. Transgenic pups could only be identified for Fo#37, Fo#43, and Fo#53. The founder Fo#38 and Fo#48 F1 pups were all non-transgenic.

Samples of cortex (Co), hippocampus (Hi) and spinal cord (SC) of different transgenic F1 pups of Fo#37, Fo#43, and Fo#53 (age: 2 to 3.5 months) were investigated by RT-qPCR together with one non-transgenic control pup. The results are shown in Figure 17.

The highest mRNA levels could be detected in Fo#53 samples.

## Claims

1. A transgenic non-human animal for overexpressing Qpct comprising cells containing a DNA transgene encoding Qpct.

2. The transgenic non-human animal of claim 1, wherein the animal is heterozygous for the transgene.

3. The transgenic non-human animal of claim 1, wherein the animal is homozygous for the transgene.

4. The transgenic non-human animal of any of claims 1 to 3, wherein the animal is a mouse.

5. The transgenic non-human animal of any of claims 1 to 4, wherein the transgene is of murine origin.

6. The transgenic non-human animal of any of claims 1 to 4, wherein the transgene is of human origin.

7. The transgenic non-human animal of any of claims 1 to 6, wherein the transgene is a recombinant gene.

8. The transgenic non-human animal of claim 7, wherein the recombinant transgene encodes a chimeric or humanized polypeptide.

9. The transgenic non-human animal of any of claims 1 to 8, wherein the transgene encodes an isoenzyme of Qpct.

10. The transgenic non-human animal of claim 9, wherein the isoenyzme is a protein selected from the group consisting of any one of SEQ ID NOS: 15-25 or an analogue thereof having at least 50% / 75% sequence identity / similarity.

11. The transgenic non-human animal according to any of claims 1 to 10, wherein the transgene is operably linked to a tissue-specific promoter.

12. A method for screening for biologically active agents that inhibit or promote Qpct production in vino, comprising:
administering a test agent to the transgenic non-human animal of any of claims 1 to 11, and
determining the effect of the agent on the amount of Qpct produced.

13. A cell or cell line derived from the transgenic non-human animal according to any of claims 1 to 11.

14. A transgenic mouse comprising a transgenic nucleotide sequence encoding Qpct operably linked to a promoter, integrated into the genome of the mouse, wherein the mouse demonstrates a phenotype that can be reversed or ameliorated with a Qpct inhibitor.

15. The mouse of claim 14, wherein the mouse overexpresses Qpct.

16. The mouse of claims 14 or 15, wherein the mouse is heterozygous for Qpct.

17. The mouse of claims 14 or 15, wherein the mouse is homozygous for Qpct.

18. The mouse of claims 14 or 15, wherein the transgenic sequence encodes murine Qpct.

19. The mouse of claims 14 or 15, wherein the transgenic sequence encodes human Qpct.

20. The mouse of any of claims 14 to 19, wherein the transgene encodes an isoenzmye of Qpct.

21. The mouse of claim 20, wherein the isoenyzme is a protein selected from the group consisting of any one of SEQ ID NOS: 15-25 or an analogue thereof having at least 50% / 75% sequence identity / similarity.

22. A method for screening for therapeutic agents that inhibit or promote Qpct activity comprising
(a) administering test agents to the transgenic mouse of any of claims 14 to 21
(b) evaluating the effects of the test agent on the neurological phenotype of the mouse, and
(c) selecting a test agent which inhibits or promotes Qpct activity.


SEQUENCE LISTING

<110>  Probiodrug AG

<120>  IN VIVO SCREENING MODELS FOR TREATMENT OF ALZHEIMER'S DISEASE AND
OTHER QPCT-RELATED DISORDERS

<130>  PBD 00061

<150>  US 60/885,649
<151>  2007-01-19

<160>  43

<170>  PatentIn version 3.1

<210>  1
<211>  661
<212>  DNA
<213>  Artificial sequence

<220>
<223>  Synthetic nucleotide

<220>
<221>  misc_feature
<222>  (1)..(1)
<223>  n is selected from A, C, G or T.

<220>
<221>  misc_feature
<222>  (530)..(530)
<223>  n is selected from A, C, G or T.

<400>  1
ncgggagatc ttctagctcg acattgatta ttgactagtt attaatagta atcaattacg      60

gggtcattag ttcatagccc atatatggag ttccgcgtta cataacttac ggtaaatggc     120

ccgcctggct gaccgcccaa cgacccccgc ccattgacgt caataatgac gtatgttccc     180

atagtaacgc caatagggac tttccattga cgtcaatggg tggagtattt acggtaaact     240

gcccacttgg cagtacatca agtgtatcat atgccaagta cgccccctat tgacgtcaat     300

gacggtaaat ggcccgcctg gcattatgcc cagtacatga ccttatggga ctttcctact     360

tggcagtaca tctacgtatt agtcatcgct attaccatgg tcgaggtgag ccccacgttc     420

tgcttcactc tccccatctc ccccccctcc ccacccccaa ttttgtattt atttattttt     480

taattatttt gtgcagcgat gggggcgggg gggggggggg ggggcgcgcn gggcggggcg     540

gggcggggcg aggggcgggg cggggcgagg cggagaggtg cggcggcagc caatcagagc     600

ggcgcgctcc gaaagtttcc ttttatggcg aggcggcggc ggcggcggcc ctataaaaag     660

c                                                                     661

<210>  2
<211>  1527
<212>  DNA

<213>  Artificial sequence

<220>
<223>  Synthetic nucleotide

<400>  2
ccaccttctg ccgccgccac cacagccact ttctcctctg cctccctcta ctgtcctcgc      60

cctctgtcga ggggcgcgcc gggatcccta gaccgcggct agaaagcttg atatcgaatt     120

cctgcatggc aggcagcgaa gacaagcgcg tcgtgggcac tctccacctg ctgctactgc     180

aggcgacagt cctgtctctg acagctggga atctgagtct ggtctccgct gcctggacgc     240

aggagaagaa ccaccaccaa ccagcacatt tgaattcttc atctcttcag caagttgcag     300

aaggcactag catttctgaa atgtggcaaa acgacttgag accattgctg atagaacgat     360

atcctggatc gcccggaagc tattctgctc gtcagcacat catgcaacga attcagagac     420

ttcaggctga gtgggtcgtg gaagttgaca ccttcctgag taggactccc tatggctatc     480

ggtccttctc aaatatcatc agcactctta acccggaagc gaaacgacac ctggtcctcg     540

cctgccacta cgactccaaa tattttcctc gatgggacag cagagtgttt gtgggagcca     600

cggattcagc tgtgccatgt gcaatgatgt tggaacttgc ccgtgcctta gacaagaaac     660

tccattcctt gaaggatgtc tctggttcca agccagatct ctcactccag ctaattttct     720

ttgatggtga agaggctttt catcactggt cccctcaaga ttctctgtat gggtctcggc     780

acttagctca gaagatggca tcaagccctc accctcctgg atcaagaggc accaaccaac     840

tggatggcat ggatctgttg gtcttactag atttaattgg agcagcaaat ccaacattcc     900

ctaatttttt ccccaagact accagatggt ttaatagact tcaagcaatt gaaaaggaac     960

tctatgaatt gggattactc aaggatcatt ctttggagag gaagtatttt cagaattttg    1020

gctatggaaa tattatccag gatgaccaca ttccattttt aagaaaaggt gtcccagttc    1080

ttcacctgat agcttctcct ttccctgaag tctggcacac catggatgac aatgaagaaa    1140

atctacatgc gtcaaccatt gacaatctca acaaaatcat tcaagtcttt gtgttggaat    1200

atcttcactt gtaaaagcgc tctagagtat ccctcgagtc tagagggccc gtttaaaccc    1260

gctgatcagc ctcgactgtg ccttctagtt gccagccatc tgttgtttgc ccctcccccg    1320

tgccttcctt gaccctggaa ggtgccactc ccactgtcct ttcctaataa aatgaggaaa    1380

ttgcatcgca ttgtctgagt aggtgtcatt ctattctggg gggtggggtg gggcaggaca    1440

gcaaggggga ggattgggaa gacaatagca ggcatgctgg ggatgcggtg ggctctatgg    1500

cttctgaggc ggaaagaacc agctggg                                        1527


<210>  3
<211>  22
<212>  DNA
<213>  Artificial sequence

<220>

<223>  DNA Primer

<400>  3
ggttccgcgc acatttcccc ga                                                22


<210>  4
<211>  21
<212>  DNA
<213>  Artificial sequence

<220>
<223>  DNA Primer

<400>  4
gctggttatt gtgctgtctc a                                                 21


<210>  5
<211>  18
<212>  DNA
<213>  Artificial sequence

<220>
<223>  DNA Primer

<400>  5
gcttaatgcg ccgctaca                                                     18


<210>  6
<211>  27
<212>  DNA
<213>  Artificial sequence

<220>
<223>  DNA Primer

<400>  6
ttctttccgc ctcagaagcc atagagc                                           27


<210>  7
<211>  20
<212>  DNA
<213>  Artificial sequence

<220>
<223>  DNA Primer

<400>  7
tgtaggtggc agagagacta                                                   20


<210>  8
<211>  22
<212>  DNA
<213>  Artificial sequence

<220>
<223>  DNA Primer

<400>  8
ctgccaagta ggaaagtccc at                                                22

<210>  9
<211>  1089
<212>  DNA
<213>  Mus musculus

<400>  9
atggcaggca gcgaagacaa gcgcgtcgtg ggcactctcc acctgctgct actgcaggcg      60

acagtcctgt ctctgacagc tgggaatctg agtctggtct ccgctgcctg gacgcaggag     120

aagaaccacc accaaccagc acatttgaat tcttcatctc ttcagcaagt tgcagaaggc     180

actagcattt ctgaaatgtg gcaaaacgac ttgagaccat tgctgataga acgatatcct     240

ggatcgcccg gaagctattc tgctcgtcag cacatcatgc aacgaattca gagacttcag     300

gctgagtggg tcgtggaagt tgacaccttc ctgagtagga ctccctatgg ctatcggtcc     360

ttctcaaata tcatcagcac tcttaacccg gaagcgaaac gacacctggt cctcgcctgc     420

cactacgact ccaaatattt tcctcgatgg gacagcagag tgtttgtggg agccacggat     480

tcagctgtgc catgtgcaat gatgttggaa cttgcccgtg ccttagacaa gaaactccat     540

tccttgaagg atgtctctgg ttccaagcca gatctctcac tccagctaat tttctttgat      600

ggtgaagagg cttttcatca ctggtcccct caagattctc tgtatgggtc tcggcactta     660

gctcagaaga tggcatcaag ccctcaccct cctggatcaa gaggcaccaa ccaactggat     720

ggcatggatc tgttggtctt actagattta attggagcag caaatccaac attccctaat     780

tttttcccca agactaccag atggtttaat agacttcaag caattgaaaa ggaactctat     840

gaattgggat tactcaagga tcattctttg gagaggaagt attttcagaa ttttggctat     900

ggaaatatta tccaggatga ccacattcca tttttaagaa aaggtgtccc agttcttcac     960

ctgatagctt ctcctttccc tgaagtctgg cacaccatgg atgacaatga agaaaatcta    1020

catgcgtcaa ccattgacaa tctcaacaaa atcattcaag tctttgtgtt ggaatatctt    1080

cacttgtaa                                                            1089

<210>  10
<211>  362
<212>  PRT
<213>  Mus musculus

<400>  10

Met Ala Gly Ser Glu Asp Lys Leu Val Val Gly Thr Leu His Leu Leu
1               5                   10                  15

Leu Leu Gln Ala Thr Val Leu Ser Leu Thr Ala Gly Asn Leu Ser Leu
            20                  25                  30

Val Ser Ala Ala Trp Thr Gln Glu Lys Asn His His Gln Pro Ala His
        35                  40                  45

Leu Asn Ser Ser Ser Leu Gln Gln Val Ala Glu Gly Thr Ser Ile Ser
    50                  55                  60

Glu Met Trp Gln Asn Asp Leu Arg Pro Leu Leu Ile Glu Arg Tyr Pro
65                  70                  75                  80

Gly Ser Pro Gly Ser Tyr Ser Ala Arg Gln His Ile Met Gln Arg Ile
                85                  90                  95

Gln Arg Leu Gln Ala Glu Trp Val Val Glu Val Asp Thr Phe Leu Ser
            100                 105                 110

Arg Thr Pro Tyr Gly Tyr Arg Ser Phe Ser Asn Ile Ile Ser Thr Leu
        115                 120                 125

Asn Pro Glu Ala Lys Arg His Leu Val Leu Ala Cys His Tyr Asp Ser
    130                 135                 140

Lys Tyr Phe Pro Arg Trp Asp Ser Arg Val Phe Val Gly Ala Thr Asp
145                 150                 155                 160

Ser Ala Val Pro Cys Ala Met Met Leu Glu Leu Ala Arg Ala Leu Asp
                165                 170                 175

Lys Lys Leu His Ser Leu Lys Asp Val Ser Gly Ser Lys Pro Asp Leu
            180                 185                 190

Ser Leu Arg Leu Ile Phe Phe Asp Gly Glu Glu Ala Phe His His Trp
        195                 200                 205

Ser Pro Gln Asp Thr Leu Tyr Gly Ser Arg His Leu Ala Gln Lys Met
    210                 215                 220

Ala Ser Ser Pro His Pro Pro Gly Ser Arg Gly Thr Asn Gln Leu Asp
225                 230                 235                 240

Gly Met Asp Leu Leu Val Leu Leu Asp Leu Ile Gly Ala Ala Asn Pro
                245                 250                 255

Thr Phe Pro Asn Phe Phe Pro Lys Thr Thr Arg Trp Phe Asn Arg Leu
            260                 265                 270

Gln Ala Ile Glu Lys Glu Leu Tyr Glu Leu Gly Leu Leu Lys Asp His
        275                 280                 285

Ser Leu Glu Arg Lys Tyr Phe Gln Asn Phe Gly Tyr Gly Asn Ile Ile
    290                 295                 300

Gln Asp Asp His Ile Pro Phe Leu Arg Lys Gly Val Pro Val Leu His

305                 310                 315                 320

Leu Ile Ala Ser Pro Ser Pro Glu Val Trp His Thr Met Asp Asp Asn
                325                 330                 335

Glu Glu Asn Leu His Ala Ser Thr Ile Asp Asn Leu Asn Lys Ile Ile
            340                 345                 350

Gln Val Phe Val Leu Glu Tyr Leu His Leu
        355                 360

<210>  11
<211>  32
<212>  DNA
<213>  Artificial sequence

<220>
<223>  DNA Primer

<400>  11
atatgcatgc atggcaggca gcgaagacaa gc                                    32

<210>  12
<211>  40
<212>  DNA
<213>  Artificial sequence

<220>
<223>  DNA Primer

<400>  12
atataagctt ttacaagtga agatattcca acacaaagac                            40

<210>  13
<211>  33
<212>  DNA
<213>  Artificial sequence

<220>
<223>  DNA Primer

<400>  13
atactcgaga aaagagcctg gacgcaggag aag                                   33

<210>  14
<211>  31
<212>  DNA
<213>  Artificial sequence

<220>
<223>  DNA Primer

<400>  14
atatctagat tacaagtgaa gatattccaa c                                     31

<210>  15
<211>  361

<212>  PRT
<213>  human

<400>  15

Met Ala Gly Gly Arg His Arg Arg Val Val Gly Thr Leu His Leu Leu
1               5                   10                  15

Leu Leu Val Ala Ala Leu Pro Trp Ala Ser Arg Gly Val Ser Pro Ser
            20                  25                  30

Ala Ser Ala Trp Pro Glu Glu Lys Asn Tyr His Gln Pro Ala Ile Leu
        35                  40                  45

Asn Ser Ser Ala Leu Arg Gln Ile Ala Glu Gly Thr Ser Ile Ser Glu
    50                  55                  60

Met Trp Gln Asn Asp Leu Gln Pro Leu Leu Ile Glu Arg Tyr Pro Gly
65                  70                  75                  80

Ser Pro Gly Ser Tyr Ala Ala Arg Gln His Ile Met Gln Arg Ile Gln
                85                  90                  95

Arg Leu Gln Ala Asp Trp Val Leu Glu Ile Asp Thr Phe Leu Ser Gln
            100                 105                 110

Thr Pro Tyr Gly Tyr Arg Ser Phe Ser Asn Ile Ile Ser Thr Leu Asn
        115                 120                 125

Pro Thr Ala Lys Arg His Leu Val Leu Ala Cys His Tyr Asp Ser Lys
    130                 135                 140

Tyr Phe Ser His Trp Asn Asn Arg Val Phe Val Gly Ala Thr Asp Ser
145                 150                 155                 160

Ala Val Pro Cys Ala Met Met Leu Glu Leu Ala Arg Ala Leu Asp Lys
                165                 170                 175

Lys Leu Leu Ser Leu Lys Thr Val Ser Asp Ser Lys Pro Asp Leu Ser
            180                 185                 190

Leu Gln Leu Ile Phe Phe Asp Gly Glu Glu Ala Phe Leu His Trp Ser
        195                 200                 205

Pro Gln Asp Ser Leu Tyr Gly Ser Arg His Leu Ala Ala Lys Met Ala
    210                 215                 220

Ser Thr Pro His Pro Pro Gly Ala Arg Gly Thr Ser Gln Leu His Gly
225                 230                 235                 240

Met Asp Leu Leu Val Leu Leu Asp Leu Ile Gly Ala Pro Asn Pro Thr
                245                 250                 255

Phe Pro Asn Phe Phe Pro Asn Ser Ala Arg Trp Phe Glu Arg Leu Gln
            260                 265                 270

Ala Ile Glu His Glu Leu His Glu Leu Gly Leu Leu Lys Asp His Ser
        275                 280                 285

Leu Glu Gly Arg Tyr Phe Gln Asn Tyr Ser Tyr Gly Gly Val Ile Gln
    290                 295                 300

Asp Asp His Ile Pro Phe Leu Arg Arg Gly Val Pro Val Leu His Leu
305                 310                 315                 320

Ile Pro Ser Pro Phe Pro Glu Val Trp His Thr Met Asp Asp Asn Glu
                325                 330                 335

Glu Asn Leu Asp Glu Ser Thr Ile Asp Asn Leu Asn Lys Ile Leu Gln
            340                 345                 350

Val Phe Val Leu Glu Tyr Leu His Leu
        355                 360

<210>  16
<211>  382
<212>  PRT
<213>  human

<400>  16

Met Arg Ser Gly Gly Arg Gly Arg Pro Arg Leu Arg Leu Gly Glu Arg
1               5                   10                  15

Gly Leu Met Glu Pro Leu Leu Pro Pro Lys Arg Arg Leu Leu Pro Arg
            20                  25                  30

Val Arg Leu Leu Pro Leu Leu Leu Ala Leu Ala Val Gly Ser Ala Phe
        35                  40                  45

Tyr Thr Ile Trp Ser Gly Trp His Arg Arg Thr Glu Glu Leu Pro Leu
    50                  55                  60

Gly Arg Glu Leu Arg Val Pro Leu Ile Gly Ser Leu Pro Glu Ala Arg
65                  70                  75                  80

Leu Arg Arg Val Val Gly Gln Leu Asp Pro Gln Arg Leu Trp Ser Thr
                85                  90                  95

Tyr Leu Arg Pro Leu Leu Val Val Arg Thr Pro Gly Ser Pro Gly Asn
            100                 105                 110

Leu Gln Val Arg Lys Phe Leu Glu Ala Thr Leu Arg Ser Leu Thr Ala
        115                 120                 125

Gly Trp His Val Glu Leu Asp Pro Phe Thr Ala Ser Thr Pro Leu Gly
    130                 135                 140

Pro Val Asp Phe Gly Asn Val Val Ala Thr Leu Asp Pro Arg Ala Ala
145                 150                 155                 160

Arg His Leu Thr Leu Ala Cys His Tyr Asp Ser Lys Leu Phe Pro Pro
                165                 170                 175

Gly Ser Thr Pro Phe Val Gly Ala Thr Asp Ser Ala Val Pro Cys Ala
            180                 185                 190

Leu Leu Leu Glu Leu Ala Gln Ala Leu Asp Leu Glu Leu Ser Arg Ala
        195                 200                 205

Lys Lys Gln Ala Ala Pro Val Thr Leu Gln Leu Leu Phe Leu Asp Gly
    210                 215                 220

Glu Glu Ala Leu Lys Glu Trp Gly Pro Lys Asp Ser Leu Tyr Gly Ser
225                 230                 235                 240

Arg His Leu Ala Gln Leu Met Glu Ser Ile Pro His Ser Pro Gly Pro
                245                 250                 255

Thr Arg Ile Gln Ala Ile Glu Leu Phe Met Leu Leu Asp Leu Leu Gly
            260                 265                 270

Ala Pro Asn Pro Thr Phe Tyr Ser His Phe Pro Arg Thr Val Arg Trp
        275                 280                 285

Phe His Arg Leu Arg Ser Ile Glu Lys Arg Leu His Arg Leu Asn Leu
    290                 295                 300

Leu Gln Ser His Pro Gln Glu Val Met Tyr Phe Gln Pro Gly Glu Pro
305                 310                 315                 320

Phe Gly Ser Val Glu Asp Asp His Ile Pro Phe Leu Arg Arg Gly Val
                325                 330                 335

Pro Val Leu His Leu Ile Ser Thr Pro Phe Pro Ala Val Trp His Thr
            340                 345                 350

Pro Ala Asp Thr Glu Val Asn Leu His Pro Pro Thr Val His Asn Leu
        355                 360                 365

Cys Arg Ile Leu Ala Val Phe Leu Ala Glu Tyr Leu Gly Leu
    370                 375                 380

<210>  17
<211>  364
<212>  PRT
<213>  human

<400>  17

<210>  18
<211>  382
<212>  PRT
<213>  Macaca fascicularis

<400>  18

Met Arg Ser Gly Gly Arg Gly Arg Pro Arg Leu Arg Leu Gly Glu Arg
1               5                   10                  15

Gly Val Met Glu Pro Leu Leu Pro Pro Lys Arg Arg Leu Leu Pro Arg
            20                  25                  30

Val Arg Leu Leu Pro Leu Leu Leu Ala Leu Ala Val Gly Ser Ala Phe
        35                  40                  45

Tyr Thr Ile Trp Ser Gly Trp His Arg Arg Thr Glu Glu Leu Pro Leu
    50                  55                  60

Gly Arg Glu Leu Arg Val Pro Leu Ile Gly Ser Leu Pro Glu Ala Arg
65                  70                  75                  80

Leu Arg Arg Val Val Gly Gln Leu Asp Pro Gln Arg Leu Trp Gly Thr
                85                  90                  95

Tyr Leu Arg Pro Leu Leu Val Val Arg Thr Pro Gly Ser Pro Gly Asn
            100                 105                 110

Leu Gln Val Arg Lys Phe Leu Glu Ala Thr Leu Arg Ser Leu Thr Ala
        115                 120                 125

Gly Trp His Val Glu Leu Asp Pro Phe Thr Ala Ser Thr Pro Leu Gly
    130                 135                 140

Pro Val Asp Phe Gly Asn Val Val Ala Thr Leu Asp Pro Gly Ala Ala
145                 150                 155                 160

Arg His Leu Thr Leu Ala Cys His Tyr Asp Ser Lys Leu Phe Pro Pro
                165                 170                 175

Gly Ser Thr Pro Phe Val Gly Ala Thr Asp Ser Ala Val Pro Cys Ala
            180                 185                 190

Leu Leu Leu Glu Leu Ala Gln Ala Leu Asp Leu Glu Leu Ser Arg Ala
        195                 200                 205

Lys Glu Gln Ala Ala Pro Val Thr Leu Gln Leu Leu Phe Leu Asp Gly
    210                 215                 220

Glu Glu Ala Leu Lys Glu Trp Gly Pro Lys Asp Ser Leu Tyr Gly Ser
225                 230                 235                 240

Arg His Leu Ala Gln Leu Met Glu Ser Ile Pro His Ser Pro Gly Pro
                245                 250                 255

Thr Arg Ile Gln Ala Ile Glu Leu Phe Met Leu Leu Asp Leu Leu Gly
            260                 265                 270

Ala Pro Asn Pro Thr Phe Tyr Ser His Phe Pro Arg Thr Val Arg Trp
        275                 280                 285

Phe His Arg Leu Arg Ser Ile Glu Lys Arg Leu His Arg Leu Asn Leu
    290                 295                 300

Leu Gln Ser His Pro Gln Glu Val Met Tyr Phe Gln Pro Gly Glu Pro
305                 310                 315                 320

Phe Gly Ser Val Glu Asp Asp His Ile Pro Phe Leu Arg Arg Gly Val
                325                 330                 335

Pro Val Leu His Leu Ile Ser Thr Pro Phe Pro Ala Val Trp His Thr
            340                 345                 350

Pro Ala Asp Thr Glu Ala Asn Leu His Pro Pro Thr Val His Asn Leu
        355                 360                 365

Ser Arg Ile Leu Ala Val Phe Leu Ala Glu Tyr Leu Gly Leu
    370                 375                 380

<210>  19
<211>  382
<212>  PRT
<213>  Macaca mulatta

<400>  19

Met Arg Ser Gly Gly Arg Gly Arg Pro Arg Leu Arg Leu Gly Glu Arg
1               5                   10                  15

Gly Val Met Glu Pro Leu Leu Pro Pro Lys Arg Arg Leu Leu Pro Arg
            20                  25                  30

Val Arg Leu Leu Pro Leu Leu Leu Ala Leu Ala Val Gly Ser Ala Phe
        35                  40                  45

Tyr Thr Ile Trp Ser Gly Trp His Arg Arg Thr Glu Glu Leu Pro Leu
    50                  55                  60

Gly Arg Glu Leu Arg Val Pro Leu Ile Gly Ser Leu Pro Glu Ala Arg
65                  70                  75                  80

Leu Arg Arg Val Val Gly Gln Leu Asp Pro Gln Arg Leu Trp Gly Thr
                85                  90                  95

Tyr Leu Arg Pro Leu Leu Val Val Arg Thr Pro Gly Ser Pro Gly Asn
            100                 105                 110

Leu Gln Val Arg Lys Phe Leu Glu Ala Thr Leu Arg Ser Leu Thr Ala
        115                 120                 125

Gly Trp His Val Glu Leu Asp Pro Phe Thr Ala Ser Thr Pro Leu Gly
    130                 135                 140

Pro Val Asp Phe Gly Asn Val Val Ala Thr Leu Asp Pro Gly Ala Ala
145                 150                 155                 160

Arg His Leu Thr Leu Ala Cys His Tyr Asp Ser Lys Leu Phe Pro Pro
                165                 170                 175

Gly Ser Thr Pro Phe Val Gly Ala Thr Asp Ser Ala Val Pro Cys Ala
            180                 185                 190

Leu Leu Leu Glu Leu Ala Gln Ala Leu Asp Leu Glu Leu Ser Arg Ala
        195                 200                 205

Lys Glu Gln Ala Ala Pro Val Thr Leu Gln Leu Leu Phe Leu Asp Gly
    210                 215                 220

Glu Glu Ala Leu Lys Glu Trp Gly Pro Lys Asp Ser Leu Tyr Gly Ser
225                 230                 235                 240

Arg His Leu Ala Gln Leu Met Glu Ser Ile Pro His Ser Pro Gly Pro
                245                 250                 255

Thr Arg Ile Gln Ala Ile Glu Leu Phe Met Leu Leu Asp Leu Leu Gly
            260                 265                 270

Ala Pro Asn Pro Thr Phe Tyr Ser His Phe Pro Arg Thr Val Arg Trp
        275                 280                 285

Phe His Arg Leu Arg Ser Ile Glu Lys Arg Leu His Arg Leu Asn Leu
    290                 295                 300

Leu Gln Ser His Pro Gln Glu Val Met Tyr Phe Gln Pro Gly Glu Pro
305                 310                 315                 320

Phe Gly Ser Val Glu Asp Asp His Ile Pro Phe Leu Arg Arg Gly Val
                325                 330                 335

Pro Val Leu His Leu Ile Ser Thr Pro Phe Pro Ala Val Trp His Thr
            340                 345                 350

Pro Ala Asp Thr Glu Ala Asn Leu His Pro Pro Thr Val His Asn Leu
        355                 360                 365

Ser Arg Ile Leu Ala Val Phe Leu Ala Glu Tyr Leu Gly Leu
    370                 375                 380

<210>  20
<211>  383
<212>  PRT
<213>  Canis familiaris

<400>  20
   Met Pro Ser Gly Gly Arg Gly Arg Ser Arg Leu Arg Leu Gly Glu Arg
   1               5                   10                  15
   Gly Leu Leu Glu Pro Pro Ser Pro Pro Lys Arg Arg Leu Leu Pro Arg
               20                  25                  30
   Ala His Phe Leu Pro Leu Leu Leu Leu Ala Leu Ala Leu Ala Ser Ala
           35                  40                  45
   Thr Tyr Thr Ile Trp Ser Gly Trp His His Gln Thr Glu Glu Leu Pro
       50                  55                  60
   Arg Gly Arg Glu Leu Arg Gly Arg Leu Ile Gly Ser Leu Ser Glu Ala
   65                  70                  75                  80
   Arg Leu Arg Arg Val Val Gly Gln Leu Asp Pro His Arg Leu Trp Asn
                   85                  90                  95
   Thr Tyr Leu Arg Pro Leu Leu Val Val Arg Thr Pro Gly Ser Pro Gly
               100                 105                 110
   Asn Leu Gln Val Arg Lys Phe Leu Glu Ala Thr Leu Arg Thr Leu Thr
           115                 120                 125
   Ala Gly Trp His Val Glu Leu Asp Pro Phe Thr Ala Leu Thr Pro Leu
       130                 135                 140
   Gly Pro Leu Asp Phe Gly Asn Val Val Ala Thr Leu Asp Pro Gly Ala
   145                 150                 155                 160
   Ala Arg His Leu Thr Leu Ala Cys His Tyr Asp Ser Lys Leu Phe Ala
                   165                 170                 175
   Ser Glu Ser Val Pro Phe Val Gly Ala Thr Asp Ser Ala Val Pro Cys
               180                 185                 190
   Ala Leu Leu Leu Glu Leu Ala Gln Ala Leu Asp Arg Glu Leu Ser Arg
           195                 200                 205
   Ala Lys Glu Gln Glu Ala Pro Val Thr Leu Gln Leu Leu Phe Leu Asp
       210                 215                 220
   Gly Glu Glu Ala Leu Lys Glu Trp Gly Pro Thr Asp Ser Leu Tyr Gly
   225                 230                 235                 240
   Ser Arg His Leu Ala Gln Leu Met Glu Ser Ala Pro His Ser Pro Gly
                   245                 250                 255
   Pro Thr Arg Ile Gln Ala Ile Glu Leu Phe Met Leu Leu Asp Leu Leu
               260                 265                 270
   Gly Ala Pro Asn Pro Asn Phe Tyr Ser His Phe Pro His Thr Ala Arg
           275                 280                 285
   Trp Phe His Arg Leu Arg Ser Ile Glu Lys Arg Leu His Arg Met Asn
   290                 295                 300
   Leu Leu Gln Ser His Pro Gln Glu Val Met Tyr Phe Gln Pro Gly Glu
   305                 310                 315                 320
   Pro Pro Gly Ser Val Glu Asp Asp His Ile Pro Phe Leu Arg Arg Gly
                   325                 330                 335
   Val Pro Val Leu His Leu Ile Ser Met Pro Phe Pro Ser Val Trp His
               340                 345                 350
   Thr Pro Asp Asp Ser Glu Ala Asn Leu His Pro Pro Thr Val His Asn
           355                 360                 365
   Leu Ser Arg Ile Leu Ala Val Phe Leu Ala Glu Tyr Leu Gly Leu
       370                 375                 380

<210>  21
<211>  383
<212>  PRT
<213>  Rattus norvegicus

<400>  21

Met Ser Pro Ala Ser Arg Gly Arg Ser Arg Gln Arg Leu Gly Asp Arg
1               5                   10                  15

Gly Leu Met Lys Pro Pro Ser Leu Ser Lys Arg Arg Leu Leu Pro Arg
            20                  25                  30

Val Gln Leu Leu Pro Leu Leu Leu Leu Ala Leu Ala Leu Gly Leu Ala
        35                  40                  45

Phe Tyr Ile Val Trp Asn Ser Trp His Pro Gly Val Glu Glu Val Ser
    50                  55                  60

Arg Ser Arg Asp Leu Arg Val Pro Leu Ile Gly Ser Leu Ser Glu Ala
65                  70                  75                  80

Lys Leu Arg Leu Val Val Gly Gln Leu Asp Pro Gln Arg Leu Trp Gly
                85                  90                  95

Thr Phe Leu Arg Pro Leu Leu Ile Val Arg Pro Pro Gly Ser Pro Gly
            100                 105                 110

Asn Leu Gln Val Arg Lys Phe Leu Glu Ala Thr Leu Gln Ser Leu Ser
        115                 120                 125

Ala Gly Trp His Val Glu Leu Asp Pro Phe Thr Ala Ser Thr Pro Leu
    130                 135                 140

Gly Pro Leu Asp Phe Gly Asn Val Val Ala Thr Leu Asp Pro Gly Ala
145                 150                 155                 160

Ala Arg His Leu Thr Leu Ala Cys His Tyr Asp Ser Lys Phe Phe Pro
                165                 170                 175

Pro Gly Leu Pro Pro Phe Val Gly Ala Thr Asp Ser Ala Val Pro Cys
            180                 185                 190

Ala Leu Leu Leu Glu Leu Val Gln Ala Leu Asp Val Met Leu Ser Arg
        195                 200                 205

Ile Lys Gln Gln Ala Ala Pro Val Thr Leu Gln Leu Leu Phe Leu Asp
    210                 215                 220

Gly Glu Glu Ala Leu Lys Glu Trp Gly Pro Lys Asp Ser Leu Tyr Gly
225                 230                 235                 240

Ser Arg His Leu Ala Gln Ile Met Glu Ser Ile Pro His Ser Pro Gly
                245                 250                 255

Pro Thr Arg Ile Gln Ala Ile Glu Leu Phe Val Leu Leu Asp Leu Leu
            260                 265                 270

Gly Ala Pro Ser Pro Ile Phe Phe Ser His Phe Pro Arg Thr Ala Arg
        275                 280                 285

Trp Phe Gln Arg Leu Arg Ser Ile Glu Lys Arg Leu His Arg Leu Asn
    290                 295                 300

Leu Leu Gln Ser His Pro Gln Glu Val Met Tyr Phe Gln Pro Gly Glu
305                 310                 315                 320

Pro Pro Gly Pro Val Glu Asp Asp His Ile Pro Phe Leu Arg Arg Gly
                325                 330                 335

Val Pro Val Leu His Leu Ile Ala Met Pro Phe Pro Ala Val Trp His
            340                 345                 350

Thr Pro Ala Asp Thr Glu Ala Asn Leu His Pro Pro Thr Val His Asn
        355                 360                 365

Leu Ser Arg Ile Leu Ala Val Phe Leu Ala Glu Tyr Leu Gly Leu
    370                 375                 380

<210>  22
<211>  383
<212>  PRT
<213>  Mus musculus

<400>  22

Met Ser Pro Gly Ser Arg Gly Arg Pro Arg Gln Arg Leu Glu Asp Arg
1               5                   10                  15

Gly Leu Met Lys Pro Pro Ser Leu Ser Lys Arg Arg Leu Leu Pro Arg
            20                  25                  30

Val Gln Phe Leu Pro Leu Leu Leu Leu Ala Leu Ala Met Gly Leu Ala
        35                  40                  45

Phe Tyr Ile Val Trp Asn Ser Trp His Pro Gly Val Glu Glu Met Ser
    50                  55                  60

Arg Ser Arg Asp Leu Arg Val Pro Leu Ile Gly Ser Leu Ser Glu Ala
65                  70                  75                  80

Lys Leu Arg Leu Val Val Gly Gln Leu Asp Pro Gln Arg Leu Trp Gly
                85                  90                  95

Thr Phe Leu Arg Pro Leu Leu Ile Val Arg Pro Pro Gly Ser Ser Gly
            100                 105                 110

Asn Leu Gln Val Arg Lys Phe Leu Glu Ala Thr Leu Gln Ser Leu Ser
        115                 120                 125

Ala Gly Trp His Val Glu Leu Asp Pro Phe Thr Ala Ser Thr Pro Leu
    130                 135                 140

Gly Pro Leu Asp Phe Gly Asn Val Val Ala Thr Leu Asp Pro Gly Ala
145                 150                 155                 160

Ala Arg His Leu Thr Leu Ala Cys His Tyr Asp Ser Lys Phe Phe Pro
                165                 170                 175

Pro Gly Leu Pro Pro Phe Val Gly Ala Thr Asp Ser Ala Val Pro Cys
            180                 185                 190

Ala Leu Leu Leu Glu Leu Val Gln Ala Leu Asp Ala Met Leu Ser Arg
        195                 200                 205

Ile Lys Gln Gln Ala Ala Pro Val Thr Leu Gln Leu Leu Phe Leu Asp
    210                 215                 220

Gly Glu Glu Ala Leu Lys Glu Trp Gly Pro Lys Asp Ser Leu Tyr Gly
225                 230                 235                 240

Ser Arg His Leu Ala Gln Ile Met Glu Ser Ile Pro His Ser Pro Gly
                245                 250                 255

Pro Thr Arg Ile Gln Ala Ile Glu Leu Phe Val Leu Leu Asp Leu Leu
            260                 265                 270

Gly Ala Ser Ser Pro Ile Phe Phe Ser His Phe Pro Arg Thr Ala Arg
        275                 280                 285

Trp Phe Gln Arg Leu Arg Ser Ile Glu Lys Arg Leu His Arg Leu Asn
    290                 295                 300

Leu Leu Gln Ser His Pro Gln Glu Val Met Tyr Phe Gln Pro Gly Glu
305                 310                 315                 320

Pro Pro Gly Pro Val Glu Asp Asp His Ile Pro Phe Leu Arg Arg Gly
                325                 330                 335

Val Pro Val Leu His Leu Ile Ala Thr Pro Phe Pro Ala Val Trp His
            340                 345                 350

Thr Pro Ala Asp Thr Glu Ala Asn Leu His Pro Pro Thr Val His Asn
        355                 360                 365

Leu Ser Arg Ile Leu Ala Val Phe Leu Ala Glu Tyr Leu Gly Leu
    370                 375                 380

<210>  23
<211>  383
<212>  PRT
<213>  Bos taurus

<400>  23

Met Pro Ser Gly Gly Arg Gly Arg Pro Arg Leu Gln Val Gly Glu Arg
1               5                   10                  15

Ser Leu Leu Glu Arg Pro Ser Pro Pro Lys Arg Arg Leu Ile Pro Arg
            20                  25                  30

Ala Gln Leu Leu Pro Gln Leu Leu Leu Ala Leu Thr Val Ala Ser Val
        35                  40                  45

Phe Tyr Thr Ile Trp Arg Ile Trp His Ser Gln Thr Glu Glu Leu Pro
    50                  55                  60

Leu Gly Arg Glu Leu Arg Gly Pro Leu Ile Gly Ser Leu Pro Glu Ala
65                  70                  75                  80

Arg Val Arg Arg Val Val Gly Gln Leu Asp Pro His Arg Leu Trp Asn
                85                  90                  95

Thr Phe Leu Arg Pro Leu Leu Val Val Arg Thr Pro Gly Ser Pro Gly
            100                 105                 110

Asn Leu Gln Val Arg Lys Phe Leu Glu Ala Thr Leu Arg Thr Leu Ser
        115                 120                 125

Ala Gly Trp His Ile Glu Leu Asp Ser Phe Thr Ala Ser Thr Pro Val
    130                 135                 140

Gly Pro Leu Asp Phe Ser Asn Val Val Ala Thr Leu Asp Pro Gly Ala
145                 150                 155                 160

Ala Arg His Leu Thr Leu Ala Cys His Tyr Asp Ser Lys Leu Phe Pro
                165                 170                 175

Ser Asp Ser Ala Pro Phe Val Gly Ala Thr Asp Ser Ala Val Pro Cys
            180                 185                 190

Ser Leu Leu Leu Glu Leu Ala Gln Ala Leu Asp Gln Glu Leu Gly Lys
        195                 200                 205

Ala Lys Glu Arg Ala Ala Pro Met Thr Leu Gln Leu Ile Phe Leu Asp
    210                 215                 220

Gly Glu Glu Ala Leu Lys Gln Trp Gly Pro Lys Asp Ser Leu Tyr Gly
225                 230                 235                 240

Ser Arg His Leu Ala Gln Leu Met Glu Ser Thr Pro His Gly Leu Gly
                245                 250                 255

Ser Thr Arg Ile Gln Ala Ile Glu Leu Phe Met Leu Leu Asp Leu Leu
            260                 265                 270

Gly Ala Pro Asn Pro Thr Phe Tyr Ser His Phe Pro Arg Thr Ala Arg
        275                 280                 285

Trp Phe His Arg Leu Arg Ser Ile Glu Lys Arg Leu His Arg Leu Asn
    290                 295                 300

Leu Leu Gln Ser His Pro Trp Glu Val Met Tyr Phe Gln Thr Gly Glu
305                 310                 315                 320

Pro Pro Gly Ser Val Glu Asp Asp His Ile Pro Phe Leu Arg Arg Gly
                325                 330                 335

Val Pro Val Leu His Leu Ile Ala Thr Pro Phe Pro Ser Val Trp His
            340                 345                 350

Thr Ser Asp Asp Ser Glu Ala Asn Leu His Pro Pro Thr Val His Asn

        355                 360                 365

Leu Ser Arg Ile Leu Ala Val Phe Leu Ala Glu Tyr Leu Gly Leu
    370                 375                 380

<210> 24
<211> 481
<212> PRT
<213> human

<400> 24

<210>  25
<211>  359
<212>  PRT
<213>  human

<400>  25

Ala Ala Met Arg Ser Gly Gly Arg Gly Arg Pro Arg Leu Arg Leu Gly
1               5                   10                  15

Glu Arg Gly Leu Met Glu Pro Leu Leu Pro Pro Lys Arg Arg Leu Leu
            20                  25                  30

Pro Arg Val Arg Leu Leu Pro Leu Leu Leu Ala Leu Ala Val Gly Ser
        35                  40                  45

Ala Phe Tyr Thr Ile Trp Ser Gly Trp His Arg Arg Thr Glu Glu Leu
    50                  55                  60

Pro Leu Gly Arg Glu Leu Arg Val Pro Leu Ile Gly Ser Leu Pro Glu
65                  70                  75                  80

Ala Arg Leu Arg Arg Val Val Gly Gln Leu Asp Pro Gln Arg Leu Trp
                85                  90                  95

Ser Thr Tyr Leu Arg Pro Leu Leu Val Val Arg Thr Pro Gly Ser Pro
            100                 105                 110

Gly Asn Leu Gln Val Arg Lys Ala Ala Pro Val Thr Leu Gln Leu Leu
        115                 120                 125

Phe Leu Asp Gly Glu Glu Ala Leu Lys Glu Trp Gly Pro Lys Asp Ser
    130                 135                 140

Leu Tyr Gly Ser Arg His Leu Ala Gln Leu Met Glu Ser Ile Pro His
145                 150                 155                 160

Ser Pro Gly Pro Thr Arg Ile Gln Ala Ile Glu Leu Phe Met Leu Leu
                165                 170                 175

Asp Leu Leu Gly Ala Pro Asn Pro Thr Phe Tyr Ser His Phe Pro Arg
            180                 185                 190

Thr Val Arg Trp Phe His Arg Leu Arg Ser Ile Glu Lys Arg Leu His
        195                 200                 205

Arg Leu Asn Leu Leu Gln Ser His Pro Gln Glu Val Met Tyr Phe Gln
    210                 215                 220

Pro Gly Glu Pro Phe Gly Ser Val Glu Asp Asp His Ile Pro Phe Leu
225                 230                 235                 240

Arg Arg Gly Val Pro Val Leu His Leu Ile Ser Thr Pro Phe Pro Ala
                245                 250                 255

Val Trp His Thr Pro Ala Asp Thr Glu Val Asn Leu His Pro Pro Thr
            260                 265                 270

Val His Asn Leu Cys Arg Ile Leu Ala Val Phe Leu Ala Glu Tyr Leu
        275                 280                 285

Gly Leu Arg Ala Trp Pro Met Thr Val Glu Arg Thr Val Arg Glu Lys
    290                 295                 300

Val Pro Ala Gly Ala Ser Glu Ala Gln Ala Gly Ser Ala Gly Val Leu
305                 310                 315                 320

Val Cys Pro Phe His Thr Phe Val Ser Leu Cys Tyr Asn Trp Lys Thr
                325                 330                 335

Phe Phe Leu Leu Ile Val Ser Ser Cys His Pro Ser Arg Thr Gly Lys
            340                 345                 350

Arg Pro Leu Trp Asp Asp Ser
        355

<210>  26
<211>  1086
<212>  DNA
<213>  human

<400>  26
atggcaggcg gaagacaccg gcgcgtcgtg ggcaccctcc acctgctgct gctggtggcc      60

gccctgccct gggcatccag gggggtcagt ccgagtgcct cagcctggcc agaggagaag     120

aattaccacc agccagccat tttgaattca tcggctcttc ggcaaattgc agaaggcacc     180

agtatctctg aaatgtggca aaatgactta cagccattgc tgatagagcg atacccggga     240

tcccctggaa gctatgctgc tcgtcagcac atcatgcagc gaattcagag gcttcaggct     300

gactgggtct tggaaataga caccttcttg agtcagacac cctatgggta ccggtctttc     360

tcaaatatca tcagcaccct caatcccact gctaaacgac atttggtcct cgcctgccac     420

tatgactcca agtatttttc ccactggaac aacagagtgt ttgtaggagc cactgattca     480

gccgtgccat gtgcaatgat gttggaactt gctcgtgcct tagacaagaa actcctttcc     540

ttaaagactg tttcagactc caagccagat ttgtcactcc agctgatctt ctttgatggt 600

gaagaggctt ttcttcactg gtctcctcaa gattctctct atgggtctcg acacttagct 660

gcaaagatgg catcgacccc gcacccacct ggagcgagag gcaccagcca actgcatggc 720

atggatttat tggtcttatt ggatttgatt ggagctccaa acccaacgtt tcccaatttt 780

tttccaaact cagccaggtg gttcgaaaga cttcaagcaa ttgaacatga acttcatgaa 840

ttgggtttgc tcaaggatca ctctttggag ggcggtattt tccagaatta cagttatgga 900

ggtgtgattc aggatgacca tattccattt ttaagaagag gtgttccagt tctgcatctg 960

ataccgtctc ctttccctga agtctggcac accatggatg acaatgaaga aaatttggat 1020

gaatcaacca ttgacaatct aaacaaaatc ctacaagtct ttgtgttgga atatcttcat 1080

ttgtaa 1086

<210> 27
<211> 1149
<212> DNA
<213> human

<400> 27
atgcgttccg ggggccgcgg gcgaccccgc ctgcggctgg gggaacgtgg cctcatggag 60

ccactcttgc cgccgaagcg ccgcctgcta ccgcgggttc ggctcttgcc tctgttgctg 120

gcgctggccg tgggctcggc gttctacacc atttggagcg gctggcaccg caggactgag 180

gagctgccgc tgggccggga gctgcgggtc ccattgatcg gaagcctccc cgaagcccgg 240

ctgcggaggg tggtgggaca actggatcca cagcgtctct ggagcactta tctgcgcccc 300

ctgctggttg tgcgaacccc gggcagcccg ggaaatctcc aagtcagaaa gttcctggag 360

gccacgctgc ggtccctgac agcaggttgg cacgtggagc tggatccctt cacagcctca 420

acacccctgg ggccagtgga ctttggcaat gtggtggcca cactggaccc aagggctgcc 480

cgtcacctca cccttgcctg ccattatgac tcgaagctct tcccacccgg atcgaccccc 540

tttgtagggg ccacggattc ggctgtgccc tgtgccctgc tgctggagct ggcccaagca 600

cttgacctgg agctgagcag ggccaaaaaa caggcagccc cggtgaccct gcaactgctc 660

ttcttggatg gtgaagaggc gctgaaggag tggggaccca aggactccct ttacggttcc 720

cggcacctgg cccagctcat ggagtctata cctcacagcc ccggccccac caggatccag 780

gctattgagc tctttatgct tcttgatctc ctgggagccc ccaatcccac cttctacagc 840

cacttccctc gcacggtccg ctggttccat cggctgagga gcattgagaa gcgtctgcac 900

cgtttgaacc tgctgcagtc tcatccccag gaagtgatgt acttccaacc cggggagccc 960

tttggctctg tggaagacga ccacatcccc ttcctccgca gaggggtacc cgtgctccat 1020

ctcatctcca cgcccttccc tgctgtctgg cacacccctg cggacaccga ggtcaatctc 1080

cacccaccca cggtacacaa cttgtgccgc attctcgctg tgttcctggc tgaatacctg 1140

gggctctag                                                          1149

<210>  28
<211>  1145
<212>  DNA
<213>  human

<400>  28
atgcgttccg ggggccgcgg gcgaccccgc ctgcggctgg gggaacgtgg atggagccac      60

tcttgccgcc gaagcgccgc ctgctaccgc gggttcggct cttgcctctg ttgctggcgc     120

tggccgtggg ctcggcgttc tacaccattt ggagcggctg gcaccgcagg actgaggagc     180

tgccgctggg ccgggagctg cgggtcccat tgatcggaag cctccccgaa gcccggctgc     240

ggagggtggt gggacaactg gatccacagc gtctctggag cacttatctg cgccccctgc     300

tggttgtgcg aaccccgggc agcccgggaa atctccaagt cagaaagttc ctggaggcca     360

cgctgcggtc cctgacagca ggttggcacg tggagctgga tcccttcaca gcctcaacac     420

ccctggggcc agtggacttt ggcaatgtgg tggccacact ggacccaagg gctgcccgtc     480

acctcaccct tgcctgccat tatgactcga agctcttccc acccggatcg accccctttg     540

taggggccac ggattcggct gtgccctgtg ccctgctgct ggagctggcc caagcacttg     600

acctggagct gagcagggcc aaaaaacagg cagccccggt gaccctgcaa ctgctcttct     660

tggatggtga agaggcgctg aaggagtggg gacccaagga ctccctttac ggttcccggc     720

acctggccca gctcatggag tctatacctc acagccccgg ccccaccagg atccaggcta     780

ttgagctctt tatgcttctt gatctcctgg gagcccccaa tcccaccttc tacagccact     840

tccctcgcac ggtccgctgg ttccatcggc tgaggagcat tgagaagcgt ctgcaccgtt     900

tgaacctgct gcagtctcat ccccaggaag tgatgtactt ccaacccggg gagccctttg     960

gctctgtgga agacgaccac atccccttcc tccgcagagg ggtacccgtg ctccatctca    1020

tctccacgcc cttccctgct gtctggcaca cccctgcgga caccgaggtc aatctccacc    1080

cacccacggt acacaacttg tgccgcattc tcgctgtgtt cctggctgaa tacctggggc    1140

tctag                                                               1145

<210>  29
<211>  1149
<212>  DNA
<213>  Macaca fascicularis

<400>  29
atgcgttccg ggggccgcgg gcggccccgc ctgcggctag gggaacgtgg cgttatggag      60

ccactcttgc ccccgaagcg ccgcctgcta ccgcgggttc ggctcttgcc cctgttgctg     120

gcgctggccg tgggctcggc gttctacacc atttggagcg gctggcaccg caggactgag     180

gagctgccgc tgggccggga gctgcgggtc ccgttgatcg gaagccttcc cgaagcccgg     240

ctgcggaggg tggtgggaca actggaccca cagcgtctct ggggcactta tctgcgcccc 300

ctgctggttg tgcgaacccc aggcagcccg ggaaatctcc aagtcagaaa gttcctggag 360

gccacgctgc ggtccctgac agcaggttgg cacgtggagc tggatccctt cacagcctcg 420

acgcccctgg ggccagtgga ctttggcaat gtggtggcca cgctggaccc gggggctgcc 480

cgtcacctca cccttgcctg ccattatgac tcgaagctct tcccacccgg atcgaccccg 540

tttgtagggg ccacggactc ggctgtgccc tgtgccctgc tgctggagct ggcccaggca 600

cttgacctgg agctgagcag ggccaaagaa caggcagccc cggtgaccct gcaactgctc 660

ttcctggatg gtgaagaggc gctgaaggag tggggaccca aggactccct ttacggttcc 720

cggcacctgg cccagctcat ggagtctata cctcatagcc ccggccccac caggatccag 780

gctattgagc tctttatgct tcttgatctc ctgggagccc ccaatcccac cttctacagc 840

cacttccctc gcacggtccg ctggttccat cggctgagaa gcattgagaa gcgtctgcac 900

cgtttgaacc tgctgcagtc tcatccccag gaagtgatgt acttccaacc cggggagccc 960

ttcggctctg tggaagacga ccacatcccc ttcctccgca gaggggtccc cgtgctccat 1020

ctcatctcta cgcccttccc tgctgtctgg cacacccctg cggacacaga ggccaatctc 1080

cacccgccca cggtacacaa cttaagccgc attctggccg tgttcctggc tgaatacctg 1140

gggctctag 1149

<210> 30
<211> 1149
<212> DNA
<213> Macaca mulatta

<400> 30
atgcgttccg gggccgcggg gcggccccgc ctgcggctag ggaacgtggc cgttatggag 60

ccactcttgc ccccgaagcg ccgcctgcta ccgcgggttc ggctcttgcc cctgttgctg 120

gcgctggccg tgggctcggc gttctacacc atttggagcg gctggcaccg caggactgag 180

gagctgccgc tgggccggga gctgcgggtc ccgttgatcg gaagccttcc cgaagcccgg 240

ctgcggaggg tggtgggaca actggaccca cagcgtctct ggggcactta tctgcgcccc 300

ctgctggttg tgcgaacccc aggcagcccg ggaaatctcc aagtcagaaa gttcctggag 360

gccacgctgc ggtccctgac agcaggttgg cacgtggagc tggatccctt cacagcctcg 420

acgcccctgg ggccagtgga ctttggcaat gtggtggcca cgctggaccc gggggctgcc 480

cgtcacctca cccttgcctg ccattatgac tcgaagctct tcccacccgg atcgaccccg 540

tttgtagggg ccacagactc ggctgtgccc tgtgccctgc tgctggagct ggcccaggca 600

cttgacctgg agctgagcag ggccaaagaa caggcagccc cggtgaccct gcaactgctc 660

ttcctggatg gtgaagaggc gctgaaggag tggggaccca aggactccct ttacggttcc 720

cggcacctgg cccagctcat ggagtctata cctcatagcc ccggccccac caggatccag 780

gctattgagc tctttatgct tcttgatctc ctgggagccc ccaatcccac cttctacagc 840

cacttccctc gcacggtccg ctggttccat cggctgagaa gcattgagaa gcgtctgcac 900

cgtttgaacc tgctgcagtc tcatccccag gaagtgatgt acttccaacc cggggagccc 960

tttggctctg tggaagacga ccacatcccc ttcctccgca gaggggtccc cgtgctccat 1020

ctcatctcta cgcccttccc tgctgtctgg cacacccctg cggacacaga ggccaatctc 1080

cacccgccca cggtacacaa cttaagccgc attctggccg tgttcctggc tgaatacctg 1140

gggctctag 1149

<210> 31
<211> 1152
<212> DNA
<213> Canis familiaris

<400> 31
atgccttccg ggggccgcgg gcggtcccgg ctacggctcg gggaacgtgg cctcttggag 60

ccgccctccc cgcccaagcg ccgcctgctc ccgcgggcgc acttcttgcc tctgcttctg 120

ctggccctgg ccctggcttc ggcgacctac accatctgga gcggctggca ccaccagact 180

gaggagctgc cgcggggccg ggagctgcgg ggccgcttga tcggaagcct ctccgaagcc 240

cggctgcggc gggtggtggg gcaactggac ccacaccgtc tctggaacac ttatctgcgc 300

cccctgctgg ttgtgcggac cccgggcagc cccggcaatc tccaagtcag aaagttcctg 360

gaggctacac tacggacctt gacagcaggc tggcatgtgg aactggaccc cttcacagcc 420

ttgacacccc tggggccact ggactttggc aatgtggtgg ccacgctgga cccaggggct 480

gcccgtcacc tcacccttgc ctgccattat gactccaagc tcttcgcatc tgagtcggtt 540

ccctttgtgg gggcaacaga ttcggctgta ccttgcgccc tgctgctgga gctggctcag 600

gccctcgaca gggagttgag tagggccaag gagcaggaag ccccggtgac tctgcagctg 660

ctctttttgg atggtgaaga agcactgaag gagtggggac ccacagactc cctctatggc 720

tcccggcacc tggcccagct catggagtct gcaccccaca gcccgggccc caccaggatc 780

caggctatcg agctcttcat gctccttgat ctcctgggtg ccccgaatcc aaacttctac 840

agtcacttcc ctcatacagc ccgctggttc catcggctga ggagcatcga gaagcgcctt 900

caccgcatga acctgctgca gtctcatccc caggaagtga tgtacttcca gcccggggag 960

ccccctggtt ctgtggaaga tgaccacatc cccttcctcc gccgaggggt ccctgtgctc 1020

cacctcatct ccatgccctt cccctccgtc tggcacaccc ccgatgactc tgaggccaac 1080

ctgcacccac ccaccgtaca caatctgagc cgcatcctcg ccgtgttcct ggccgaatat 1140

ctggggctct ag 1152

<210> 32
<211> 1152
<212> DNA

<213>  Rattus norvegicus

<400>  32
   atgagtccgg ccagccgcgg gcggtctcgg cagcggctcg gggatcgcgg cctcatgaaa      60
   ccaccctcac tttccaagcg ccgtcttctg ccgcgggtgc agctcctgcc cctgctgctg     120
   ctggcgctgg ccctgggctt ggctttttat atcgtctgga atagctggca ccctggggtt     180
   gaggaggtat cacggagccg ggatctgcgg gtcccgctga tcggaagcct ttcagaagcc     240
   aagctgcggc ttgtggtagg gcagctggat ccacagcgtc tctggggaac ttttctgcgt     300
   cccttgttga ttgtacgacc cccaggtagt cctggcaatc tccaagtgag aaagttcctg     360
   gaggctacgt tgcagtccct atcggcaggc tggcacgtgg aactggaccc attcacagcc     420
   tcaaccccct tggggccact ggacttcggg aacgtggtgg ccacccttga cccaggagct     480
   gcccgtcacc tcaccctcgc ctgccattat gactctaagt tcttccctcc tgggttaccc     540
   ccctttgtgg gggccacaga ttcagccgtg ccctgtgccc tgcttctgga gttagtccag     600
   gcccttgatg tcatgctgag cagaatcaag cagcaggcag caccagtgac cctgcagctg     660
   ctcttcttgg acggggagga ggcactgaag gagtggggac caaaggactc cctctatggt     720
   tcccggcacc tagctcagat catggagtct ataccgcaca gccctggccc caccaggatc     780
   caggctattg agctctttgt ccttcttgac cttctgggag cgcccagtcc aatcttcttc     840
   agtcacttcc cccgcacagc ccgctggttc caacgactgc ggagcatcga gaagcgcctt     900
   caccgtctga acctactgca gtctcacccc caggaagtga tgtacttcca acccggggag     960
   ccccctggcc ctgtggaaga tgaccacatc cccttccttc gcagaggggt cccggtgctc    1020
   cacctcattg cgatgccctt ccctgccgtg tggcacacac ctgctgacac tgaggctaac    1080
   ctccacccgc ccacggtgca caacctgagc cgcatcctcg ccgtgttcct ggctgagtac    1140
   ctgggtctct ag                                                        1152


<210>  33
<211>  1152
<212>  DNA
<213>  Mus musculus

<400>  33
atgagtcccg ggagccgcgg gcggccccgg cagcggctcg aggatcgtgg cctcatgaaa      60

ccaccctcac tttccaagcg ccgtcttctg ccgcgagtgc agttcctgcc cctgctgctg     120

ctggcgctgg ctatgggctt ggctttctat atcgtctgga acagctggca ccctggggtt     180

gaggagatgt cacggagccg ggatctgcgg gtcccgctga tcggaagcct ttcagaagcc     240

aagctgcggc tggtggtagg gcagctggat ccgcagcgtc tctggggaac tttcctgcgt     300

cccttattga ttgtgcgacc cccgggtagt tctggcaatc tccaagtgag aaagttcctg     360

gaggctacgt tgcagtccct gtcggcaggc tggcatgttg aactggaccc attcacggcc     420

tcaaccccct tggggccact ggacttcggg aacgtggtgg ccacacttga cccaggagct     480

gcccgtcacc tcaccctcgc ctgccattat gactctaagt tcttccctcc ggggttgccc 540

ccctttgtgg gggccacaga ttcagctgtg ccctgtgccc tgcttctgga gttggtccag 600

gcccttgatg ccatgctgag cagaatcaag cagcaggcag caccggtgac cctgcagctg 660

cttttcttgg atggggagga ggcactgaag gagtggggac caaaggactc cctctatggc 720

tcccggcacc tagctcagat catggagtct ataccacaca gccctggccc caccaggatc 780

caggctattg agctctttgt cctcctcgac cttctgggag catccagtcc gatcttcttc 840

agtcacttcc ctcgcacagc ccgctggttc cagcgactga ggagcattga gaagcgcctt 900

caccggctga acctactgca gtctcacccc caggaagtga tgtacttcca acccggggag 960

ccccccggcc ctgtggaaga tgaccacatc cccttccttc gcagaggggt cccggtgctc 1020

cacctcattg ccacgccctt ccctgctgtg tggcacacac ctgctgacac cgaggccaac 1080

ctccacccac ccactgtgca taacctgagc cgcatccttg ctgtgttcct ggccgagtac 1140

ctgggactct ag 1152

<210> 34
<211> 1152
<212> DNA
<213> Bos taurus

<400> 34
atgccttccg ggggccgcgg gcggccccgg ctccaggtcg gggaacgcag ccttttggag 60

cgaccctcac cgcccaagcg ccgcctgata ccgcgggcac agctgttgcc ccagctgctg 120

ctggctctga cggtagcctc ggtgttctat accatttgga ggatctggca tagccagact 180

gaagagctac cgctggggcg ggagctgcgg ggccctttga tcggaagcct ccccgaagct 240

cgggtgcgga gggtagtggg gcaactggac cctcaccgtc tctggaacac tttcctgcgc 300

cctctgctgg ttgtacggac tccgggcagc ccgggcaatc tccaagtgag aaagttcctg 360

gaggctacgc tgcggacact ttcagcaggc tggcatatag aactcgactc cttcactgcc 420

tccacacccg tggggccatt ggacttcagc aatgtggtgg ccacgctgga cccaggggct 480

gcccgccacc ttacccttgc ctgccattat gactccaagc tcttcccatc tgactcagcc 540

ccctttgtgg gggccacgga ttcggcagtg ccttgctccc tgctactgga gctggcccaa 600

gcccttgacc aggagctggg caaagccaag gagagggcag cgccaatgac cttgcagctg 660

atcttcctgg atggtgaaga ggcactgaag cagtggggac ccaaggactc gctttatggc 720

tcccggcacc tggcccagct catggagtct acaccccacg gcctgggctc caccaggatc 780

caggctattg agctctttat gcttcttgat ctcctgggag cccccaaccc gaccttctac 840

agtcacttcc ctcgcacggc ccgctggttc catcggctca ggagcattga gaagcgcctg 900

caccgtctga acctcctgca gtctcatcct tgggaagtga tgtacttcca gaccggggag 960

ccccccggct ccgtggaaga cgaccacatc ccgttcctcc gccgaggagt tcccgtgctc 1020

cacctcatcg ccacaccctt cccctctgtc tggcacacgt ccgatgactc cgaggccaac 1080

ctgcacccac ccacggtaca caacctgagc cgcatcctgg ccgtgttcct ggctgagtac 1140

ctggggctct ag 1152

<210> 35
<211> 1457
<212> DNA
<213> human

<400> 35
gtctggtaca ggtttcaggg caaagcggcc atgcgttccg ggggccgcgg gcgaccccgc 60

ctgcggctgg gggaacgtgg cctcatggag ccactcttgc cgccgaagcg ccgcctgcta 120

ccgcgggttc ggctcttgcc tctgttgctg gcgctggccg tgggctcggc gttctacacc 180

atttggagcg gctggcaccg caggactgag gagctgccgc tgggccggga gctgcgggtc 240

ccattgatcg gaagcctccc cgaagcccgg ctgcggaggg tggtgggaca actggatcca 300

cagcgtctct ggagcactta tctgcgcccc ctgctggttg tgcgaacccc gggcagcccg 360

ggaaatctcc aagtcagaaa gttcctggag gccacgctgc ggtccctgac agcaggttgg 420

cacgtggagc tggatccctt cacagcctca acacccctgg ggccagtgga ctttggcaat 480

gtggtggcca cactggaccc aagggctgcc cgtcacctca cccttgcctg ccattatgac 540

tcgaagctct tcccacccgg atcgaccccc tttgtagggg ccacggattc ggctgtgccc 600

tgtgccctgc tgctggagct ggcccaagca cttgacctgg agctgagcag ggccaaaaaa 660

caggcagccc cggtgaccct gcaactgctc ttcttggatg gtgaagaggc gctgaaggag 720

tggggaccca aggactccct ttacggttcc cggcacctgg cccagctcat ggagtctata 780

cctcacagcc ccggccccac caggatccag gctattgagc tctttatgct tcttgatctc 840

ctgggagccc ccaatcccac cttctacagc cacttccctc gcacggtccg ctggttccat 900

cggctgagga gcattgagaa gcgtctgcac cgtttgaacc tgctgcagtc tcatccccag 960

gaagtgatgt acttccaacc cggggagccc tttggctctg tggaagacga ccacatcccc 1020

ttcctccgca gaggggtacc cgtgctccat ctcatctcca cgcccttccc tgctgtctgg 1080

cacacccctg cggacaccga ggtcaatctc cacccaccca cggtacacaa cttgtgccgc 1140

attctcgctg tgttcctggc tgaatacctg gggctctagc gtgcttggcc aatgactgtg 1200

gagaggactg tgagagagaa ggtcccagcg ggggccagtg aagctcaggc aggatctgcc 1260

tagggtgtgc tggtttgtcc ttttcatacc tttgtctcct aattgtgcta caattggaag 1320

accttctttc ttttgattgt ctcaagctgc cacccttcaa ggacagggaa gagaccactg 1380

tgggatgaca gccagaggaa taagaacttg ctccctcccc agaggtaaac acttggtcca 1440

aaggtttgca gggacca 1457

<210>  36
<211>  1088
<212>  DNA
<213>  human

<400>  36
agcggccatg cgttccgggg gccgcgggcg accccgcctg cggctggggg aacgtggcct      60

catggagcca ctcttgccgc cgaagcgccg cctgctaccg cgggttcggc tcttgcctct     120

gttgctggcg ctggccgtgg gctcggcgtt ctacaccatt tggagcggct ggcaccgcag     180

gactgaggag ctgccgctgg gccgggagct gcgggtccca ttgatcggaa gcctccccga     240

agcccggctg cggagggtgg tgggacaact ggatccacag cgtctctgga gcacttatct     300

gcgccccctg ctggttgtgc gaaccccggg cagcccggga aatctccaag tcagaaaggc     360

agccccggtg accctgcaac tgctcttctt ggatggtgaa gaggcgctga aggagtgggg     420

acccaaggac tccctttacg gttcccggca cctggcccag ctcatggagt ctatacctca     480

cagccccggc cccaccagga tccaggctat tgagctcttt atgcttcttg atctcctggg     540

agcccccaat cccaccttct acagccactt ccctcgcacg gtccgctggt tccatcggct     600

gaggagcatt gagaagcgtc tgcaccgttt gaacctgctg cagtctcatc cccaggaagt     660

gatgtacttc caacccgggg agccctttgg ctctgtggaa gacgaccaca tccccttcct     720

ccgcagaggg gtacccgtgc tccatctcat ctccacgccc ttccctgctg tctggcacac     780

ccctgcggac accgaggtca atctccaccc acccacggta cacaacttgt gccgcattct     840

cgctgtgttc ctggctgaat acctggggct ctagcgtgct tggccaatga ctgtggagag     900

gactgtgaga gagaaggtcc cagcgggggc cagtgaagct caggcaggat ctgcctaggg     960

tgtgctggtt tgtccttttc atacctttgt ctcctaattg tgctacaatt ggaagacctt    1020

ctttcttttg attgtctcaa gctgccaccc ttcaaggaca gggaagagac cactgtggga    1080

tgacagcc                                                             1088


<210>  37
<211>  23
<212>  DNA
<213>  Artificial sequence

<220>
<223>  PCR primer

<400>  37
tcctacaagt ctttgtgttg gaa                                              23


<210>  38
<211>  19
<212>  DNA
<213>  Artificial sequence

<220>
<223>  PCR Primer

<400>  38
gaaggacttg gggagggag                                                    19


<210>  39
<211>  27
<212>  DNA
<213>  Artificial sequence

<220>
<223>  PCR primer

<400>  39
caagtaagtc gaggtccttc ctctgca                                           27


<210>  40
<211>  22
<212>  DNA
<213>  Artificial sequence

<220>
<223>  PCR primer

<400>  40
agcaagcctg gaagacctgg ga                                                22


<210>  41
<211>  22
<212>  DNA
<213>  Artificial sequence

<220>
<223>  PCR primer

<400>  41
agactcagcc catccactcc tt                                                22


<210>  42
<211>  38
<212>  DNA
<213>  Artificial sequence

<220>
<223>  PCR Primer

<400>  42
aataatctcg aggccaccat ggcaggcgga agacaccg                               38


<210>  43
<211>  26
<212>  DNA
<213>  Artificial sequence

<220>
<223>  PCR primer

<400>  43
acatatgtac attacaaatg aagata                                            26